(19) Europäisches Patentamt
European Patent Office
Office européen des brevets

(11) EP 2 892 509 B1

(12) **EUROPEAN PATENT SPECIFICATION**

(45) Date of publication and mention of the grant of the patent:
**11.12.2024 Bulletin 2024/50**

(21) Application number: **13834720.8**

(22) Date of filing: **04.09.2013**

(51) International Patent Classification (IPC):
***A61K 9/127*** *(2006.01)* ***A61P 35/00*** *(2006.01)*
***A61P 29/00*** *(2006.01)* ***A61P 25/28*** *(2006.01)*
***A61K 38/00*** *(2006.01)* ***A61K 47/18*** *(2017.01)*
***A61K 38/05*** *(2006.01)*

(52) Cooperative Patent Classification (CPC):
**A61K 38/05; A61K 9/1272; A61K 47/18; A61K 47/186; A61P 25/28; A61P 29/00; A61P 35/00**

(86) International application number:
**PCT/US2013/057960**

(87) International publication number:
**WO 2014/039504 (13.03.2014 Gazette 2014/11)**

(54) **BOLAAMPHIPHILIC COMPOUNDS, COMPOSITIONS AND USES THEREOF**

BOLAAMPHIPHILE VERBINDUNGEN, ZUSAMMENSETZUNGEN UND VERWENDUNGEN DAVON

COMPOSÉS BOLAAMPHIPHILES, COMPOSITIONS ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.09.2012 US 201261696798 P**

(43) Date of publication of application:
**15.07.2015 Bulletin 2015/29**

(73) Proprietor: **Lauren Sciences LLC**
**New York, NY 10128 (US)**

(72) Inventors:
- **LINDER, Charles**
**New York, NY 10128 (US)**
- **HELDMAN, Eliahu**
**New York, NY 10128 (US)**
- **GRINBERG,Sarina**
**New York, NY 10128 (US)**

(74) Representative: **2s-ip Schramm Schneider Bertagnoll**
**Patent- und Rechtsanwälte Part mbB**
**Denninger Straße 169**
**81925 München (DE)**

(56) References cited:
**WO-A1-00/56728 WO-A2-2010/128504**
**US-A1- 2008 069 868 US-A1- 2010 190 722**

- **POPOV MARY ET AL: "Site-directed decapsulation of bolaamphiphilic vesicles with enzymatic cleavable surface groups", JOURNAL OF CONTROLLED RELEASE, vol. 160, no. 2, 29 December 2011 (2011-12-29), pages 306 - 314, XP028926636, ISSN: 0168-3659, DOI: 10.1016/J.JCONREL.2011.12.022**
- **PAOLINO ET AL: "Innovative bola-surfactant niosomes as topical delivery systems of 5-fluorouracil for the treatment of skin cancer", INTERNATIONAL JOURNAL OF PHARMACEUTICS, ELSEVIER BV, NL, vol. 353, no. 1-2, 28 November 2007 (2007-11-28), pages 233 - 242, XP022513723, ISSN: 0378-5173, DOI: 10.1016/J.IJPHARM.2007.11.037**
- **S. GRINBERG ET AL: "Synthesis of novel cationic bolaamphiphiles from vernonia oil and their aggregated structures", CHEMISTRY AND PHYSICS OF LIPIDS., vol. 153, no. 2, 1 June 2008 (2008-06-01), IR, pages 85 - 97, XP055565983, ISSN: 0009-3084, DOI: 10.1016/j.chemphyslip.2008.01.006**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 892 509 B1

- CHALON SYLVIE ET AL.: "Pharmacological Characterization of (E)-N-(4-Fluorobut-2-enyl)-2beta-carbomethoxy-3beta-(4'tolyl)nortropane (LBT-999) as a Highly Promising Fluorinated Ligand for the Dopamine Transporter.", THE JOURNAL OF PHARMACOLOGY AND EXPERIMENTAL THERAPEUTICS, vol. 317, no. 1, 2006, pages 147 - 152, XP055238698

## Description

### FIELD

Provided herein are nanovesicles comprising bolaamphiphilic compounds, and complexes thereof with biologically active molecules, and pharmaceutical compositions thereof. Also provided are methods of delivering biologically active molecules into the human brain and animal brain using the compounds, complexes and pharmaceutical compositions provided herein.

### BACKGROUND

Many drugs and biologically active molecules cannot penetrate the BBB and thus require direct administration into the CNS tissue or the cerebral spinal fluid (CSF) in order to achieve a biological or therapeutic effect. Even direct administration into a particular CNS site is often limited due to poor diffusion of the active agent because of local absorption/adsorption into the CNS matrix. Present modalities for drug delivery through the BBB entail disruption of the BBB by, for example, osmotic means (hyperosmotic solutions) or biochemical means (e.g., use of vasoactive substances such as. bradykinin), processes with serious side effects.

The brain is a highly specialized organ, and its sensitive components and functioning are protected by a barrier known as the blood-brain barrier (BBB). The brain capillary endothelial cells (BCECs) that form the BBB play important role in brain physiology by maintaining selective permeability and preventing passage of various compounds from the blood into the brain. One consequence of the highly effective barrier properties of the BBB is the limited penetration of therapeutic agents into the brain, which makes treatment of many brain diseases extremely challenging.

Efforts to improve the permeation of biologically active compounds across the BBB using amphphilic vesicles have been attempted.

For example, complexation of the anionic carboxyfluorescein (CF) with single headed amphiphiles of opposite charge in cationic vesicles, formed by mixing single-tailed cationic and anionic surfactants has been reported (Danoff et al. 2007).

Furthermore, WO 02/055011 and WO 03/047499, both of the same applicant, disclose amphiphilic derivatives composed of at least one fatty acid chain derived from natural vegetable oils such as vernonia oil, lesquerella oil and castor oil, in which functional groups such as epoxy, hydroxy and double bonds were modified into polar and ionic headgroups.

Additionally, WO 10/128504 reports a series of amphiphiles and bolamphiphiles (amphiphiles with two head groups) useful for targeted drug delivery of insulin, insulin analogs, TNF, GDNF, DNA, RNA (including siRNA), enkephalin class of analgesics, and others.

These synthetic bolaamphiphiles (bolas) have recently been shown to form nanovesicles that interact with and encapsulate a variety of small and large molecules including peptides, proteins and plasmid DNAs delivering them across biological membranes. These bolaamphiphiles are a unique class of compounds that have two hydrophilic headgroups placed at each ends of a hydrophobic domain. Bolaamphiphiles can form vesicles that consist of monolayer membrane that surrounds an aqueous core. Vesicles made from natural bolaamphiphiles, such as those extracted from archaebacteria (archaesomes), are very stable and, therefore, might be employed for targeted drug delivery. However, bolaamphiphiles from archaebacteria are heterogeneous and cannot be easily extracted or chemically synthesized.

Thus, there remains a need to make new compositions and for novel methods to deliver biologically active drugs into the brain. The compounds, compositions, and methods described herein are directed toward this end.

WO2010/128504 provides ano-sized particles comprising at least one multi-headed amphiphilic compound, in which at least one headgroup of said multi-headed amphiphilic compound is selectively cleavable or contains a selectively cleavable group, and at least one biologically active agent, which is both encapsulated within the nano-particle and non-covalently associated thereto.

POPOV MARY ET AL, "Site-directed decapsulation of bolaamphiphilic vesicles with enzymatic cleavable surface groups", JOURNAL OF CONTROLLED RELEASE, (20111229), vol. 160, no. 2, doi:10.1016/J.JCONREL.2011.12.022, ISSN 0168-3659, pages 306 - 314, XP028926636 [I] relates to stable nano-sized vesicles with a monolayer encapsulating membrane which were prepared from novel bolaamphiphiles with choline ester head groups. The head groups were covalently bound to the alkyl chain of the bolaamphiphiles either via the nitrogen atom of the choline moiety, or via the choline ester's methyl group. Both types of bolaamphiphiles competed with acetylthiocholine for binding to acetylcholine esterase (AChE), yet, only the choline ester head groups bound to the alkyl chain via the nitrogen atom of the choline moiety were hydrolyzed by the enzyme. Likewise, only vesicles composed of bolaamphiphiles with head groups that were hydrolyzed by AChE released their encapsulated material upon exposure to the enzyme. Injection of carboxyfluorescein (CF)-loaded vesicles with cleavable choline ester head groups into mice resulted in the accumulation of CF in tissues that express high AChE activity, including the brain.

PAOLINO ET AL, "Innovative bola-surfactant niosomes as topical delivery systems of 5-fluorouracil for the treatment of skin cancer", INTERNATIONAL JOURNAL OF PHARMACEUTICS, ELSEVIER BV, NL, (20071128), vol. 353, no. 1-2, doi: 10.1016/J.IJPHARM.2007.11.037, ISSN 0378-5173, pages 233 - 242, XP022513723 propose an innovative niosomal system made up of $\alpha,\omega$-hexadecyl-bis-(1-aza-18-crown-6) (Bola), Span 80® and cholesterol (2:5:2 molar ratio) was proposed as a topical delivery system for 5-fluorouracil (5-FU), largely used in the treatment of different forms of skin cancers. Bola-niosomes showed a mean size of ~400 nm, which were reduced to ~200 nm by a sonication procedure with a polydispersion index value of 0.1. Bola-niosomes showed a loading capacity of ~40% with respect to the amount of 5-FU added during the preparation. 5-FU-loaded bola-niosomes were tested on SKMEL-28 (human melanoma) and HaCaT (non-melanoma skin cancer with a specific mutations in the p53 tumor suppressor gene) to assess the cytotoxic activity with respect to the free drug. 5-FU-loaded bola-niosomes showed an improvement of the cytotoxic effect with respect to the free drug. US 2008/069868 A1 discloses liposomes encapsulating CPT-11.

**SUMMARY OF THE INVENTION**

The present invention provides pharmaceutical compositions or formulations with the features of claim 1.

In further aspects, provided herein are novel nano-sized vesicles comprising of bolaamphiphilic compounds.

In certain aspects, provided herein are novel bolaamphiphile complexes comprising one or more bolaamphiphilic compounds and a biologically active compound.

In one embodiment, the bolaamphiphilic compound consists of two hydrophilic headgroups linked through a long hydrophobic chain. In another embodiment, the hydrophilic headgroup is an amino containing group. In a specific embodiment, the hydrophilic headgroup is a tertiary or quaternary amino containing group.

The bolaamphiphilic compound is a compound according to formula I:

$$HG^2 - L^1 - HG^1$$

I

or a pharmaceutically acceptable salt, solvate, hydrate, prodrug, stereoisomer, tautomer, isotopic variant, or N-oxide thereof, or a combination thereof;
wherein:

each $HG^1$ and $HG^2$ is independently a hydrophilic head group; and
$L^1$ is alkylene, alkenyl, heteroalkylene, or heteroalkenyl linker; unsubstituted or substituted with $C_1$-$C_{20}$ alkyl, hydroxyl, or oxo.

The pharmaceutically acceptable salt is a quaternary ammonium salt.

In one embodiment, with respect to the bolaamphiphilic compound of formula I, the bolaamphiphilic compound is a compound according to formula II, III, IV, V, or VI:

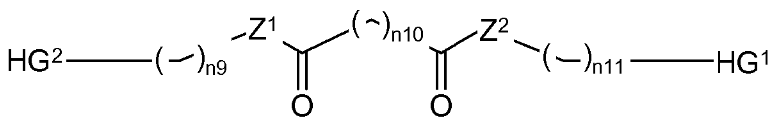

II

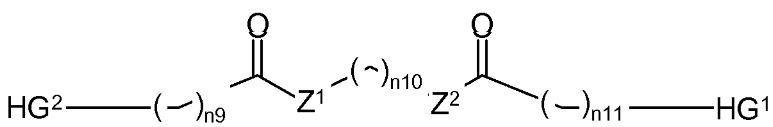

III

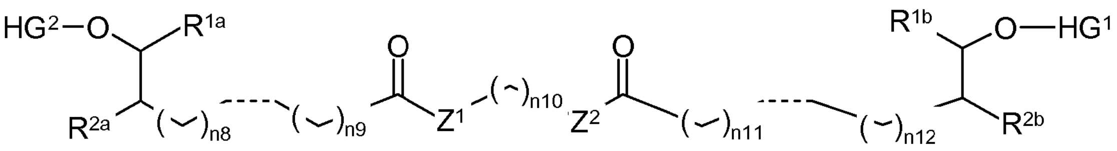

IV

,

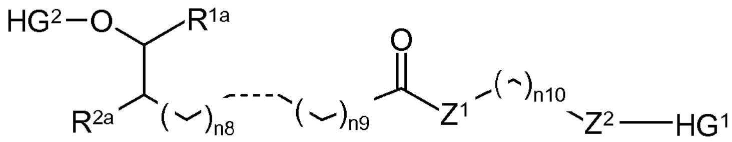

V

,

or

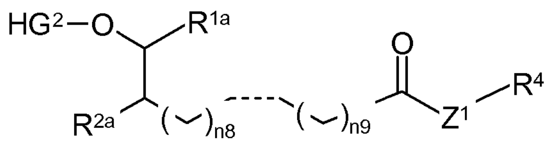

VI

or a pharmaceutically acceptable salt, solvate, hydrate, prodrug, stereoisomer, tautomer, isotopic variant, or N-oxide thereof, or a combination thereof;
wherein:

each $HG^1$ and $HG^2$ is independently a hydrophilic head group;
each $Z^1$ and $Z^2$ is independently $-C(R^3)_2-$, $-N(R^3)-$ or -O-;
each $R^{1a}$, $R^{1b}$, $R^3$, and $R^4$ is independently H or $C_1$-$C_8$ alkyl;
each $R^{2a}$ and $R^{2b}$ is independently H , $C_1$-$C_8$ alkyl, OH, alkoxy, or O-$HG^1$ or O-$HG^2$;
each n8, n9, n11, and n12 is independently an integer from 1-20;
n10 is an integer from 2-20; and
each dotted bond is independently a single or a double bond.

In one embodiment, with respect to the bolaamphiphilic compound of formula I, II, III, IV, V, or VI, each $HG^1$ and $HG^2$ is independently selected from:

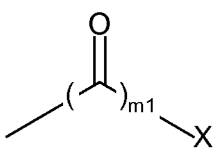

,

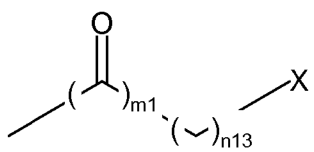

,

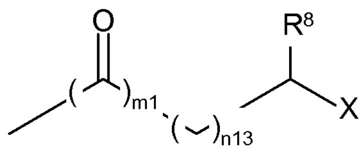

,

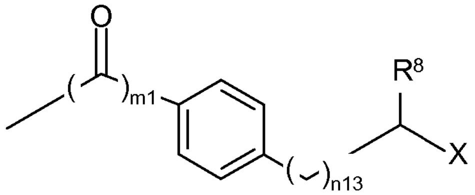

and

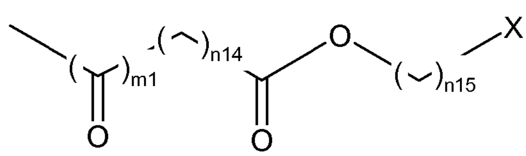

wherein:

X is $-NR^{5a}R^{5b}$, or $-N^+R^{5a}R^{5b}R^{5c}$; each $R^{5a}$, and $R^{5b}$ is independently H or substituted or unsubstituted $C_1$-$C_{20}$ alkyl or $R^{5a}$ and $R^{5b}$ may join together to form an N containing substituted or unsubstituted heteroaryl, or substituted or unsubstituted heterocyclyl;
each $R^{5c}$ is independently substituted or unsubstituted $C_1$-$C_{20}$ alkyl; each $R^8$ is independently H, substituted or unsubstituted $C_1$-$C_{20}$ alkyl, alkoxy, or carboxy;
m1 is 0 or 1; and
each n13, n14, and n15 is independently an integer from 1-20.

Other objects and advantages will become apparent to those skilled in the art from a consideration of the ensuing detailed description.

**FIGURES**

Figure 1: TEM micrograph of vesicles from GLH-20 (A) and their size distribution determined by DLS (B).
Figure 2: Head group hydrolysis by AChE (A) of GLH-19 (blue) and GLH-20 (red) and release of CF from GLH-19 vesicles (B) and GLH-20 vesicles (C)
Figure 3: CF accumulation in brain after i.v. injection of encapsulated and non-encapsulated CF. Only GLH-20 vesicles allow accumulation of CF in the brain (A). CS improves GLH-20 vesicles' penetration into the brain (B).
Figure 4: Analgesia after i.v. injection of enkephalin non-encapsulated and encapsulated in vesicles. Analgesia (compared with morphine, which was used as a positive control) is obtained only when enkephalin is encapsulated in GLH-20 vesicles (A), the head groups of which are hydrolyzed by ChE. The vesicles do not disrupt the BBB since non-encapsulated enkephalin co- injected with empty vesicles (extravesicular enkephalin) did not cause analgesia (B). **Significantly different from free leu-enkephalin (t-test, $P<0.01$). ***Significantly different from free leu-enkephalin (t-test, $P<0.001$).
Figure 5: Fluorescence in mouse cerebral cortex after i.v. injection of albumin-FITC (non-encapsulated) (A) encapsulated in GLH-20 vesicles (B).
Figure 6: Brain delivery of analgesic peptide kyotorphin.

**DEFINITIONS**

*Chemical Definitions*

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Compounds described herein can comprise one or more asymmetric centers, and thus can exist in various isomeric forms, *e.g.*, enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The invention additionally encompasses compounds described herein as individual isomers substantially free of other isomers, and alternatively, as mixtures of various isomers.

When a range of values is listed, it is intended to encompass each value and sub-range within the range. For example "$C_{1-6}$ alkyl" is intended to encompass, $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$, and $C_{5-6}$ alkyl.

The following terms are intended to have the meanings presented therewith below and are useful in understanding the description and intended scope of the present invention. When describing the invention, which may include compounds, pharmaceutical compositions containing such compounds and methods of using such compounds and compositions, the following terms, if present, have the following meanings unless otherwise indicated. It should also be understood that when described herein any of the moieties defined forth below may be substituted with a variety of substituents, and that the respective definitions are intended to include such substituted moieties within their scope as set out below. Unless otherwise stated, the term "substituted" is to be defined as set out below. It should be further understood that the terms "groups" and "radicals" can be considered interchangeable when used herein. The articles "a" and "an" may be used herein to refer to one or to more than one (*i.e.* at least one) of the grammatical objects of the article. By way of example "an analogue" means one analogue or more than one analogue.

"Alkyl" refers to a radical of a straight-chain or branched saturated hydrocarbon group having from 1 to 20 carbon atoms ("$C_{1-20}$ alkyl"). In some embodiments, an alkyl group has 1 to 12 carbon atoms ("$C_{1-12}$ alkyl"). In some embodiments, an alkyl group has 1 to 10 carbon atoms ("$C_{1-10}$ alkyl"). In some embodiments, an alkyl group has 1 to 9 carbon atoms ("$C_{1-9}$ alkyl"). In some embodiments, an alkyl group has 1 to 8 carbon atoms ("$C_{1-8}$ alkyl"). In some embodiments, an alkyl group has 1 to 7 carbon atoms ("$C_{1-7}$ alkyl"). In some embodiments, an alkyl group has 1 to 6 carbon atoms ("$C_{1-6}$ alkyl", also referred to herein as "lower alkyl"). In some embodiments, an alkyl group has 1 to 5 carbon atoms ("$C_{1-5}$ alkyl"). In some embodiments, an alkyl group has 1 to 4 carbon atoms ("$C_{1-4}$ alkyl"). In some embodiments, an alkyl group has 1 to 3 carbon atoms ("$C_{1-3}$ alkyl"). In some embodiments, an alkyl group has 1 to 2 carbon atoms ("$C_{1-2}$ alkyl"). In some embodiments, an alkyl group has 1 carbon atom ("$C_1$ alkyl"). In some embodiments, an alkyl group has 2 to 6 carbon atoms ("$C_{2-6}$ alkyl"). Examples of $C_{1-6}$ alkyl groups include methyl ($C_1$), ethyl ($C_2$), n-propyl ($C_3$), isopropyl ($C_3$), n-butyl ($C_4$), tert-butyl ($C_4$), sec-butyl ($C_4$), iso-butyl ($C_4$), n-pentyl ($C_5$), 3-pentanyl ($C_5$), amyl ($C_5$), neopentyl ($C_5$), 3-methyl-2-butanyl ($C_5$), tertiary amyl ($C_5$), and n-hexyl ($C_6$). Additional examples of alkyl groups include n-heptyl ($C_7$), n-octyl ($C_8$) and the like. Unless otherwise specified, each instance of an alkyl group is independently optionally substituted, *i.e.,* unsubstituted (an "unsubstituted alkyl") or substituted (a "substituted alkyl") with one or more substituents; *e.g.*, for instance from 1 to 5 substituents, 1 to 3 substituents, or 1 substituent. In certain embodiments, the alkyl group is unsubstituted $C_{1-10}$ alkyl (*e.g.,* $-CH_3$). In certain embodiments, the alkyl group is substituted $C_{1-10}$ alkyl.

"Alkylene" refers to a substituted or unsubstituted alkyl group, as defined above, wherein two hydrogens are removed to provide a divalent radical. Exemplary divalent alkylene groups include, but are not limited to, methylene ($-CH_2-$), ethylene ($-CH_2CH_2-$), the propylene isomers (*e.g.*, $-CH_2CH_2CH_2-$ and $-CH(CH_3)CH_2-$) and the like.

"Alkenyl" refers to a radical of a straight-chain or branched hydrocarbon group having from 2 to 20 carbon atoms, one or more carbon-carbon double bonds, and no triple bonds ("$C_{2-20}$ alkenyl"). In some embodiments, an alkenyl group has 2 to 10 carbon atoms ("$C_{2-10}$ alkenyl"). In some embodiments, an alkenyl group has 2 to 9 carbon atoms ("$C_{2-9}$ alkenyl"). In some embodiments, an alkenyl group has 2 to 8 carbon atoms ("$C_{2-8}$ alkenyl"). In some embodiments, an alkenyl group has 2 to 7 carbon atoms ("$C_{2-7}$ alkenyl"). In some embodiments, an alkenyl group has 2 to 6 carbon atoms ("$C_{2-6}$ alkenyl"). In some embodiments, an alkenyl group has 2 to 5 carbon atoms ("$C_{2-5}$ alkenyl"). In some embodiments, an alkenyl group has 2 to 4 carbon atoms ("$C_{2-4}$ alkenyl"). In some embodiments, an alkenyl group has 2 to 3 carbon atoms ("$C_{2-3}$ alkenyl"). In some embodiments, an alkenyl group has 2 carbon atoms ("$C_2$ alkenyl"). The one or more carbon-carbon double bonds can be internal (such as in 2-butenyl) or terminal (such as in 1-butenyl). Examples of $C_{2-4}$ alkenyl groups include ethenyl ($C_2$), 1-propenyl ($C_3$), 2-propenyl ($C_3$), 1-butenyl ($C_4$), 2-butenyl ($C_4$), butadienyl ($C_4$), and the like. Examples of $C_{2-6}$ alkenyl groups include the aforementioned $C_{2-4}$ alkenyl groups as well as pentenyl ($C_5$), pentadienyl ($C_5$), hexenyl ($C_6$), and the like. Additional examples of alkenyl include heptenyl ($C_7$), octenyl ($C_8$), octatrienyl ($C_8$), and the like. Unless otherwise specified, each instance of an alkenyl group is independently optionally substituted, *i.e.,* unsubstituted (an "unsubstituted alkenyl") or substituted (a "substituted alkenyl") with one or more substituents *e.g.,* for instance from 1 to 5 substituents, 1 to 3 substituents, or 1 substituent. In certain embodiments, the alkenyl group is unsubstituted $C_{2-10}$ alkenyl. In certain embodiments, the alkenyl group is substituted $C_{2-10}$ alkenyl.

"Alkenylene" refers a substituted or unsubstituted alkenyl group, as defined above, wherein two hydrogens are removed to provide a divalent radical. Exemplary divalent alkenylene groups include, but are not limited to, ethenylene (-CH=CH-), propenylenes (e.g., $-CH=CHCH_2-$ and $-C(CH_3)=CH-$ and $-CH=C(CH_3)-$) and the like.

"Alkynyl" refers to a radical of a straight-chain or branched hydrocarbon group having from 2 to 20 carbon atoms, one or more carbon-carbon triple bonds, and optionally one or more double bonds ("$C_{2-20}$ alkynyl"). In some embodiments, an alkynyl group has 2 to 10 carbon atoms ("$C_{2-10}$ alkynyl"). In some embodiments, an alkynyl group has 2 to 9 carbon atoms ("$C_{2-9}$ alkynyl"). In some embodiments, an alkynyl group has 2 to 8 carbon atoms ("$C_{2-8}$ alkynyl"). In some embodiments, an alkynyl group has 2 to 7 carbon atoms ("$C_{2-7}$ alkynyl"). In some embodiments, an alkynyl group has 2 to 6 carbon atoms ("$C_{2-6}$ alkynyl"). In some embodiments, an alkynyl group has 2 to 5 carbon atoms ("$C_{2-5}$ alkynyl"). In some embodiments, an alkynyl group has 2 to 4 carbon atoms ("$C_{2-4}$ alkynyl"). In some embodiments, an alkynyl group has 2 to 3 carbon atoms ("$C_{2-3}$ alkynyl"). In some embodiments, an alkynyl group has 2 carbon atoms ("$C_2$ alkynyl"). The one or more carbon-carbon triple bonds can be internal (such as in 2-butynyl) or terminal (such as in 1-butynyl). Examples of $C_{2-4}$ alkynyl groups include, without limitation, ethynyl ($C_2$), 1-propynyl ($C_3$), 2-propynyl ($C_3$),

1-butynyl ($C_4$), 2-butynyl ($C_4$), and the like. Examples of $C_{2-6}$ alkenyl groups include the aforementioned $C_{2-4}$ alkynyl groups as well as pentynyl ($C_5$), hexynyl ($C_6$), and the like. Additional examples of alkynyl include heptynyl ($C_7$), octynyl ($C_8$), and the like. Unless otherwise specified, each instance of an alkynyl group is independently optionally substituted, *i.e.,* unsubstituted (an "unsubstituted alkynyl") or substituted (a "substituted alkynyl") with one or more substituents; *e.g.*, for instance from 1 to 5 substituents, 1 to 3 substituents, or 1 substituent. In certain embodiments, the alkynyl group is unsubstituted $C_{2-10}$ alkynyl. In certain embodiments, the alkynyl group is substituted $C_{2-10}$ alkynyl.

"Alkynylene" refers a substituted or unsubstituted alkynyl group, as defined above, wherein two hydrogens are removed to provide a divalent radical. Exemplary divalent alkynylene groups include, but are not limited to, ethynylene, propynylene, and the like.

'"Aryl" refers to a radical of a monocyclic or polycyclic (*e.g.*, bicyclic or tricyclic) 4n+2 aromatic ring system (*e.g.*, having 6, 10, or 14 $\pi$ electrons shared in a cyclic array) having 6-14 ring carbon atoms and zero heteroatoms provided in the aromatic ring system ("$C_{6-14}$ aryl"). In some embodiments, an aryl group has six ring carbon atoms ("$C_6$ aryl"; *e.g.*, phenyl). In some embodiments, an aryl group has ten ring carbon atoms ("$C_{10}$ aryl"; *e.g.*, naphthyl such as 1-naphthyl and 2-naphthyl). In some embodiments, an aryl group has fourteen ring carbon atoms ("$C_{14}$ aryl"; *e.g.*, anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted $C_{6-14}$ aryl. In certain embodiments, the aryl group is substituted $C_{6-14}$ aryl.

In certain embodiments, an aryl group substituted with one or more of groups selected from halo, $C_1$-$C_8$ alkyl, $C_1$-$C_8$ haloalkyl, cyano, hydroxy, $C_1$-$C_8$ alkoxy, and amino.

Examples of representative substituted aryls include the following

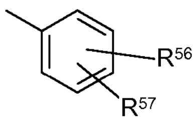 , 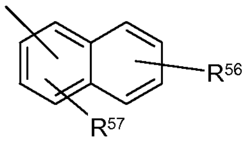 and 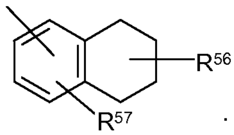 .

In these formulae one of $R^{56}$ and $R^{57}$ may be hydrogen and at least one of $R^{56}$ and $R^{57}$ is each independently selected from $C_1$-$C_8$ alkyl, $C_1$-$C_8$ haloalkyl, 4-10 membered heterocyclyl, alkanoyl, $C_1$-$C_8$ alkoxy, heteroaryloxy, alkylamino, arylamino, heteroarylamino, $NR^{58}COR^{59}$, $NR^{58}SOR^{59}$ $NR^{58}SO_2R^{59}$, COOalkyl, COOaryl, $CONR^{58}R^{59}$, $CONR^{58}OR^{59}$, $NR^{58}R^{59}$, $SO_2NR^{58}R^{59}$, S-alkyl, SOalkyl, $SO_2$alkyl, Saryl, SOaryl, $SO_2$aryl; or $R^{56}$ and $R^{57}$ may be joined to form a cyclic ring (saturated or unsaturated) from 5 to 8 atoms, optionally containing one or more heteroatoms selected from the group N, O, or S. $R^{60}$ and $R^{61}$ are independently hydrogen, $C_1$-$C_8$ alkyl, $C_1$-$C_4$ haloalkyl, $C_3$-$C_{10}$ cycloalkyl, 4-10 membered heterocyclyl, $C_6$-$C_{10}$ aryl, substituted $C_6$-$C_{10}$ aryl, 5-10 membered heteroaryl, or substituted 5-10 membered heteroaryl.

"Fused aryl" refers to an aryl having two of its ring carbon in common with a second aryl ring or with an aliphatic ring.

"Aralkyl" is a subset of alkyl and aryl, as defined herein, and refers to an optionally substituted alkyl group substituted by an optionally substituted aryl group.

"Heteroaryl" refers to a radical of a 5-10 membered monocyclic or bicyclic 4n+2 aromatic ring system (*e.g.*, having 6 or 10 $\pi$ electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein

one ring does not contain a heteroatom (*e.g.*, indolyl, quinolinyl, carbazolyl, and the like) the point of attachment can be on either ring, *i.e.,* either the ring bearing a heteroatom (*e.g.*, 2-indolyl) or the ring that does not contain a heteroatom (*e.g.*, 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Examples of representative heteroaryls include the following:

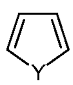 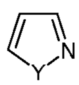 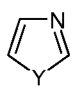 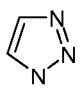 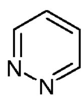 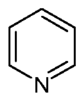

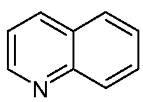 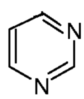 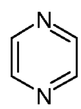 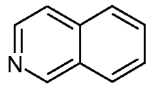

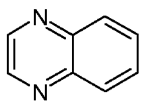 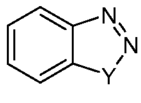 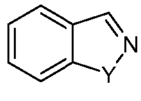 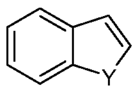

wherein each Y is selected from carbonyl, N, $NR^{65}$, O, and S; and $R^{65}$ is independently hydrogen, $C_1$-$C_8$ alkyl, $C_3$-$C_{10}$ cycloalkyl, 4-10 membered heterocyclyl, $C_6$-$C_{10}$ aryl, and 5-10 membered heteroaryl.

Examples of representative aryl having hetero atoms containing substitution include the following:

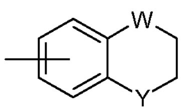 , 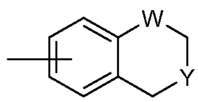 and 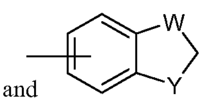 ,

wherein each W is selected from $C(R^{66})_2$, $NR^{66}$, O, and S; and each Y is selected from carbonyl, $NR^{66}$, O and S; and

$R^{66}$ is independently hydrogen, $C_1$-$C_8$ alkyl, $C_3$-$C_{10}$ cycloalkyl, 4-10 membered heterocyclyl, $C_6$-$C_{10}$ aryl, and 5-10 membered heteroaryl.

"Heteroaralkyl" is a subset of alkyl and heteroaryl, as defined herein, and refers to an optionally substituted alkyl group substituted by an optionally substituted heteroaryl group.

"Carbocyclyl" or "carbocyclic" refers to a radical of a non-aromatic cyclic hydrocarbon group having from 3 to 10 ring carbon atoms ("$C_{3-10}$ carbocyclyl") and zero heteroatoms in the non-aromatic ring system. In some embodiments, a carbocyclyl group has 3 to 8 ring carbon atoms ("$C_{3-8}$ carbocyclyl"). In some embodiments, a carbocyclyl group has 3 to 6 ring carbon atoms ("$C_{3-6}$ carbocyclyl"). In some embodiments, a carbocyclyl group has 3 to 6 ring carbon atoms ("$C_{3-6}$ carbocyclyl"). In some embodiments, a carbocyclyl group has 5 to 10 ring carbon atoms ("$C_{5-10}$ carbocyclyl"). Exemplary $C_{3-6}$ carbocyclyl groups include, without limitation, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), and the like. Exemplary $C_{3-8}$ carbocyclyl groups include, without limitation, the aforementioned $C_{3-6}$ carbocyclyl groups as well as cycloheptyl ($C_7$), cycloheptenyl ($C_7$), cycloheptadienyl ($C_7$), cycloheptatrienyl ($C_7$), cyclooctyl ($C_8$), cyclooctenyl ($C_8$), bicyclo[2.2.1]heptanyl ($C_7$), bicyclo[2.2.2]octanyl ($C_8$), and the like. Exemplary $C_{3-10}$ carbocyclyl groups include, without limitation, the aforementioned $C_{3-8}$ carbocyclyl groups as well as cyclononyl ($C_9$), cyclononenyl ($C_9$), cyclodecyl ($C_{10}$), cyclodecenyl ($C_{10}$), octahydro-1*H*-indenyl ($C_9$), decahydronaphthalenyl ($C_{10}$), spiro[4.5]decanyl ($C_{10}$), and the like. As the foregoing examples illustrate, in certain embodiments, the carbocyclyl group is either monocyclic ("monocyclic carbocyclyl") or contain a fused, bridged or spiro ring system such as a bicyclic system ("bicyclic carbocyclyl") and can be saturated or can be partially unsaturated. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Unless otherwise specified, each instance of a carbocyclyl group is independently optionally substituted, *i.e.,* unsubstituted (an "unsubstituted carbocyclyl") or substituted (a "substituted carbocyclyl") with one or more substituents. In certain embodiments, the carbocyclyl group is unsubstituted $C_{3-10}$ carbocyclyl. In certain embodiments, the carbocyclyl group is a substituted $C_{3-10}$ carbocyclyl.

In some embodiments, "carbocyclyl" is a monocyclic, saturated carbocyclyl group having from 3 to 10 ring carbon atoms ("$C_{3-10}$ cycloalkyl"). In some embodiments, a cycloalkyl group has 3 to 8 ring carbon atoms ("$C_{3-8}$ cycloalkyl"). In some embodiments, a cycloalkyl group has 3 to 6 ring carbon atoms ("$C_{3-6}$ cycloalkyl"). In some embodiments, a cycloalkyl group has 5 to 6 ring carbon atoms ("$C_{5-6}$ cycloalkyl"). In some embodiments, a cycloalkyl group has 5 to 10 ring carbon atoms ("$C_{5-10}$ cycloalkyl"). Examples of $C_{5-6}$ cycloalkyl groups include cyclopentyl ($C_5$) and cyclohexyl ($C_5$). Examples of $C_{3-6}$ cycloalkyl groups include the aforementioned $C_{5-6}$ cycloalkyl groups as well as cyclopropyl ($C_3$) and cyclobutyl ($C_4$). Examples of $C_{3-8}$ cycloalkyl groups include the aforementioned $C_{3-6}$ cycloalkyl groups as well as cycloheptyl ($C_7$) and cyclooctyl ($C_8$). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is unsubstituted $C_{3-10}$ cycloalkyl. In certain embodiments, the cycloalkyl group is substituted $C_{3-10}$ cycloalkyl.

"Heterocyclyl" or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. Unless otherwise specified, each instance of heterocyclyl is independently optionally substituted, *i.e.,* unsubstituted (an "unsubstituted heterocyclyl") or substituted (a "substituted heterocyclyl") with one or more substituents. In certain embodiments, the heterocyclyl group is unsubstituted 3-10 membered heterocyclyl. In certain embodiments, the heterocyclyl group is substituted 3-10 membered heterocyclyl.

In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl").

In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a $C_6$ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

Particular examples of heterocyclyl groups are shown in the following illustrative examples:

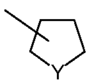 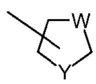 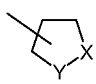 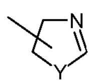 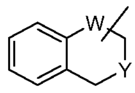

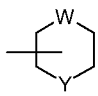 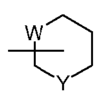 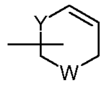 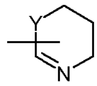 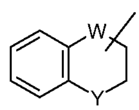

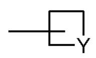 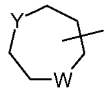 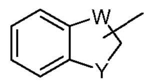

wherein each W is selected from $CR^{67}$, $C(R^{67})_2$, $NR^{67}$, O, and S; and each Y is selected from $NR^{67}$, O, and S; and $R^{67}$ is independently hydrogen, $C_1$-$C_8$ alkyl, $C_3$-$C_{10}$ cycloalkyl, 4-10 membered heterocyclyl, $C_6$-$C_{10}$ aryl, 5-10 membered heteroaryl. These heterocyclyl rings may be optionally substituted with one or more substituents selected from the group consisting of the group consisting of acyl, acylamino, acyloxy, alkoxy, alkoxycarbonyl, alkoxycarbonylamino, amino, substituted amino, aminocarbonyl (carbamoyl or amido), aminocarbonylamino, aminosulfonyl, sulfonylamino, aryl, aryloxy, azido, carboxyl, cyano, cycloalkyl, halogen, hydroxy, keto, nitro, thiol, -S-alkyl, -S-aryl, -S(O)-alkyl,-S(O)-aryl, $-S(O)_2$-alkyl, and $-S(O)_2$-aryl. Substituting groups include carbonyl or thiocarbonyl which provide, for example, lactam and urea derivatives.

"Hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.,* heteroalkyl, cycloalkyl, *e.g.,* heterocyclyl, aryl, *e.g,.* heteroaryl, cycloalkenyl, *e.g,.* cycloheteroalkenyl, and the like having from 1 to 5, and particularly from 1 to 3 heteroatoms.

"Acyl" refers to a radical $-C(O)R^{20}$, where $R^{20}$ is hydrogen, substituted or unsubstitued alkyl, substituted or unsubstitued alkenyl, substituted or unsubstitued alkynyl, substituted or unsubstitued carbocyclyl, substituted or unsubstituted heterocyclyl, substituted or unsubstituted aryl, or substituted or unsubstitued heteroaryl, as defined herein. "Alkanoyl" is an acyl group wherein $R^{20}$ is a group other than hydrogen. Representative acyl groups include, but are not limited to, formyl (-CHO), acetyl ($-C(=O)CH_3$), cyclohexylcarbonyl, cyclohexylmethylcarbonyl, benzoyl (-C(=O)Ph), benzylcarbonyl ($-C(=O)CH_2Ph$), -C(O)-$C_1$-$C_8$ alkyl, $-C(O)-(CH_2)_t(C_6$-$C_{10}$ aryl), $-C(O)-(CH_2)_t$(5-10 membered heteroaryl), $-C(O)-(CH_2)_t(C_3$-$C_{10}$ cycloalkyl), and $-C(O)-(CH_2)_t$(4-10 membered heterocyclyl), wherein t is an integer from 0 to 4. In certain embodiments, $R^{21}$ is $C_1$-$C_8$ alkyl, substituted with halo or hydroxy; or $C_3$-$C_{10}$ cycloalkyl, 4-10 membered heterocyclyl, $C_6$-$C_{10}$ aryl, arylalkyl, 5-10 membered heteroaryl or heteroarylalkyl, each of which is substituted with unsubsti-

tuted $C_1$-$C_4$ alkyl, halo, unsubstituted $C_1$-$C_4$ alkoxy, unsubstituted $C_1$-$C_4$ haloalkyl, unsubstituted $C_1$-$C_4$ hydroxyalkyl, or unsubstituted $C_1$-$C_4$ haloalkoxy or hydroxy.

"Acylamino" refers to a radical -$NR^{22}C(O)R^{23}$, where each instance of $R^{22}$ and R23 is independently hydrogen, substituted or unsubstitued alkyl, substituted or unsubstitued alkenyl, substituted or unsubstitued alkynyl, substituted or unsubstitued carbocyclyl, substituted or unsubstituted heterocyclyl, substituted or unsubstituted aryl, or substituted or unsubstitued heteroaryl" as defined herein, or $R^{22}$ is an amino protecting group. Exemplary "acylamino" groups include, but are not limited to, formylamino, acetylamino, cyclohexylcarbonylamino, cyclohexylmethyl-carbonylamino, benzoylamino and benzylcarbonylamino. Particular exemplary "acylamino" groups are -$NR^{24}C(O)$-$C_1$-$C_8$ alkyl, $NR^{24}C(O)$-$(CH_2)_t$($C_6$-$C_{10}$ aryl), - $NR^{24}C(O)$-$(CH_2)_t$(5-10 membered heteroaryl), -$NR^{24}C(O)$-$(CH_2)_t$($C_3$-$C_{10}$ cycloalkyl), and - $NR^{24}C(O)$-$(CH_2)_t$(4-10 membered heterocyclyl), wherein t is an integer from 0 to 4, and each $R^{24}$ independently represents H or $C_1$-$C_8$ alkyl. In certain embodiments, $R^{25}$ is H, $C_1$-$C_8$ alkyl, substituted with halo or hydroxy; $C_3$-$C_{10}$ cycloalkyl, 4-10 membered heterocyclyl, $C_6$-$C_{10}$ aryl, arylalkyl, 5-10 membered heteroaryl or heteroarylalkyl, each of which is substituted with unsubstituted $C_1$-$C_4$ alkyl, halo, unsubstituted $C_1$-$C_4$ alkoxy, unsubstituted $C_1$-$C_4$ haloalkyl, unsubstituted $C_1$-$C_4$ hydroxyalkyl, or unsubstituted $C_1$-$C_4$ haloalkoxy or hydroxy; and $R^{26}$ is H, $C_1$-$C_8$ alkyl, substituted with halo or hydroxy;

$C_3$-$C_{10}$ cycloalkyl, 4-10 membered heterocyclyl, $C_6$-$C_{10}$ aryl, arylalkyl, 5-10 membered heteroaryl or heteroarylalkyl, each of which is substituted with unsubstituted $C_1$-$C_4$ alkyl, halo, unsubstituted $C_1$-$C_4$ alkoxy, unsubstituted $C_1$-$C_4$haloalkyl, unsubstituted $C_1$-$C_4$ hydroxyalkyl, or unsubstituted $C_1$-$C_4$ haloalkoxy or hydroxyl; provided that at least one of $R^{25}$ and $R^{26}$ is other than H.

"Acyloxy" refers to a radical -$OC(O)R^{27}$, where $R^{27}$ is hydrogen, substituted or unsubstitued alkyl, substituted or unsubstitued alkenyl, substituted or unsubstitued alkynyl, substituted or unsubstitued carbocyclyl, substituted or unsubstituted heterocyclyl, substituted or unsubstituted aryl, or substituted or unsubstituted heteroaryl, as defined herein. Representative examples include, but are not limited to, formyl, acetyl, cyclohexylcarbonyl, cyclohexylmethylcarbonyl, benzoyl and benzylcarbonyl. In certain embodiments, $R^{28}$ is $C_1$-$C_8$ alkyl, substituted with halo or hydroxy; $C_3$-$C_{10}$ cycloalkyl, 4-10 membered heterocyclyl, $C_6$-$C_{10}$ aryl, arylalkyl, 5-10 membered heteroaryl or heteroarylalkyl, each of which is substituted with unsubstituted $C_1$-$C_4$ alkyl, halo, unsubstituted $C_1$-$C_4$ alkoxy, unsubstituted $C_1$-$C_4$ haloalkyl, unsubstituted $C_1$-$C_4$ hydroxyalkyl, or unsubstituted $C_1$-$C_4$ haloalkoxy or hydroxy.

"Alkoxy" refers to the group -$OR^{29}$ where $R^{29}$ is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, *i.e.* with between 1 and 6 carbon atoms. Further particular alkoxy groups have between 1 and 4 carbon atoms.

In certain embodiments, $R^{29}$ is a group that has 1 or more substituents, for instance, from 1 to 5 substituents, and particularly from 1 to 3 substituents, in particular 1 substituent, selected from the group consisting of amino, substituted amino, $C_6$-$C_{10}$ aryl, aryloxy, carboxyl, cyano, $C_3$-$C_{10}$ cycloalkyl, 4-10 membered heterocyclyl, halogen, 5-10 membered heteroaryl, hydroxyl, nitro, thioalkoxy, thioaryloxy, thiol, alkyl-S(O)-, aryl-S(O)-, alkyl-$S(O)_2$- and aryl-$S(O)_2$-. Exemplary 'substituted alkoxy' groups include, but are not limited to, -O-$(CH_2)_t$($C_6$-$C_{10}$ aryl), -O-$(CH_2)_t$(5-10 membered heteroaryl), -O-$(CH_2)_t$($C_3$-$C_{10}$ cycloalkyl), and - O-$(CH_2)_t$(4-10 membered heterocyclyl), wherein t is an integer from 0 to 4 and any aryl, heteroaryl, cycloalkyl or heterocyclyl groups present, may themselves be substituted by unsubstituted $C_1$-$C_4$ alkyl, halo, unsubstituted $C_1$-$C_4$ alkoxy, unsubstituted $C_1$-$C_4$ haloalkyl, unsubstituted $C_1$-$C_4$ hydroxyalkyl, or unsubstituted $C_1$-$C_4$ haloalkoxy or hydroxy. Particular exemplary 'substituted alkoxy' groups are -$OCF_3$, -$OCH_2CF_3$, -$OCH_2Ph$, -$OCH_2$-cyclopropyl, - $OCH_2CH_2OH$, and -$OCH_2CH_2NMe_2$.

"Amino" refers to the radical -$NH_2$.

"Substituted amino" refers to an amino group of the formula -$N(R^{38})_2$ wherein $R^{38}$ is hydrogen, substituted or unsubstituted alkyl, substituted or unsubstitued alkenyl, substituted or unsubstitued alkynyl, substituted or unsubstitued carbocyclyl, substituted or unsubstituted heterocyclyl, substituted or unsubstituted aryl, substituted or unsubstitued heteroaryl, or an amino protecting group, wherein at least one of $R^{38}$ is not a hydrogen. In certain embodiments, each $R^{38}$ is independently selected from: hydrogen, $C_1$-$C_8$ alkyl, $C_3$-$C_8$ alkenyl, $C_3$-$C_8$ alkynyl, $C_6$-$C_{10}$ aryl, 5-10 membered heteroaryl, 4-10 membered heterocyclyl, or $C_3$-$C_{10}$ cycloalkyl; or $C_1$-$C_8$ alkyl, substituted with halo or hydroxy; $C_3$-$C_8$ alkenyl, substituted with halo or hydroxy; $C_3$-$C_8$ alkynyl, substituted with halo or hydroxy, or -$(CH_2)_t$($C_6$-$C_{10}$ aryl), -$(CH_2)_t$(5-10 membered heteroaryl), - $(CH_2)_t$($C_3$-$C_{10}$ cycloalkyl), or -$(CH_2)_t$(4-10 membered heterocyclyl), wherein t is an integer between 0 and 8, each of which is substituted by unsubstituted $C_1$-$C_4$ alkyl, halo, unsubstituted $C_1$-$C_4$ alkoxy, unsubstituted $C_1$-$C_4$ haloalkyl, unsubstituted $C_1$-$C_4$ hydroxyalkyl, or unsubstituted $C_1$-$C_4$ haloalkoxy or hydroxy; or both $R^{38}$ groups are joined to form an alkylene group.

Exemplary 'substituted amino' groups are -$NR^{39}$-$C_1$-$C_8$ alkyl, -$NR^{39}$-$(CH_2)_t$($C_6$-$C_{10}$ aryl), -$NR^{39}$-$(CH_2)_t$(5-10 membered heteroaryl), -$NR^{39}$-$(CH_2)_t$($C_3$-$C_{10}$ cycloalkyl), and - $NR^{39}$-$(CH_2)_t$(4-10 membered heterocyclyl), wherein t is an integer from 0 to 4, for instance 1 or 2, each $R^{39}$ independently represents H or $C_1$-$C_8$ alkyl; and any alkyl groups

present, may themselves be substituted by halo, substituted or unsubstituted amino, or hydroxy; and any aryl, heteroaryl, cycloalkyl, or heterocyclyl groups present, may themselves be substituted by unsubstituted $C_1$-$C_4$ alkyl, halo, unsubstituted $C_1$-$C_4$ alkoxy, unsubstituted $C_1$-$C_4$ haloalkyl, unsubstituted $C_1$-$C_4$ hydroxyalkyl, or unsubstituted $C_1$-$C_4$ haloalkoxy or hydroxy. For the avoidance of doubt the term ‘substituted amino’ includes the groups alkylamino, substituted alkylamino, alkylarylamino, substituted alkylarylamino, arylamino, substituted arylamino, dialkylamino, and substituted dialkylamino as defined below. Substituted amino encompasses both monosubstituted amino and disubstituted amino groups.

"Azido" refers to the radical $-N_3$.

"Carbamoyl" or "amido" refers to the radical $-C(O)NH_2$.

"Substituted carbamoyl" or "substituted amido" refers to the radical $-C(O)N(R^{62})_2$ wherein each $R^{62}$ is independently hydrogen, substituted or unsubstituted alkyl, substituted or unsubstitued alkenyl, substituted or unsubstitued alkynyl, substituted or unsubstitued carbocyclyl, substituted or unsubstituted heterocyclyl, substituted or unsubstituted aryl, substituted or unsubstitued heteroaryl, or an amino protecting group, wherein at least one of $R^{62}$ is not a hydrogen. In certain embodiments, $R^{62}$ is selected from H, $C_1$-$C_8$ alkyl, $C_3$-$C_{10}$ cycloalkyl, 4-10 membered heterocyclyl, $C_6$-$C_{10}$ aryl, aralkyl, 5-10 membered heteroaryl, and heteroaralkyl; or $C_1$-$C_8$ alkyl substituted with halo or hydroxy; or $C_3$-$C_{10}$ cycloalkyl, 4-10 membered heterocyclyl, $C_6$-$C_{10}$ aryl, aralkyl, 5-10 membered heteroaryl, or heteroaralkyl, each of which is substituted by unsubstituted $C_1$-$C_4$ alkyl, halo, unsubstituted $C_1$-$C_4$ alkoxy, unsubstituted $C_1$-$C_4$ haloalkyl, unsubstituted $C_1$-$C_4$ hydroxyalkyl, or unsubstituted $C_1$-$C_4$ haloalkoxy or hydroxy; provided that at least one $R^{62}$ is other than H.

Exemplary ‘substituted carbamoyl’ groups include, but are not limited to, $-C(O)\ NR^{64}$-$C_1$-$C_8$ alkyl, $-C(O)NR^{64}-(CH_2)_t(C_6$-$C_{10}$ aryl), $-C(O)N^{64}-(CH_2)_t$(5-10 membered heteroaryl), $-C(O)NR^{64}-(CH_2)_t(C_3$-$C_{10}$ cycloalkyl), and $-C(O)NR^{64}-(CH_2)_t$(4-10 membered heterocyclyl), wherein t is an integer from 0 to 4, each $R^{64}$ independently represents H or $C_1$-$C_8$ alkyl and any aryl, heteroaryl, cycloalkyl or heterocyclyl groups present, may themselves be substituted by unsubstituted $C_1$-$C_4$ alkyl, halo, unsubstituted $C_1$-$C_4$ alkoxy, unsubstituted $C_1$-$C_4$ haloalkyl, unsubstituted $C_1$-$C_4$ hydroxyalkyl, or unsubstituted $C_1$-$C_4$ haloalkoxy or hydroxy.

’Carboxy’ refers to the radical -C(O)OH.

"Cyano" refers to the radical -CN.

"Halo" or "halogen" refers to fluoro (F), chloro (Cl), bromo (Br), and iodo (I). In certain embodiments, the halo group is either fluoro or chloro. In further embodiments, the halo group is iodo.

"Hydroxy" refers to the radical -OH.

"Nitro" refers to the radical $-NO_2$.

"Cycloalkylalkyl" refers to an alkyl radical in which the alkyl group is substituted with a cycloalkyl group. Typical cycloalkylalkyl groups include, but are not limited to, cyclopropylmethyl, cyclobutylmethyl, cyclopentylmethyl, cyclohexylmethyl, cycloheptylmethyl, cyclooctylmethyl, cyclopropylethyl, cyclobutylethyl, cyclopentylethyl, cyclohexylethyl, cycloheptylethyl, and cyclooctylethyl, and the like.

"Heterocyclylalkyl" refers to an alkyl radical in which the alkyl group is substituted with a heterocyclyl group. Typical heterocyclylalkyl groups include, but are not limited to, pyrrolidinylmethyl, piperidinylmethyl, piperazinylmethyl, morpholinylmethyl, pyrrolidinylethyl, piperidinylethyl, piperazinylethyl, morpholinylethyl, and the like.

"Cycloalkenyl" refers to substituted or unsubstituted carbocyclyl group having from 3 to 10 carbon atoms and having a single cyclic ring or multiple condensed rings, including fused and bridged ring systems and having at least one and particularly from 1 to 2 sites of olefinic unsaturation. Such cycloalkenyl groups include, by way of example, single ring structures such as cyclohexenyl, cyclopentenyl, cyclopropenyl, and the like.

"Fused cycloalkenyl" refers to a cycloalkenyl having two of its ring carbon atoms in common with a second aliphatic or aromatic ring and having its olefinic unsaturation located to impart aromaticity to the cycloalkenyl ring.

"Ethenyl" refers to substituted or unsubstituted -(C=C)-.

"Ethylene" refers to substituted or unsubstituted -(C-C)-.

"Ethynyl" refers to -(C=C)-.

"Nitrogen-containing heterocyclyl" group means a 4- to 7- membered non-aromatic cyclic group containing at least one nitrogen atom, for example, but without limitation, morpholine, piperidine (*e.g.* 2-piperidinyl, 3-piperidinyl and 4-piperidinyl), pyrrolidine (*e.g.* 2-pyrrolidinyl and 3-pyrrolidinyl), azetidine, pyrrolidone, imidazoline, imidazolidinone, 2-pyrazoline, pyrazolidine, piperazine, and N-alkyl piperazines such as N-methyl piperazine. Particular examples include azetidine, piperidone and piperazone.

"Thioketo" refers to the group =S.

Alkyl, alkenyl, alkynyl, carbocyclyl, heterocyclyl, aryl, and heteroaryl groups, as defined herein, are optionally substituted (*e.g.*, "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group (*e.g.*, a carbon or nitrogen atom) is replaced with a permissible substituent, *e.g.,* a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety.

Exemplary carbon atom substituents include, but are not limited to, halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3^+X^-$, $-N(OR^{cc})R^{bb}$, - SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, - $C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, - $C(=NR^{bb})R^{aa}$, $-C(=NR^{bb})OR^{aa}$, $-OC(=NR^{bb})R^{aa}$, $-OC(=NR^{bb})OR^{aa}$, $-C(=NR^{bb})N(R^{bb})_2$, - $OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, - $SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$ $-C(=S)N(R^{bb})_2$, - $C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, - $SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, - $P(=O)_2N(R^{bb})_2$, $-OP(=O)_2N(R^{bb})_2$, $-P(=O)(NR^{bb})_2$, $-OP(=O)(NR^{bb})_2$, $-NR^{bb}P(=O)(OR^{cc})_2$, - $NR^{bb}P(=O)(NR^{bb})_2$, $-P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, $C_{1-10}$ alkyl, $C_{1-10}$ perhaloalkyl, $C_{2-10}$ alkenyl, $C_{2-10}$ alkynyl, $C_{3-10}$ carbocyclyl, 3-14 membered heterocyclyl, $C_{6-14}$ aryl, and 5-14 membered heteroaryl, wherein each alkyl, alkenyl, alkynyl, carbocyclyl, heterocyclyl, aryl, and heteroaryl is independently substituted with 0, 1, 2, 3, 4, or 5 $R^{dd}$ groups;

or two geminal hydrogens on a carbon atom are replaced with the group =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$, or $=NOR^{cc}$;

each instance of $R^{aa}$ is, independently, selected from $C_{1-10}$ alkyl, $C_{1-10}$ perhaloalkyl, $C_{2-10}$ alkenyl, $C_{2-10}$ alkynyl, $C_{3-10}$ carbocyclyl, 3-14 membered heterocyclyl, $C_{6-14}$ aryl, and 5-14 membered heteroaryl, or two $R^{aa}$ groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 $R^{dd}$ groups;

each instance of $R^{bb}$ is, independently, selected from hydrogen, -OH, $-OR^{aa}$, $-N(R^{cc})_2$, -CN, - $C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, - $P(=O)(R^{aa})_2$, $-P(=O)_2N(R^{cc})_2$, $-P(=O)(NR^{cc})_2$, $C_{1-10}$ alkyl, $C_{1-10}$ perhaloalkyl, $C_{2-10}$ alkenyl, $C_{2-10}$ alkynyl, $C_{3-10}$ carbocyclyl, 3-14 membered heterocyclyl, $C_{6-14}$ aryl, and 5-14 membered heteroaryl, or two $R^{bb}$ groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 $R^{dd}$ groups;

each instance of $R^{cc}$ is, independently, selected from hydrogen, $C_{1-10}$ alkyl, $C_{1-10}$ perhaloalkyl, $C_{2-10}$ alkenyl, $C_{2-10}$ alkynyl, $C_{3-10}$ carbocyclyl, 3-14 membered heterocyclyl, $C_{6-14}$ aryl, and 5-14 membered heteroaryl, or two $R^{cc}$ groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 $R^{dd}$ groups;

each instance of $R^{dd}$ is, independently, selected from halogen, -CN, $-NO_2$, $-N_3$, $-SO_2H$, $-SO_3H$, -OH, $-OR^{ee}$, $-ON(R^{ff})_2$, $-N(R^{ff})_2$, $-N(R^{ff})_3^+X^-$, $-N(OR^{ee})R^{ff}$, -SH, $-SR^{ee}$, $-SSR^{ee}$, $-C(=O)R^{ee}$, - $CO_2H$, $-CO_2R^{ee}$, $-OC(=O)R^{ee}$, $-OCO_2R^{ee}$, $-C(=O)N(R^{ff})_2$, $-OC(=O)N(R^{ff})_2$, $-NR^{ff}C(=O)R^{ee}$, - $NR^{ff}CO_2R^{ee}$, $-NR^{ff}C(=O)N(R^{ff})_2$, $-C(=NR^{ff})OR^{ee}$, $-OC(=NR^{ff})R^{ee}$, $-OC(=NR^{ff})OR^{ee}$, - $C(=NR^{ff})N(R^{ff})_2$, $-OC(=NR^{ff})N(R^{ff})_2$, $-NR^{ff}C(=NR^{ff})N(R^{ff})_2$,$-NR^{ff}SO_2R^{ee}$, $-SO_2N(R^{ff})_2$, - $SO_2R^{ee}$, $-SO_2OR^{ee}$, $-OSO_2R^{ee}$, $-S(=O)R^{ee}$, $-Si(R^{ee})_3$, $-OSi(R^{ee})_3$, $-C(=S)N(R^{ff})_2$, $-C(=O)SR^{ee}$, - $C(=S)SR^{ee}$, $-SC(=S)SR^{ee}$, $-P(=O)_2R^{ee}$, $-P(=O)(R^{ee})_2$, $-OP(=O)(R^{ee})_2$, $-OP(=O)(OR^{ee})_2$, $C_{1-6}$ alkyl, $C_{1-6}$ perhaloalkyl, $C_{2-6}$ alkenyl, $C_{2-6}$ alkynyl, $C_{3-10}$ carbocyclyl, 3-10 membered heterocyclyl, $C_{6-10}$ aryl, 5-10 membered heteroaryl, wherein each alkyl, alkenyl, alkynyl, carbocyclyl, heterocyclyl, aryl, and heteroaryl is independently substituted with 0, 1, 2, 3, 4, or 5 $R^{gg}$ groups, or two geminal $R^{dd}$ substituents can be joined to form =O or =S;

each instance of $R^{ee}$ is, independently, selected from $C_{1-6}$ alkyl, $C_{1-6}$ perhaloalkyl, $C_{2-6}$ alkenyl, $C_{2-6}$ alkynyl, $C_{3-10}$ carbocyclyl, $C_{6-10}$ aryl, 3-10 membered heterocyclyl, and 3-10 membered heteroaryl, wherein each alkyl, alkenyl, alkynyl, carbocyclyl, heterocyclyl, aryl, and heteroaryl is independently substituted with 0, 1, 2, 3, 4, or 5 $R^{gg}$ groups;

each instance of $R^{ff}$ is, independently, selected from hydrogen, $C_{1-6}$ alkyl, $C_{1-6}$ perhaloalkyl, $C_{2-6}$ alkenyl, $C_{2-6}$ alkynyl, $C_{3-10}$ carbocyclyl, 3-10 membered heterocyclyl, $C_{6-10}$ aryl and 5-10 membered heteroaryl, or two $R^{ff}$ groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 $R^{gg}$ groups;

and each instance of $R^{gg}$ is, independently, halogen, -CN, $-NO_2$, $-N_3$, $-SO_2H$, $-SO_3H$, -OH, $-OC_{1-6}$ alkyl, $-ON(C_{1-6}$ alkyl$)_2$, $-N(C_{1-6}$ alkyl$)_2$, $-N(C_{1-6}$ alkyl$)_3^+X^-$, $-NH(C_{1-6}$ alkyl$)_2^+X^-$, $-NH_2(C_{1-6}$ alkyl) $^+X^-$, $-NH_3^+X^-$, $-N(OC_{1-6}$ alkyl)($C_{1-6}$ alkyl), -N(OH)($C_{1-6}$ alkyl), -NH(OH), -SH, $-SC_{1-6}$ alkyl, -SS($C_{1-6}$ alkyl), -C(=O)($C_{1-6}$ alkyl), $-CO_2H$, $-CO_2(C_{1-6}$ alkyl), -OC(=O)($C_{1-6}$ alkyl), - $OCO_2(C_{1-6}$ alkyl), $-C(=O)NH_2$, $-C(=O)N(C_{1-6}$ alkyl$)_2$, -OC(=O)NH($C_{1-6}$ alkyl), -NHC(=O)( $C_{1-6}$

alkyl), $-N(C_{1-6}$ alkyl)C(=O)( $C_{1-6}$ alkyl), $-NHCO_2(C_{1-6}$ alkyl), $-NHC(=O)N(C_{1-6}$ alkyl$)_2$, - NHC(=O)NH($C_{1-6}$ alkyl), $-NHC(=O)NH_2$, -C(=NH)O($C_{1-6}$ alkyl),-OC(=NH)($C_{1-6}$ alkyl), - OC(=NH)O$C_{1-6}$ alkyl, -C(=NH)N($C_{1-6}$ alkyl$)_2$, -C(=NH)NH($C_{1-6}$ alkyl), $-C(=NH)NH_2$, - OC(=NH)N($C_{1-6}$ alkyl$)_2$, -OC(NH)NH($C_{1-6}$ alkyl), $-OC(NH)NH_2$, -NHC(NH)N($C_{1-6}$ alkyl$)_2$, - $NHC(=NH)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$,$-SO_2C_{1-6}$ alkyl, $-SO_2OC_{1-6}$ alkyl, $-OSO_2C_{1-6}$ alkyl, $-SOC_{1-6}$ alkyl, -Si($C_{1-6}$ alkyl$)_3$, -OSi($C_{1-6}$ alkyl$)_3$ -C(=S)N($C_{1-6}$ alkyl$)_2$, C(=S)NH($C_{1-6}$ alkyl), $C(=S)NH_2$, -C(=O)S($C_{1-6}$ alkyl), - C(=S)S$C_{1-6}$ alkyl, -SC(=S)S$C_{1-6}$ alkyl, $-P(=O)_2(C_{1-6}$ alkyl), -P(=O)($C_{1-6}$ alkyl$)_2$, -OP(=O)($C_{1-6}$ alkyl$)_2$, -OP(=O)(O$C_{1-6}$ alkyl$)_2$, $C_{1-6}$ alkyl, $C_{1-6}$ perhaloalkyl, $C_{2-6}$ alkenyl, $C_{2-6}$ alkynyl, $C_{3-10}$ carbocyclyl, $C_{6-10}$ aryl, 3-10 membered heterocyclyl, 5-10 membered heteroaryl; or two geminal $R^{gg}$ substituents can be joined to form =O or =S; wherein $X^-$ is a counterion.

A "counterion" or "anionic counterion" is a negatively charged group associated with a cationic quaternary amino group in order to maintain electronic neutrality. Exemplary counterions include halide ions (*e.g.*, $F^-$, $Cl^-$, $Br^-$, $I^-$), $NO_3^-$, $ClO_4^-$, $OH^-$, $H_2PO_4^-$, $HSO_4^-$, sulfonate ions (*e.g.*, methansulfonate, trifluoromethanesulfonate, p-toluenesulfonate, benzenesulfonate, 10-camphor sulfonate, naphthalene-2-sulfonate, naphthalene-1-sulfonic acid-5-sulfonate, ethan-1-sulfonic acid-2-sulfonate, and the like), and carboxylate ions (*e.g.*, acetate, ethanoate, propanoate, benzoate, glycerate, lactate, tartrate, glycolate, and the like).

Nitrogen atoms can be substituted or unsubstituted as valency permits, and include primary, secondary, tertiary, and quarternary nitrogen atoms. Exemplary nitrogen atom substitutents include, but are not limited to, hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{bb})R^{aa}$, $-C(=NR^{cc})OR^{aa}$, $-C(=NR^{cc})N(R^{cc})_2$, - $SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, $C_{1-10}$ alkyl, $C_{1-10}$ perhaloalkyl, $C_{2-10}$ alkenyl, $C_{2-10}$ alkynyl, $C_{3-10}$ carbocyclyl, 3-14 membered heterocyclyl, $C_{6-14}$ aryl, and 5-14 membered heteroaryl, or two $R^{cc}$ groups attached to a nitrogen atom are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 $R^{dd}$ groups, and wherein $R^{aa}$, $R^{bb}$, $R^{cc}$ and $R^{dd}$ are as defined above.

In certain embodiments, the substituent present on a nitrogen atom is a nitrogen protecting group (also referred to as an amino protecting group). Nitrogen protecting groups include, but are not limited to, -OH, $-OR^{aa}$, $-N(R^{cc})_2$, $-C(=O)R^{aa}$, $-C(=O)N(R^{cc})_2$, $-CO_2R^{aa}$, - $SO_2R^{aa}$, $-C(=NR^{cc})R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, - $SOR^{aa}$, $-C(=S)N(R^{cc})_2$, $-C(=O)SR^{cc}$, $-C(=S)SR^{cc}$, $C_{1-10}$ alkyl (e.g., aralkyl, heteroaralkyl), $C_{2-10}$ alkenyl, $C_{2-10}$ alkynyl, $C_{3-10}$ carbocyclyl, 3-14 membered heterocyclyl, $C_{6-14}$ aryl, and 5-14 membered heteroaryl groups, wherein each alkyl, alkenyl, alkynyl, carbocyclyl, heterocyclyl, aralkyl, aryl, and heteroaryl is independently substituted with 0, 1, 2, 3, 4, or 5 $R^{dd}$ groups, and wherein $R^{aa}$, $R^{bb}$, $R^{cc}$ and $R^{dd}$ are as defined herein. Nitrogen protecting groups are well known in the art and include those described in detail in Protecting Groups in Organic Synthesis, T. W. Greene and P. G. M. Wuts, 3rd edition, John Wiley & Sons, 1999.

For example, nitrogen protecting groups such as amide groups (*e.g.*, $-C(=O)R^{aa}$) include, but are not limited to, formamide, acetamide, chloroacetamide, trichloroacetamide, trifluoroacetamide, phenylacetamide, 3-phenylpropanamide, picolinamide, 3-pyridylcarboxamide, *N*-benzoylphenylalanyl derivative, benzamide, *p*-phenylbenzamide, *o*-nitophenylacetamide, *o*-nitrophenoxyacetamide, acetoacetamide, (*N'*-dithiobenzyloxyacylamino)acetamide, 3-(*p*-hydroxyphenyl)propanamide, 3-(*o*-nitrophenyl)propanamide, 2-methyl-2-(*o*-nitrophenoxy)propanamide, 2-methyl-2-(*o*-phenylazophenoxy)propanamide, 4-chlorobutanamide, 3-methyl-3-nitrobutanamide, *o*-nitrocinnamide, *N*-acetylmethionine derivative, *o*-nitrobenzamide and *o*-(benzoyloxymethyl)benzamide.

Nitrogen protecting groups such as carbamate groups (*e.g.*, $-C(=O)OR^{aa}$) include, but are not limited to, methyl carbamate, ethyl carbamante, 9-fluorenylmethyl carbamate (Fmoc), 9-(2-sulfo)fluorenylmethyl carbamate, 9-(2,7-dibromo)fluoroenylmethyl carbamate, 2,7-di-*t*-butyl-[9-(10,10-dioxo-10,10,10,10-tetrahydrothioxanthyl)]methyl carbamate (DBD-Tmoc), 4-methoxyphenacyl carbamate (Phenoc), 2,2,2-trichloroethyl carbamate (Troc), 2-trimethylsilylethyl carbamate (Teoc), 2-phenylethyl carbamate (hZ), 1-(1-adamantyl)-1-methylethyl carbamate (Adpoc), 1,1-dimethyl-2-haloethyl carbamate, 1,1-dimethyl-2,2-dibromoethyl carbamate (DB-*t*-BOC), 1,1-dimethyl-2,2,2-trichloroethyl carbamate (TCBOC), 1-methyl-1-(4-biphenylyl)ethyl carbamate (Bpoc), 1-(3,5-di-*t*-butylphenyl)-1-methylethyl carbamate (*t*-Bumeoc), 2-(2'- and 4'-pyridyl)ethyl carbamate (Pyoc), 2-(*N,N*-dicyclohexylcarboxamido)ethyl carbamate, *t*-butyl carbamate (BOC), 1-adamantyl carbamate (Adoc), vinyl carbamate (Voc), allyl carbamate (Alloc), 1-isopropylallyl carbamate (Ipaoc), cinnamyl carbamate (Coc), 4-nitrocinnamyl carbamate (Noc), 8-quinolyl carbamate, *N*-hydroxypiperidinyl carbamate, alkyldithio carbamate, benzyl carbamate (Cbz), *p*-methoxybenzyl carbamate (Moz), *p*-nitobenzyl carbamate, *p*-bromobenzyl carbamate, *p*-chlorobenzyl carbamate, 2,4-dichlorobenzyl carbamate, 4-methylsulfinylbenzyl carbamate (Msz), 9-anthrylmethyl carbamate, diphenylmethyl carbamate, 2-methylthioethyl carbamate, 2-methylsulfonylethyl carbamate, 2-(*p*-toluenesulfonyl)ethyl carbamate, [2-(1,3-dithianyl)]methyl carbamate (Dmoc), 4-methylthiophenyl carbamate (Mtpc), 2,4-dimethylthiophenyl carbamate (Bmpc), 2-phosphonioethyl carbamate (Peoc), 2-triphenylphosphonioisopropyl carbamate (Ppoc), 1,1-dimethyl-2-cyanoethyl carbamate, *m*-chloro-p-acyloxybenzyl carbamate, *p*-(dihydroxyboryl)benzyl carbamate, 5-benzisoxazolylmethyl carbamate, 2-(trifluoromethyl)-6-chromonylmethyl carbamate

(Tcroc), *m*-nitrophenyl carbamate, 3,5-dimethoxybenzyl carbamate, *o*-nitrobenzyl carbamate, 3,4-dimethoxy-6-nitrobenzyl carbamate, phenyl(*o*-nitrophenyl)methyl carbamate, *t*-amyl carbamate, *S*-benzyl thiocarbamate, *p*-cyanobenzyl carbamate, cyclobutyl carbamate, cyclohexyl carbamate, cyclopentyl carbamate, cyclopropylmethyl carbamate, *p*-decyloxybenzyl carbamate, 2,2-dimethoxyacylvinyl carbamate, *o*-(*N,N*-dimethylcarboxamido)benzyl carbamate, 1,1-dimethyl-3-(*N,N*-dimethylcarboxamido)propyl carbamate, 1,1-dimethylpropynyl carbamate, di(2-pyridyl)methyl carbamate, 2-furanylmethyl carbamate, 2-iodoethyl carbamate, isoborynl carbamate, isobutyl carbamate, isonicotinyl carbamate, *p*-(*p*'-methoxyphenylazo)benzyl carbamate, 1-methylcyclobutyl carbamate, 1-methylcyclohexyl carbamate, 1-methyl-1-cyclopropylmethyl carbamate, 1-methyl-1-(3,5-dimethoxyphenyl)ethyl carbamate, 1-methyl-1-(*p*-phenylazophenyl)ethyl carbamate, 1-methyl-1-phenylethyl carbamate, 1-methyl-1-(4-pyridyl)ethyl carbamate, phenyl carbamate, *p*-(phenylazo)benzyl carbamate, 2,4,6-tri-*t*-butylphenyl carbamate, 4-(trimethylammonium)benzyl carbamate, and 2,4,6-trimethylbenzyl carbamate.

Nitrogen protecting groups such as sulfonamide groups (*e.g.*, $-S(=O)_2R^{aa}$) include, but are not limited to, *p*-toluenesulfonamide (Ts), benzenesulfonamide, 2,3,6,-trimethyl-4-methoxybenzenesulfonamide (Mtr), 2,4,6-trimethoxybenzenesulfonamide (Mtb), 2,6-dimethyl-4-methoxybenzenesulfonamide (Pme), 2,3,5,6-tetramethyl-4-methoxybenzenesulfonamide (Mte), 4-methoxybenzenesulfonamide (Mbs), 2,4,6-trimethylbenzenesulfonamide (Mts), 2,6-dimethoxy-4-methylbenzenesulfonamide (iMds), 2,2,5,7,8-pentamethylchroman-6-sulfonamide (Pmc), methanesulfonamide (Ms), β-trimethylsilylethanesulfonamide (SES), 9-anthracenesulfonamide, 4-(4',8'-dimethoxynaphthylmethyl)benzenesulfonamide (DNMBS), benzylsulfonamide, trifluoromethylsulfonamide, and phenacylsulfonamide.

Other nitrogen protecting groups include, but are not limited to, phenothiazinyl-(10)-acyl derivative, *N'*-*p*-toluenesulfonylaminoacyl derivative, *N'*-phenylaminothioacyl derivative, *N*-benzoylphenylalanyl derivative, *N*-acetylmethionine derivative, 4,5-diphenyl-3-oxazolin-2-one, *N*-phthalimide, *N*-dithiasuccinimide (Dts), *N*-2,3-diphenylmaleimide, *N*-2,5-dimethylpyrrole, *N*-1,1,4,4-tetramethyldisilylazacyclopentane adduct (STABASE), 5-substituted 1,3-dimethyl-1,3,5-triazacyclohexan-2-one, 5-substituted 1,3-dibenzyl-1,3,5-triazacyclohexan-2-one, 1-substituted 3,5-dinitro-4-pyridone, *N*-methylamine, *N*-allylamine, *N*-[2-(trimethylsilyl)ethoxy]methylamine (SEM), *N*-3-acetoxypropylamine, *N*-(1-isopropyl-4-nitro-2-oxo-3-pyroolin-3-yl)amine, quaternary ammonium salts, *N*-benzylamine, *N*-di(4-methoxyphenyl)methylamine, *N*-5-dibenzosuberylamine, *N*-triphenylmethylamine (Tr), *N*-[(4-methoxyphenyl)diphenylmethyl]amine (MMTr), *N*-9-phenylfluorenylamine (PhF), *N*-2,7-dichloro-9-fluorenylmethyleneamine, *N*-ferrocenylmethylamino (Fcm), *N*-2-picolylamino *N'*-oxide, *N*-1,1-dimethylthiomethyleneamine, *N*-benzylideneamine, *N*-*p*-methoxybenzylideneamine, *N*-diphenylmethyleneamine, *N*-[(2-pyridyl)mesityl]methyleneamine, *N*-(*N'*,*N'*-dimethylaminomethylene)amine, *N,N'*-isopropylidenediamine, *N*-*p*-nitrobenzylideneamine, *N*-salicylideneamine, *N*-5-chlorosalicylideneamine, *N*-(5-chloro-2-hydroxyphenyl)phenylmethyleneamine, *N*-cyclohexylideneamine, *N*-(5,5-dimethyl-3-oxo-1-cyclohexenyl)amine, *N*-borane derivative, *N*-diphenylborinic acid derivative, *N*-[phenyl(pentaacylchromium- or tungsten)acyl]amine, *N*-copper chelate, *N*-zinc chelate, *N*-nitroamine, *N*-nitrosoamine, amine *N*-oxide, diphenylphosphinamide (Dpp), dimethylthiophosphinamide (Mpt), diphenylthiophosphinamide (Ppt), dialkyl phosphoramidates, dibenzyl phosphoramidate, diphenyl phosphoramidate, benzenesulfenamide, *o*-nitrobenzenesulfenamide (Nps), 2,4-dinitrobenzenesulfenamide, pentachlorobenzenesulfenamide, 2-nitro-4-methoxybenzenesulfenamide, triphenylmethylsulfenamide, and 3-nitropyridinesulfenamide (Npys).

In certain embodiments, the substituent present on an oxygen atom is an oxygen protecting group (also referred to as a hydroxyl protecting group). Oxygen protecting groups include, but are not limited to, $-R^{aa}$, $-N(R^{bb})_2$, $-C(=O)SR^{aa}$, $-C(=O)R^{aa}$, $-CO_2R^{aa}$, $-C(=O)N(R^{bb})_2$, $-C(=NR^{bb})R^{aa}$, $-C(=NR^{bb})OR^{aa}$, $-C(=NR^{bb})N(R^{bb})_2$, $-S(=O)R^{aa}$, $-SO_2R^{aa}$, $-Si(R^{aa})_3$, $-P(R^{cc})_2$, $-P(R^{cc})_3$, $-P(=O)_2R^{aa}$, $-P(=O)(R^{aa})_2$, $-P(=O)(OR^{cc})2$, $-P(=O)_2N(R^{bb})_2$, and $-P(=O)(NR^{bb})_2$, wherein $R^{aa}$, $R^{bb}$, and $R^{cc}$ are as defined herein. Oxygen protecting groups are well known in the art and include those described in detail in Protecting Groups in Organic Synthesis, T. W. Greene and P. G. M. Wuts, 3rd edition, John Wiley & Sons, 1999.

Exemplary oxygen protecting groups include, but are not limited to, methyl, methoxylmethyl (MOM), methylthiomethyl (MTM), *t*-butylthiomethyl, (phenyldimethylsilyl)methoxymethyl (SMOM), benzyloxymethyl (BOM), *p*-methoxybenzyloxymethyl (PMBM), (4-methoxyphenoxy)methyl (*p*-AOM), guaiacolmethyl (GUM), *t*-butoxymethyl, 4-pentenyloxymethyl (POM), siloxymethyl, 2-methoxyethoxymethyl (MEM), 2,2,2-trichloroethoxymethyl, bis(2-chloroethoxy)methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 4-methoxytetrahydrothiopyranyl S,S-dioxide, 1-[(2-chloro-4-methyl)phenyl]-4-methoxypiperidin-4-yl (CTMP), 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *t*-butyl, allyl, *p*-chlorophenyl, *p*-methoxyphenyl, 2,4-dinitrophenyl, benzyl (Bn), *p*-methoxybenzyl, 3,4-dimethoxybenzyl, o-nitrobenzyl, p-nitrobenzyl, *p*-halobenzyl, 2,6-dichlorobenzyl, *p*-cyanobenzyl, *p*-phenylbenzyl, 2-picolyl, 4-picolyl, 3-methyl-2-picolyl *N*-oxido, diphenylmethyl, *p,p* '-dinitrobenzhydryl, 5-dibenzosuberyl, triphenylmethyl, α-naphthyldiphenylmethyl, *p*-methoxyphenyldiphenylmethyl, di(*p*-methoxyphenyl)phenylmethyl, tri(*p*-methoxyphenyl)methyl, 4-(4'-bromophenacyloxyphenyl)diphenylmethyl, 4,4',4''-tris(4,5-dichlorophthalimidophenyl)methyl, 4,4',4''-tris(levulinoyloxyphenyl)methyl, 4,4',4''-tris(benzoyloxyphenyl)methyl, 3-(imidazol-1-yl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodisulfuran-2-yl, benzisothiazolyl S,S-dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, *t*-butyldimethylsilyl (TBDMS), *t*-butyldiphenylsilyl (TBDPS), tribenzylsilyl, tri-*p*-xylylsilyl, triphenylsilyl, diphenylmethylsilyl (DPMS), *t*-butylmethoxyphenylsilyl (TBMPS), formate, benzoylformate, acetate, chloroacetate, dichloroacetate, trichloroacetate, trifluoroacetate, methoxyacetate, triphenylmethoxyacetate, phenoxyacetate, *p*-chlorophenoxyacetate, 3-phenylpropionate, 4-oxopentanoate (levulinate), 4,4-(ethylenedithio)pentanoate (levulinoyldithioacetal), pivaloate, adamantoate, crotonate, 4-methoxycrotonate, benzoate, *p*-phenylbenzoate, 2,4,6-trimethylbenzoate (mesitoate), alkyl methyl carbonate, 9-fluorenylmethyl carbonate (Fmoc), alkyl ethyl carbonate, alkyl 2,2,2-trichloroethyl carbonate (Troc), 2-(trimethylsilyl)ethyl carbonate (TMSEC), 2-(phenylsulfonyl) ethyl carbonate (Psec), 2-(triphenylphosphonio) ethyl carbonate (Peoc), alkyl isobutyl carbonate, alkyl vinyl carbonate alkyl allyl carbonate, alkyl *p*-nitrophenyl carbonate, alkyl benzyl carbonate, alkyl *p*-methoxybenzyl carbonate, alkyl 3,4-dimethoxybenzyl carbonate, alkyl *o*-nitrobenzyl carbonate, alkyl *p*-nitrobenzyl carbonate, alkyl *S*-benzyl thiocarbonate, 4-ethoxy-1-napththyl carbonate, methyl dithiocarbonate, 2-iodobenzoate, 4-azidobutyrate, 4-nitro-4-methylpentanoate, *o*-(dibromomethyl)benzoate, 2-formylbenzenesulfonate, 2-(methylthiomethoxy)ethyl, 4-(methylthiomethoxy)butyrate, 2-(methylthiomethoxymethyl)benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (*E*)-2-methyl-2-butenoate, *o*-(methoxyacyl)benzoate, α-naphthoate, nitrate, alkyl *N,N,N',N'*-tetramethylphosphorodiamidate, alkyl *N*-phenylcarbamate, borate, dimethylphosphinothioyl, alkyl 2,4-dinitrophenylsulfenate, sulfate, methanesulfonate (mesylate), benzylsulfonate, and tosylate (Ts).

In certain embodiments, the substituent present on an sulfur atom is an sulfur protecting group (also referred to as a thiol protecting group). Sulfur protecting groups include, but are not limited to, $-R^{aa}$, $-N(R^{bb})_2$, $-C(=O)SR^{aa}$, $-C(=O)R^{aa}$, $-CO_2R^{aa}$, $-C(=O)N(R^{bb})_2$, $-C(=NR^{bb})R^{aa}$, $-C(=NR^{bb})OR^{aa}$, $-C(=NR^{bb})N(R^{bb})_2$, $-S(=O)R^{aa}$, $-SO_2R^{aa}$, $-Si(R^{aa})_3$, $-P(R^{cc})_2$, $-P(R^{cc})_3$, $-P(=O)_2R^{aa}$, $-P(=O)(R^{aa})_2$, $-P(=O)(OR^{cc})_2$, $-P(=O)_2N(R^{bb})_2$, and $-P(=O)(NR^{bb})_2$, wherein $R^{aa}$, $R^{bb}$, and $R^{cc}$ are as defined herein. Sulfur protecting groups are well known in the art and include those described in detail in Protecting Groups in Organic Synthesis, T. W. Greene and P. G. M. Wuts, 3rd edition, John Wiley & Sons, 1999.

"Compounds of the present invention", and equivalent expressions, are meant to embrace the compounds as hereinbefore described, in particular compounds according to any of the Formula herein recited and/or described, which expression includes the prodrugs, the pharmaceutically acceptable salts, and the solvates, *e.g.*, hydrates, where the context so permits. Similarly, reference to intermediates, whether or not they themselves are claimed, is meant to embrace their salts, and solvates, where the context so permits.

These and other exemplary substituents are described in more detail in the Detailed Description, Examples, and claims. The invention is not intended to be limited in any manner by the above exemplary listing of substituents.

*Other definitions*

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

"Pharmaceutically acceptable salt" refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.*, an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non toxic organic or inorganic acids, such as hydrochloride, hydrobromide, tartrate, mesylate, acetate, maleate, oxalate and the like. The term "pharmaceutically acceptable cation" refers to an acceptable cationic counter-ion of an acidic functional group. Such cations are exemplified by sodium, potassium, calcium, magnesium, ammonium, tetraalkylammonium cations, and the like (see, *e.g.,* Berge, et al., J. Pharm. Sci. 66(1): 1-79 (Jan."77).

"Pharmaceutically acceptable vehicle" refers to a diluent, adjuvant, excipient or carrier with which a compound

of the invention is administered.

"Pharmaceutically acceptable metabolically cleavable group" refers to a group which is cleaved *in vivo* to yield the parent molecule of the structural Formula indicated herein. Examples of metabolically cleavable groups include -COR, -COOR,-CONRR and $-CH_2OR$ radicals, where R is selected independently at each occurrence from alkyl, trialkylsilyl, carbocyclic aryl or carbocyclic aryl substituted with one or more of alkyl, halogen, hydroxy or alkoxy. Specific examples of representative metabolically cleavable groups include acetyl, methoxycarbonyl, benzoyl, methoxymethyl and trimethylsilyl groups.

"Prodrugs" refers to compounds, including derivatives of the compounds of the invention,which have cleavable groups and become by solvolysis or under physiological conditions the compounds of the invention that are pharmaceutically active *in vivo.* Such examples include, but are not limited to, choline ester derivatives and the like, N-alkylmorpholine esters and the like. Other derivatives of the compounds of this invention have activity in both their acid and acid derivative forms, but in the acid sensitive form often offers advantages of solubility, tissue compatibility, or delayed release in the mammalian organism (see, Bundgard, H., Design of Prodrugs, pp. 7-9, 21-24, Elsevier, Amsterdam 1985). Prodrugs include acid derivatives well know to practitioners of the art, such as, for example, esters prepared by reaction of the parent acid with a suitable alcohol, or amides prepared by reaction of the parent acid compound with a substituted or unsubstituted amine, or acid anhydrides, or mixed anhydrides. Simple aliphatic or aromatic esters, amides and anhydrides derived from acidic groups pendant on the compounds of this invention are particular prodrugs. In some cases it is desirable to prepare double ester type prodrugs such as (acyloxy)alkyl esters or ((alkoxycarbonyl)oxy)alkylesters. Particularly the $C_1$ to $C_8$ alkyl, $C_2$-$C_8$ alkenyl, $C_2$-$C_8$ alkynyl, aryl, $C_7$-$C_{12}$ substituted aryl, and $C_7$-$C_{12}$ arylalkyl esters of the compounds of the invention.

"Solvate" refers to forms of the compound that are associated with a solvent or water (also referred to as "hydrate"), usually by a solvolysis reaction. This physical association includes hydrogen bonding. Conventional solvents include water, ethanol, acetic acid and the like. The compounds of the invention may be prepared *e.g.* in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. "Solvate" encompasses both solution-phase and isolable solvates. Representative solvates include hydrates, ethanolates and methanolates.

A "subject" to which administration is contemplated includes, but is not limited to, humans (*i.e.*, a male or female of any age group, *e.g.,* a pediatric subject (*e.g,* infant, child, adolescent) or adult subject (*e.g.*, young adult, middle-aged adult or senior adult)) and/or a non-human animal, *e.g.*, a mammal such as primates (*e.g.*, cynomolgus monkeys, rhesus monkeys), cattle, pigs, horses, sheep, goats, rodents, cats, and/or dogs. In certain embodiments, the subject is a human. In certain embodiments, the subject is a non-human animal. The terms "human", "patient" and "subject" are used interchangeably herein.

"Therapeutically effective amount" means the amount of a compound that, when administered to a subject for treating a disease, is sufficient to effect such treatment for the disease. The "therapeutically effective amount" can vary depending on the compound, the disease and its severity, and the age, weight, *etc.,* of the subject to be treated.

"Preventing" or "prevention" refers to a reduction in risk of acquiring or developing a disease or disorder (*i.e.*, causing at least one of the clinical symptoms of the disease not to develop in a subject not yet exposed to a disease-causing agent, or predisposed to the disease in advance of disease onset.

The term "prophylaxis" is related to "prevention", and refers to a measure or procedure the purpose of which is to prevent, rather than to treat or cure a disease. Non-limiting examples of prophylactic measures may include the administration of vaccines; the administration of low molecular weight heparin to hospital patients at risk for thrombosis due, for example, to immobilization; and the administration of an anti-malarial agent such as chloroquine, in advance of a visit to a geographical region where malaria is endemic or the risk of contracting malaria is high.

"Treating" or "treatment" of any disease or disorder refers, in certain embodiments, to ameliorating the disease or disorder (*i.e.*, arresting the disease or reducing the manifestation, extent or severity of at least one of the clinical symptoms thereof). In another embodiment "treating" or "treatment" refers to ameliorating at least one physical parameter, which may not be discernible by the subject. In yet another embodiment, "treating" or "treatment" refers to modulating the disease or disorder, either physically, (*e.g.*, stabilization of a discernible symptom), physiologically, (*e.g.*, stabilization of a physical parameter), or both. In a further embodiment, "treating" or "treatment" relates to slowing the progression of the disease.

The references to methods of treatment and to method of delivery of an active agent in the summary and in the detailed description of the invention in this description are to be interpreted as references to the compounds, pharmaceutical compositions and medicaments of the present invention for use in a method for treatment of the human (or animal) body by therapy.

As used herein, the term "isotopic variant" refers to a compound that contains unnatural proportions of isotopes at one or more of the atoms that constitute such compound. For example, an "isotopic variant" of a compound can

contain one or more non-radioactive isotopes, such as for example, deuterium ($^2H$ or D), carbon-13 ($^{13}C$), nitrogen-15 ($^{15}N$), or the like. It will be understood that, in a compound where such isotopic substitution is made, the following atoms, where present, may vary, so that for example, any hydrogen may be $^2H$/D, any carbon may be $^{13}C$, or any nitrogen may be $^{15}N$, and that the presence and placement of such atoms may be determined within the skill of the art. Likewise, the invention may include the preparation of isotopic variants with radioisotopes, in the instance for example, where the resulting compounds may be used for drug and/or substrate tissue distribution studies. The radioactive isotopes tritium, *i.e.,* $^3H$, and carbon-14, *i.e.,* $^{14}C$, are particularly useful for this purpose in view of their ease of incorporation and ready means of detection. Further, compounds may be prepared that are substituted with positron emitting isotopes, such as $^{11}C$, $^{18}F$, $^{15}O$ and $^{13}N$, and would be useful in Positron Emission Topography (PET) studies for examining substrate receptor occupancy. All isotopic variants of the compounds provided herein, radioactive or not, are intended to be encompassed within the scope of the invention.

It is also to be understood that compounds that have the same molecular formula but differ in the nature or sequence of bonding of their atoms or the arrangement of their atoms in space are termed "isomers". Isomers that differ in the arrangement of their atoms in space are termed "stereoisomers".

Stereoisomers that are not mirror images of one another are termed "diastereomers" and those that are non-superimposable mirror images of each other are termed "enantiomers". When a compound has an asymmetric center, for example, when it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (*i.e.,* as (+) or (-)-isomers respectively). A chiral compound can exist as either individual enantiomer or as a mixture thereof. A mixture containing equal proportions of the enantiomers is called a "racemic mixture".

"Tautomers" refer to compounds that are interchangeable forms of a particular compound structure, and that vary in the displacement of hydrogen atoms and electrons. Thus, two structures may be in equilibrium through the movement of $\pi$ electrons and an atom (usually H). For example, enols and ketones are tautomers because they are rapidly interconverted by treatment with either acid or base. Another example of tautomerism is the aci- and nitro- forms of phenylnitromethane, which are likewise formed by treatment with acid or base. Tautomeric forms may be relevant to the attainment of the optimal chemical reactivity and biological activity of a compound of interest.

As used herein a pure enantiomeric compound is substantially free from other enantiomers or stereoisomers of the compound (*i.e.*, in enantiomeric excess). In other words, an "S" form of the compound is substantially free from the "R" form of the compound and is, thus, in enantiomeric excess of the "R" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the enantiomer. In certain embodiments, the weights are based upon total weight of all enantiomers or stereoisomers of the compound.

As used herein and unless otherwise indicated, the term "enantiomerically pure R-compound" refers to at least about 80% by weight R-compound and at most about 20% by weight S-compound, at least about 90% by weight R-compound and at most about 10% by weight S-compound, at least about 95% by weight R-compound and at most about 5% by weight S-compound, at least about 99% by weight R-compound and at most about 1% by weight S-compound, at least about 99.9% by weight R-compound or at most about 0.1% by weight S-compound. In certain embodiments, the weights are based upon total weight of compound.

As used herein and unless otherwise indicated, the term "enantiomerically pure S-compound" or "S-compound" refers to at least about 80% by weight S-compound and at most about 20% by weight R-compound, at least about 90% by weight S-compound and at most about 10% by weight R-compound, at least about 95% by weight S-compound and at most about 5% by weight R-compound, at least about 99% by weight S-compound and at most about 1% by weight R-compound or at least about 99.9% by weight S-compound and at most about 0.1% by weight R-compound. In certain embodiments, the weights are based upon total weight of compound.

In the compositions provided herein, an enantiomerically pure compound or a pharmaceutically acceptable salt, solvate, hydrate or prodrug thereof can be present with other active or inactive ingredients. For example, a pharmaceutical composition comprising enantiomerically pure R-compound can comprise, for example, about 90% excipient and about 10% enantiomerically pure R-compound. In certain embodiments, the enantiomerically pure R-compound in such compositions can, for example, comprise, at least about 95% by weight R-compound and at most about 5% by weight S-compound, by total weight of the compound. For example, a pharmaceutical composition comprising enantiomerically pure S-compound can comprise, for example, about 90% excipient and about 10% enantiomerically pure S-compound. In certain embodiments, the enantiomerically pure S-compound in such compositions can, for example, comprise, at least about 95% by weight S-compound and at most about 5% by weight R-compound, by total weight of

the compound. In certain embodiments, the active ingredient can be formulated with little or no excipient or carrier.

The compounds of this invention may possess one or more asymmetric centers; such compounds can therefore be produced as individual (R)- or (S)- stereoisomers or as mixtures thereof.

Unless indicated otherwise, the description or naming of a particular compound in the specification and claims is intended to include both individual enantiomers and mixtures, racemic or otherwise, thereof. The methods for the determination of stereochemistry and the separation of stereoisomers are well-known in the art.

One having ordinary skill in the art of organic synthesis will recognize that the maximum number of heteroatoms in a stable, chemically feasible heterocyclic ring, whether it is aromatic or non aromatic, is determined by the size of the ring, the degree of unsaturation and the valence of the heteroatoms. In general, a heterocyclic ring may have one to four heteroatoms so long as the heteroaromatic ring is chemically feasible and stable.

## DETAILED DESCRIPTION OF THE PREFERRED EMBODIMENTS

The composition or a formulation of the invention is defined in the claim 1. In certain aspects, provided herein are pharmaceutical compositions comprising of a bolaamphiphile complex.

In further aspects, provided herein are novel nano-sized vesicles comprising of bolaamphiphilic compounds.

In certain aspects, provided herein are novel bolaamphiphile complexes comprising one or more bolaamphiphilic compounds and a biologically active compound.

In further aspects, provided herein are novel formulations of biologically active compounds with one or more bolaamphiphilic compounds or with bolaamhphile vesicles.

In one embodiment, the bolaamphiphilic complex comprises one bolaamphiphilic compound. In another embodiment, the bolaamphiphilic complex comprises two bolaamphiphilic compounds.

The bolamphiphilic compound of the invention is GLH-20.

A bolaamphiphilic compound is a compound according to formula I:

$$HG^2 — L^1 — HG^1$$

I

or a pharmaceutically acceptable salt, solvate, hydrate, prodrug, stereoisomer, tautomer, isotopic variant, or N-oxide thereof, or a combination thereof;
wherein:

each $HG^1$ and $HG^2$ is independently a hydrophilic head group; and
$L^1$ is alkylene, alkenyl, heteroalkylene, or heteroalkenyl linker; unsubstituted or substituted with $C_1$-$C_{20}$ alkyl, hydroxyl, or oxo.

The pharmaceutically acceptable salt is a quaternary ammonium salt.

In one embodiment, with respect to the bolaamphiphilic compound of formula I, $L^1$ is heteroalkylene, or heteroalkenyl linker comprising C, N, and O atoms; unsubstituted or substituted with $C_1$-$C_{20}$ alkyl, hydroxyl, or oxo.

In another embodiment, with respect to the bolaamphiphilic compound of formula I, $L^1$ is

$$-O-L^2-C(O)-O-(CH_2)_{n4}-O-C(O)-L^3-O-,$$

or

$$-O-L^2-C(O)-O-(CH_2)_{n5}-O-C(O)-(CH_2)_{n6}-,$$

and wherein each $L^2$ and $L^3$ is $C_4$-$C_{20}$ alkenyl linker; unsubstituted or substituted with $C_1$-$C_8$ alkyl or hydroxy; and n4, n5, and n6 is independently an integer from 4-20.

In one embodiment, each $L^2$ and $L^3$ is independently $-C(R^1)-C(OH)-CH_2-(CH=CH)-(CH_2)_{n7}-$; $R^1$ is $C_1$-$C_8$ alkyl, and n7 is independently an integer from 4-20.

In another embodiment, with respect to the bolaamphiphilic compound of formula I, $L^1$ is $-O-(CH_2)_{n1}-O-C(O)-(CH_2)_{n2}-C(O)-O-(CH_2)_{n3}-O-$.

In another embodiment, with respect to the bolaamphiphilic compound of formula I, $L^1$ is

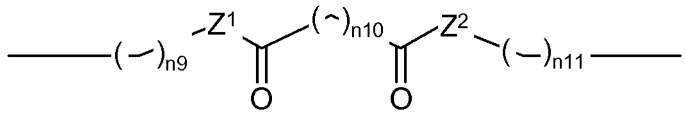

Linker AA ,

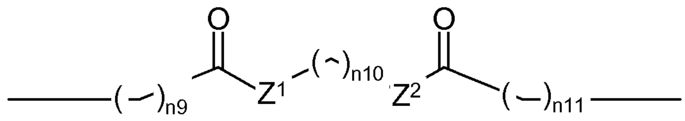

Linker BB ,

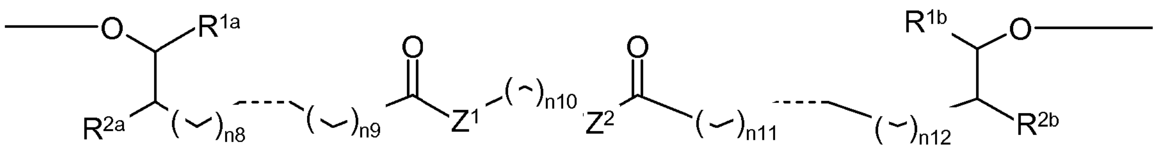

Linker CC

or

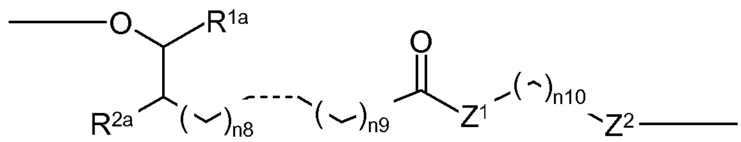

Linker DD ,

wherein:

each $Z^1$ and $Z^2$ is independently $-C(R^3)_2-$, $-N(R^3)-$ or -O-;
each $R^{1a}$, $R^{1b}$, $R^3$, and $R^4$ is independently H or $C_1$-$C_8$ alkyl;
each $R^{2a}$ and $R^{2b}$ is independently H , $C_1$-$C_8$ alkyl, OH, or alkoxy;
each n8, n9, n11, and n12 is independently an integer from 1-20;
n10 is an integer from 2-20; and
each dotted bond is independently a single or a double bond.

and wherein each methylene carbon is unsubstituted or substituted with $C_1$-$C_4$ alkyl; and each n1, n2, and n3 is independently an integer from 4-20.

In one embodiment, with respect to the bolaamphiphilic compound of formula I, the bolaamphiphilic compound is a compound according to formula II, III, IV, V, or VI:

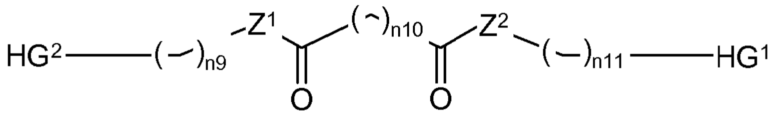

II

,

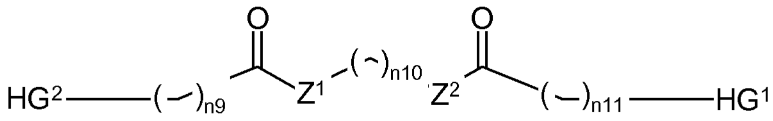

III

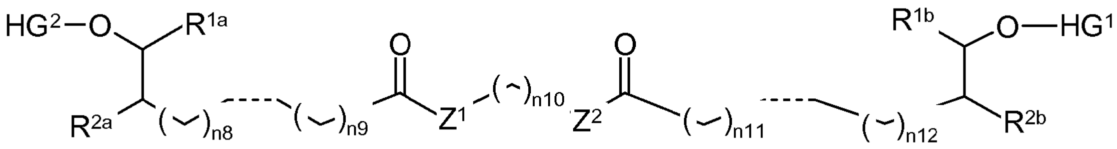

IV

,

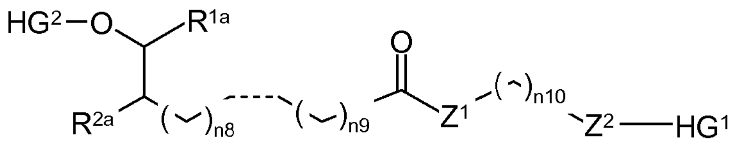

V

,

or

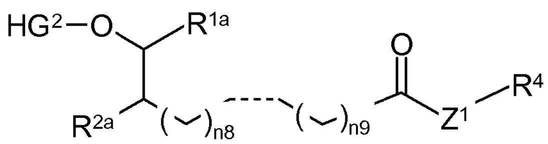

VI

or a pharmaceutically acceptable salt, solvate, hydrate, prodrug, stereoisomer, tautomer, isotopic variant, or N-oxide thereof, or a combination thereof;
wherein:

each $HG^1$ and $HG^2$ is independently a hydrophilic head group;
each $Z^1$ and $Z^2$ is independently $-C(R^3)_2-$, $-N(R^3)-$ or -O-;
each $R^{1a}$, $R^{1b}$, $R^3$, and $R^4$ is independently H or $C_1$-$C_8$ alkyl;
each $R^{2a}$ and $R^{2b}$ is independently H , $C_1$-$C_8$ alkyl, OH, alkoxy, or O-$HG^1$ or O-$HG^2$;
each n8, n9, n11, and n12 is independently an integer from 1-20;
n10 is an integer from 2-20; and
each dotted bond is independently a single or a double bond.

In one embodiment, with respect to the bolaamphiphilic compound of formula II, III, IV, V, or VI, each n9 and n11 is independently an integer from 2-12. In another embodiment, n9 and n11 is independently an integer from 4-8. In

a particular embodiment, each n9 and n11 is 7 or 11.

In one embodiment, with respect to the bolaamphiphilic compound of formula II, III, IV, V, or VI, each n8 and n12 is independently 1, 2, 3, or 4. In a particular embodiment, each n8 and n12 is 1.

In one embodiment, with respect to the bolaamphiphilic compound of formula II, III, IV, V, or VI, each $R^{2a}$ and $R^{2b}$ is independently H, OH, or alkoxy. In another embodiment, each $R^{2a}$ and $R^{2b}$ is independently H, OH, or OMe. In another embodiment, each $R^{2a}$ and $R^{2b}$ is independently-O-$HG^1$ or O-$HG^2$. In a particular embodiment, each $R^{2a}$ and $R^{2b}$ is OH.

In one embodiment, with respect to the bolaamphiphilic compound of formula II, III, IV, V, or VI, each $R^{1a}$ and $R^{1b}$ is independently H, Me, Et, n-Pr, i-Pr, n-Bu, i-Bu, sec-Bu, n-pentyl, isopentyl, n-hexyl, n-heptyl, or n-octyl. In a particular embodiment, each $R^{1a}$ and $R^{1b}$ is independently n-pentyl.

In one embodiment, with respect to the bolaamphiphilic compound of formula II, III, IV, V, or VI, each dotted bond is a single bond. In another embodiment, each dotted bond is a double bond.

In one embodiment, with respect to the bolaamphiphilic compound of formula II, III, IV, V, or VI, n10 is an integer from 2-16. In another embodiment, n10 is an integer from 2-12. In a particular embodiment, n10 is 2, 4, 6, 8, 10, 12, or 16.

In one embodiment, with respect to the bolaamphiphilic compound of formula IV, $R^4$ is H, Me, Et, n-Pr, i-Pr, n-Bu, i-Bu, sec-Bu, n-pentyl, or isopentyl. In another embodiment, $R^4$ is Me, or Et. In a particular embodiment, $R^4$ is Me.

In one embodiment, with respect to the bolaamphiphilic compound of formula II, III, IV, V, or VI, each $Z^1$ and $Z^2$ is independently $C(R^3)_2$-, or -$N(R^3)$-. In another embodiment, each $Z^1$ and $Z^2$ is independently $C(R^3)_2$-, or -$N(R^3)$-; and each $R^3$ is independently H, Me, Et, n-Pr, i-Pr, n-Bu, i-Bu, sec-Bu, n-pentyl, or isopentyl. In a particular embodiment, $R^3$ is H.

In one embodiment, with respect to the bolaamphiphilic compound of formula II, III, IV, V, or VI, each $Z^1$ and $Z^2$ is -O-.

In one embodiment, with respect to the bolaamphiphilic compound of formula I, II, III, or IV, each $HG^1$ and $HG^2$ is independently selected from:

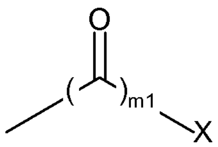
,
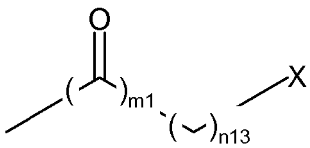
,
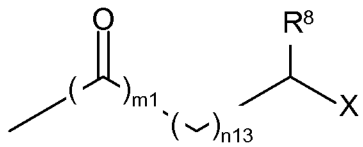
,
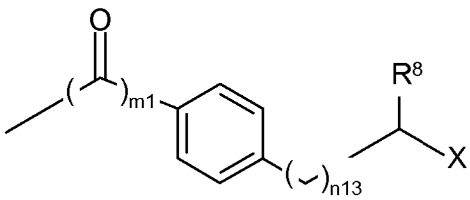
and
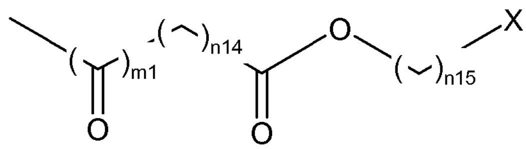

wherein:

X is -$NR^{5a}R^{5b}$, or -$N^+R^{5a}R^{5b}R^{5c}$; each $R^{5a}$, and $R^{5b}$ is independently H or substituted or unsubstituted $C_1$-$C_{20}$ alkyl or $R^{5a}$ and $R^{5b}$ may join together to form an N containing substituted or unsubstituted heteroaryl, or substituted or unsubstituted heterocyclyl;

each $R^{5c}$ is independently substituted or unsubstituted $C_1$-$C_{20}$ alkyl; each $R^8$ is independently H, substituted or unsubstituted $C_1$-$C_{20}$ alkyl, alkoxy, or carboxy;

m1 is 0 or 1; and

each n13, n14, and n15 is independently an integer from 1-20.

In one embodiment, with respect to the bolaamphiphilic compound of formula I, II, III, or IV, $HG^1$ and $HG^2$ are as defined above, and each m1 is 0.

In one embodiment, with respect to the bolaamphiphilic compound of formula I, II, III, or IV, $HG^1$ and $HG^2$ are as defined above, and each m1 is 1.

In one embodiment, with respect to the bolaamphiphilic compound of formula I, II, III, or IV, $HG^1$ and $HG^2$ are as defined above, and each n13 is 1 or 2.

In one embodiment, with respect to the bolaamphiphilic compound of formula I, II, III, or IV, $HG^1$ and $HG^2$ are as defined above, and each n14 and n15 is independently 1, 2, 3, 4, or 5. In another embodiment, each n14 and n15 is

independently 2 or 3.

In one particular embodiment, the bolaamphiphilic compound is a compound according to formula VIIa, VIIb, VIIc, or VIId:

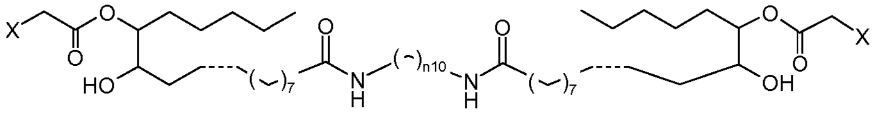

VIIa

,

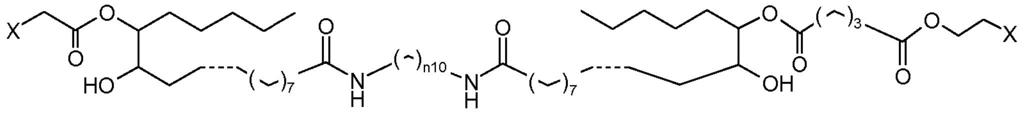

VIIb

,

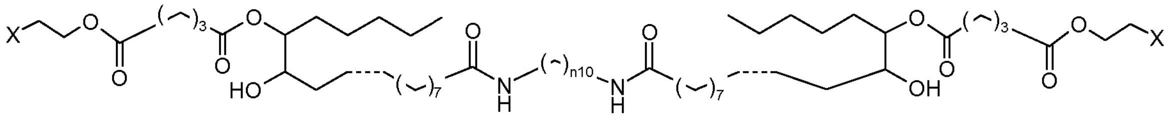

VIIc

or

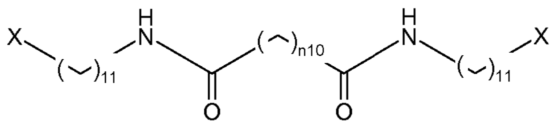

VIId

or a pharmaceutically acceptable salt, solvate, hydrate, prodrug, stereoisomer, tautomer, isotopic variant, or N-oxide thereof, or a combination thereof;
wherein:

each X is $-NR^{5a}R^{5b}$, or $-N^{+}R^{5a}R^{5b}R^{5c}$; each $R^{5a}$, and $R^{5b}$ is independently H or substituted or unsubstituted $C_1$-$C_{20}$ alkyl or $R^{5a}$ and $R^{5b}$ may join together to form an N containing substituted or unsubstituted heteroaryl, or substituted or unsubstituted heterocyclyl;

each $R^{5c}$ is independently substituted or unsubstituted $C_1$-$C_{20}$ alkyl;;
n10 is an integer from 2-20; and
each dotted bond is independently a single or a double bond.

In another particular embodiment, the bolaamphiphilic compound is a compound according to formula Villa, VIIIb, VIIIc, or VIIId:

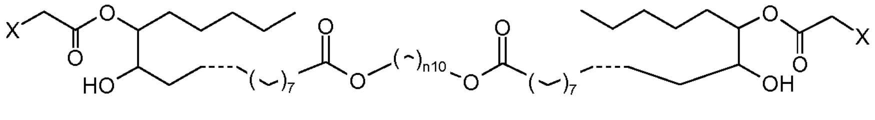

VIIIa

,

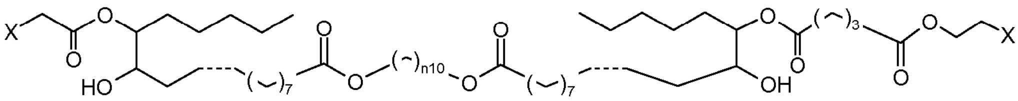

VIIIb

,

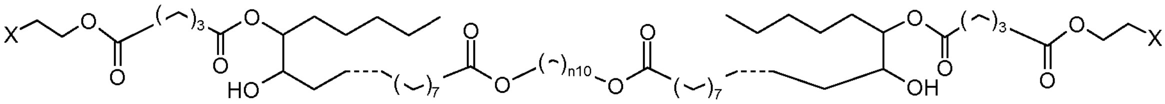

VIIIc

or

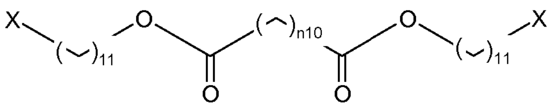

VIIId

or a pharmaceutically acceptable salt, solvate, hydrate, prodrug, stereoisomer, tautomer, isotopic variant, or N-oxide thereof, or a combination thereof;
wherein:

each X is $-NR^{5a}R^{5b}$, or $-N^{+}R^{5a}R^{5b}R^{5c}$; each $R^{5a}$, and $R^{5b}$ is independently H or substituted or unsubstituted $C_1$-$C_{20}$ alkyl or $R^{5a}$ and $R^{5b}$ may join together to form an N containing substituted or unsubstituted heteroaryl, or substituted or unsubstituted heterocyclyl;

each $R^{5c}$ is independently substituted or unsubstituted $C_1$-$C_{20}$ alkyl;;
n10 is an integer from 2-20; and
each dotted bond is independently a single or a double bond.

In another particular embodiment, the bolaamphiphilic compound is a compound according to formula IXa, IXb, or IXc:

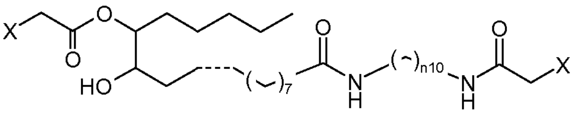

IXa

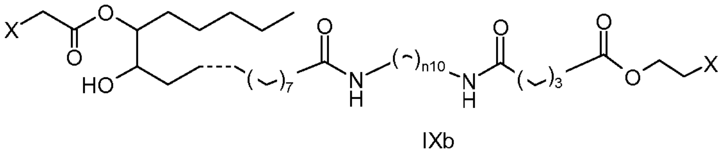

IXb

,

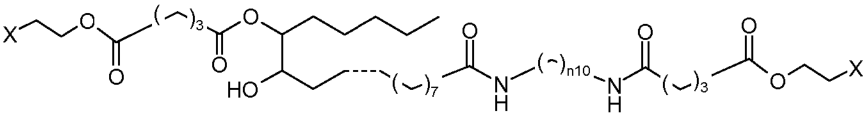

IXc

or a pharmaceutically acceptable salt, solvate, hydrate, prodrug, stereoisomer, tautomer, isotopic variant, or N-oxide thereof, or a combination thereof;
wherein:

each X is $-NR^{5a}R^{5b}$, or $-N^{+}R^{5a}R^{5b}R^{5c}$; each $R^{5a}$, and $R^{5b}$ is independently H or substituted or unsubstituted $C_1$-$C_{20}$ alkyl or $R^{5a}$ and $R^{5b}$ may join together to form an N containing substituted or unsubstituted heteroaryl, or substituted or unsubstituted heterocyclyl;

each $R^{5c}$ is independently substituted or unsubstituted $C_1$-$C_{20}$ alkyl;;
n10 is an integer from 2-20; and
each dotted bond is independently a single or a double bond.

In another particular embodiment, the bolaamphiphilic compound is a compound according to formula Xa, Xb, or Xc:

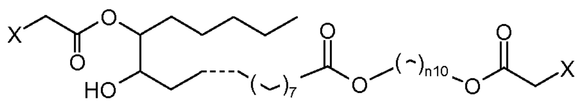

Xa

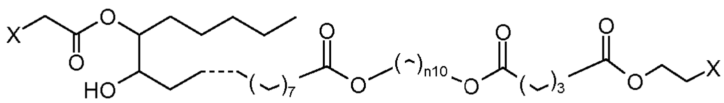

Xb

,

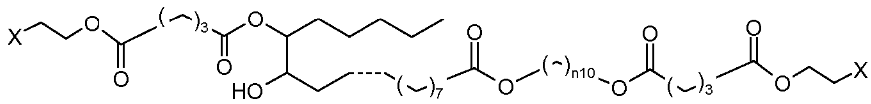

Xc

or a pharmaceutically acceptable salt, solvate, hydrate, prodrug, stereoisomer, tautomer, isotopic variant, or N-oxide thereof, or a combination thereof;
wherein:

each X is $-NR^{5a}R^{5b}$, or $-N^{+}R^{5a}R^{5b}R^{5c}$; each $R^{5a}$, and $R^{5b}$ is independently H or substituted or unsubstituted $C_1$-$C_{20}$ alkyl or $R^{5a}$ and $R^{5b}$ may join together to form an N containing substituted or unsubstituted heteroaryl, or substituted or unsubstituted heterocyclyl;

each $R^{5c}$ is independently substituted or unsubstituted $C_1$-$C_{20}$ alkyl;;
n10 is an integer from 2-20; and
each dotted bond is independently a single or a double bond.

In one embodiment, with respect to the bolaamphiphilic compound of formula VIIa-VIId, VIIIa-VIIId, IXa-IXc, or Xa-Xc, each dotted bond is a single bond. In another embodiment, each dotted bond is a double bond.
In one embodiment, with respect to the bolaamphiphilic compound of formula VIIa-VIId, VIIIa-VIIId, IXa-IXc, or Xa-Xc, n10 is an integer from 2-16.
In one embodiment, with respect to the bolaamphiphilic compound of formula VIIa-VIId, VIIIa-VIIId, IXa-IXc, or Xa-Xc, n10 is an integer from 2-12.
In one embodiment, with respect to the bolaamphiphilic compound of formula VIIa-VIId, VIIIa-VIIId, IXa-IXc, or Xa-Xc, n10 is 2, 4, 6, 8, 10, 12, or 16.
In one embodiment, with respect to the bolaamphiphilic compound of formula VIIa-VIId, VIIIa-VIIId, IXa-IXc, or Xa-Xc, each $R^{5a}$, $R^{5b}$, and $R^{5c}$ is independently substituted or unsubstituted $C_1$-$C_{20}$ alkyl.
In one embodiment, with respect to the bolaamphiphilic compound of formula VIIa-VIId, VIIIa-VIIId, IXa-IXc, or

Xa-Xc, each $R^{5a}$, $R^{5b}$, and $R^{5c}$ is independently unsubstituted $C_1$-$C_{20}$ alkyl.

In one embodiment, with respect to the bolaamphiphilic compound of formula VIIa-VIId, VIIIa-VIIId, IXa-IXc, or Xa-Xc, one of $R^{5a}$, $R^{5b}$, and $R^{5c}$ is $C_1$-$C_{20}$ alkyl substituted with -OC(O)$R^6$; and $R^6$ is $C_1$-$C_{20}$ alkyl.

In one embodiment, with respect to the bolaamphiphilic compound of formula VIIa-VIId, VIIIa-VIIId, IXa-IXc, or Xa-Xc, two of $R^{5a}$, $R^{5b}$, and $R^{5c}$ are independently $C_1$-$C_{20}$ alkyl substituted with -OC(O)$R^6$; and $R^6$ is $C_1$-$C_{20}$ alkyl. In one embodiment, $R^6$ is Me, Et, n-Pr, i-Pr, n-Bu, i-Bu, sec-Bu, n-pentyl, isopentyl, n-hexyl, n-heptyl, or n-octyl. In a particular embodiment, $R^6$ is Me.

In one embodiment, with respect to the bolaamphiphilic compound of formula VIIa-VIId, VIIIa-VIIId, IXa-IXc, or Xa-Xc, one of $R^{5a}$, $R^{5b}$, and $R^{5c}$ is $C_1$-$C_{20}$ alkyl substituted with amino, alkylamino or dialkylamino.

In one embodiment, with respect to the bolaamphiphilic compound of formula VIIa-VIId, VIIIa-VIIId, IXa-IXc, or Xa-Xc, two of $R^{5a}$, $R^{5b}$, and $R^{5c}$ are independently $C_1$-$C_{20}$ alkyl substituted with amino, alkylamino or dialkylamino.

In one embodiment, with respect to the bolaamphiphilic compound of formula VIIa-VIId, VIIIa-VIIId, IXa-IXc, or Xa-Xc, $R^{5a}$, and $R^{5b}$ together with the N they are attached to form substituted or unsubstituted heteroaryl.

In one embodiment, with respect to the bolaamphiphilic compound of formula VIIa-VIId, VIIIa-VIIId, IXa-IXc, or Xa-Xc, $R^{5a}$, and $R^{5b}$ together with the N they are attached to form substituted or unsubstituted pyridyl.

In one embodiment, with respect to the bolaamphiphilic compound of formula VIIa-VIId, VIIIa-VIIId, IXa-IXc, or Xa-Xc, $R^{5a}$, and $R^{5b}$ together with the N they are attached to form substituted or unsubstituted monocyclic or bicyclic heterocyclyl.

In one embodiment, with respect to the bolaamphiphilic compound of formula VIIa-VIId, VIIIa-VIIId, IXa-IXc, or Xa-Xc, X is substituted or unsubstituted

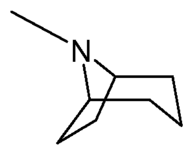

In one embodiment, with respect to the bolaamphiphilic compound of formula VIIa-VIId, VIIIa-VIIId, IXa-IXc, or Xa-Xc, X is

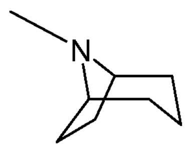

,

substituted with one or more groups selected from alkoxy, acetyl, and substituted or unsubstituted Ph.

In one embodiment, with respect to the bolaamphiphilic compound of formula VIIa-VIId, VIIIa-VIIId, IXa-IXc, or Xa-Xc, X is

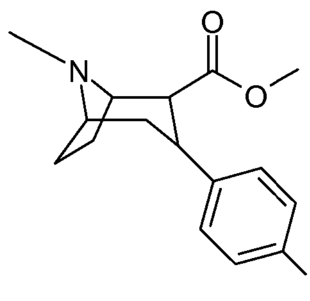

.

In one embodiment, with respect to the bolaamphiphilic compound of formula VIIa-VIId, VIIIa-VIIId, IXa-IXc, or Xa-Xc, X is $-NMe_2$ or $-N^+Me_3$.

In one embodiment, with respect to the bolaamphiphilic compound of formula VIIa-VIId, VIIIa-VIIId, IXa-IXc, or Xa-Xc, X is $-N(Me)-CH_2CH_2-OAc$ or $-N^+(Me)_2-CH_2CH_2-OAc$.

In one embodiment, with respect to the bolaamphiphilic compound of formula VIIa-VIId, VIIIa-VIIId, IXa-IXc, or Xa-Xc, X is a chitosanyl group; and the chitosanyl group is a poly-(D)glucosaminyl group with MW of 3800 to 20,000

Daltons, and is attached to the core via N.

In one embodiment, the chitosanyl group is

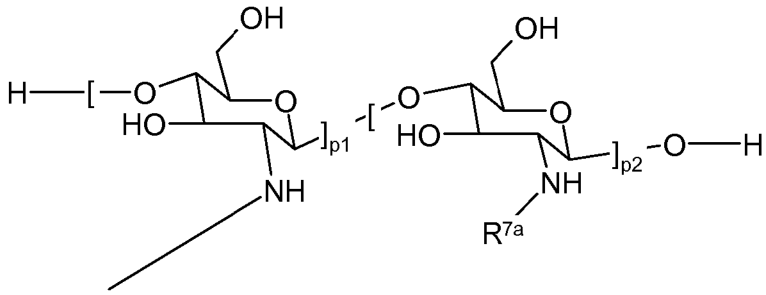

;

and wherein each p1 and p2 is independently an integer from 1-400; and each $R^{7a}$ is H or acyl.

In one embodiment, with respect to the bolaamphiphilic compound of formula VIIa-VIId, VIIIa-VIIId, IXa-IXc, or Xa-Xc, X is a substance P head group. In one embodiment, the substance P head group is bound through the ω-amino group of lysine. In another embodiment, X is -NH-$(CH2)_4$-C(H)(NH-Pro-Arg)-NH-Pro-Gly-Gly-Phe-Phe-Gly-Leu-Met.

In one embodiment, with respect to the bolaamphiphilic compound of formula VIIa-VIId, VIIIa-VIIId, IXa-IXc, or Xa-Xc, X is a headgroup comprising NK1R antagonist.

In one embodiment, the NK1R antagonist is

| | | |
|---|---|---|
| 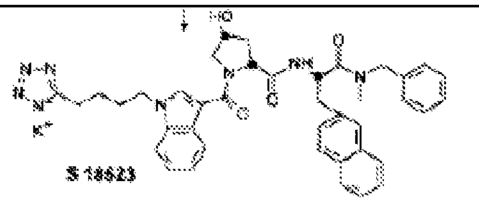 S 18523 *Potassium salt of a chemically stabilized dipeptide, S18523 a water-soluble, potent and selective $NK_1$ receptor antagonist.* | 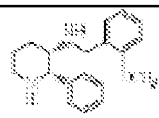 | 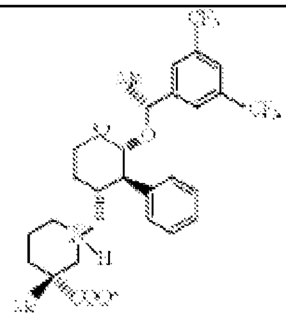 |
| I {1-[4-(1*H*-tetrazol-5-yl)butyl]indol-3-yl}carbonyl-Hyp-Nal-*N*(methyl)-Bzl, (Hyp = (*R*)-4-hydroxy-L-proline; Nal = 3-L-(β-naphthyl)-alanine) | II CP-99,994 | III (+)-[2R,3R,4R,5R,5(3'R)-2-{1-[1(3,5bis(trifluoromethyl)phenyl]ethyl}oxy}-4(-3-carboxy-3-methylpiperidin-1yl)-3-phenyl-methyltetrahydropyran |

In one embodiment, with respect to the bolaamphiphilic compound of formula I, II, III, IV, V, VI, VIIa-VIIc, VIIIa-VIIIc, IXa-IXc and Xa-Xc, the bolaamphiphilic compound is a pharmaceutically acceptable salt.

In one embodiment, with respect to the bolaamphiphilic compound of formula I, II, III, IV, V, VI, VIIa-VIIc, VIIIa-VIIIc, IXa-IXc and Xa-Xc, the bolaamphiphilic compound is in a form of a quaternary salt.

In one embodiment, with respect to the bolaamphiphilic compound of formula I, II, III, IV, V, VI, VIIa-VIIc, VIIIa-VIIIc, IXa-IXc and Xa-Xc, the bolaamphiphilic compound is in a form of a quaternary salt with pharmaceutically acceptable alkyl halide or alkyl tosylate.

In one embodiment, with respect to the bolaamphiphilic compound of formula I, II, III, IV, V, VI, VIIa-VIIc, VIIIa-VIIIc, IXa-IXc and Xa-Xc, the bolaamphiphilic compound is any one of the bolaambphilic compounds listed in Table 1.

In another specific aspect, provided herein are methods for incorporating biologically active drugs in the bolavesicles. In one embodiment, the bolavesicle comporises one or more bolaamphilic compounds described herein.

In another specific aspect, provided herein are methods for brain-targeted drug delivery using the bolavesicles incorporated with biologically active drug.

The biologically active drug in the composition or in the formulation of the invention is irinotecan (CPT-11 or (S)-4,11-diethyl-3,4,12,14-tetrahydro-4-hydroxy-3,14-dioxo1H-pyrano[3',4':6,7]-indolizino[1,2-b]quinolin-9-yl-[1,4'bipiperidine]-1'-carboxylate).

In another specific aspect, provided herein are methods for delivering CPT-11 to the brain.

In another specific aspect, provided herein are nano-particles, comprising one or more bolaamphiphilic compounds and CPT-11.

In another specific aspect, provided herein are pharmaceutical compositions, comprising a nano-sized particle comprising one or more bolaamphiphilic compounds and kyotorphine, enkephaline, or CPT-11; and a pharmaceutically acceptable carrier.

The Derivatives and Precursors disclosed can be prepared as illustrated in the Schemes provided herein. The syntheses can involve initial construction of, for example, vernonia oil or direct functionalization of natural derivatives by organic synthesis manipulations such as, but not limiting to, epoxide ring opening. In those processes involving oxiranyl ring opening, the epoxy group is opened by the addition of reagents such as carboxylic acids or organic or inorganic nucleophiles. Such ring opening results in a mixture of two products in which the new group is introduced at either of the two carbon atoms of the epoxide moiety. This provides beta substituted alcohols in which the substitution position most remote from the CO group of the main aliphatic chain of the vernonia oil derivative is arbitrarily assigned as position 1, while the neighboring substituted carbon position is designated position 2. For simplicity purposes only, the Derivatives and Precursors shown herein may indicate structures with the hydroxy group always at position 2 but the Derivatives and Precursors wherein the hydroxy is at position 1 are also encompassed by the invention. Thus, a radical of the formula --CH(OH)-CH(R)-- refers to the substitution of --OH at either the carbon closer to the CO group, designated position 2 or to the carbon at position 1. Moreover, with respect to the preparation of symmetrical bolaamphiphiles made via introducing the head groups through an epoxy moiety (*e.g.*, as in vernolic acid) or a double bond (-C=C-) as in mono unsaturated fatty acids (*e.g.*, oleic acid) a mixture of three different derivatives will be produced. In certain embodiments, vesicles are prepared using the mixture of unfractionated positional isomers. In one aspect of this embodiment, where one or more bolas are prepared from vernolic acid, and in which a hydroxy group as well as the head group introduced through an epoxy or a fatty acid with the head group introduced through a double bond (-C=C-), the bola used in vesicle preparation can actually be a mixture of three different positional isomers.

In other embodiments, the three different derivatives are isolated. Accordingly, the vesicles disclosed herein can be made from a mixture of the three isomers of each derivative or, in other embodiments, the individual isomers can be isolated and used for preparation of vesicles.

Symmetrical bolaamphiphiles can form relatively stable self aggregate vesicle structures by the use of additives such as cholesterol and cholesterol derivatives (e.g., cholesterol hemisuccinate, cholesterol oleyl ether, anionic and cationic derivatives of cholesterol and the like), or other additives including single headed amphiphiles with one, two or multiple aliphatic chains such as phospholipids, zwitterionic, acidic, or cationic lipids. Examples of zwitterionic lipids are phosphatidylcholines, phosphatidylethanol amines and sphingomyelins. Examples of acidic amphiphilic lipids are phosphatidylglycerols, phosphatidylserines, phosphatidylinositols, and phosphatidic acids. Examples of cationic amphipathic lipids are diacyl trimethylammonium propanes, diacyl dimethylammonium propanes, and stearylamines cationic amphiphiles such as spermine cholesterol carbamates, and the like, in optimum concentrations which fill in the larger spaces on the outer surfaces, and/or add additional hydrophilicity to the particles. Such additives may be added to the reaction mixture during formation of nanoparticles to enhance stability of the nanoparticles by filling in the void volumes of in the upper surface of the vesicle membrane.

Stability of nano vesicles according to the present disclosure can be demonstrated by dynamic light scattering (DLS) and transmission electron microscopy (TEM). For example, suspensions of the vesicles can be left to stand for 1, 5, 10, and 30 days to assess the stability of the nanoparticle solution/suspension and then analyzed by DLS and TEM.

Claim 1 requires presence of CPT-11 as an active agent. The vesicles disclosed herein may encapsulate within their core the active agent, which in particular embodiments is selected from peptides, proteins, nucleotides and or non-polymeric agents. In certain embodiments, the active agent is also associated via one or more non-covalent interactions to the vesicular membrane on the outer surface and/or the inner surface, optionally as pendant decorating the outer or inner surface, and may further be incorporated into the membrane surrounding the core. In certain aspects, biologically active peptides, proteins, nucleotides or non-polymeric agents that have a net electric charge, may associate ionically with oppositely charged headgroups on the vesicle surface and/or form salt complexes therewith.

In particular aspects of these embodiments, additives which may be bolaamphiphiles or single headed amphiphiles, comprise one or more branching alkyl chains bearing polar or ionic pendants, wherein the aliphatic portions act as anchors into the vesicle's membrane and the pendants (*e.g.*, chitosan derivatives or polyamines or certain peptides) decorate the surface of the vesicle to enhance penetration through various biological barriers such as the intestinal tract and the BBB, and in some instances are also selectively hydrolyzed at a given site or within a given organ. The concentration of these additives is readily adjusted according to experimental determination.

In certain embodiments, the oral formulations of the present disclosure comprise agents that enhance penetration through the membranes of the GI tract and enable passage of intact nanoparticles containing the drug. These agents may be any of the additives mentioned above and, in particular aspects of these embodiment, include chitosan and derivatives thereof, serving as vehicle surface ligands, as decorations or pendants on the vesicles, or the agents

may be excipients added to the formulation.

In other embodiments, the nanoparticles and vesicles disclosed herein may comprise the fluorescent marker carboxyfluorescein (CF) encapsulated therein while in particular aspects, the nanoparticle and vesicles of the present disclosure may be decorated with one or more of PEG, *e.g.* PEG2000-vernonia derivatives as pendants. For example, two kinds of PEG-vernonia derivatives can be used: PEG-ether derivatives, wherein PEG is bound via an ether bond to the oxygen of the opened epoxy ring of, *e.g.*, vernolic acid and PEG-ester derivatives, wherein PEG is bound via an ester bond to the carboxylic group of, *e.g.*, vernolic acid.

In other embodiments, vesicles, made from synthetic amphiphiles, as well as liposomes, made from synthetic or natural phospholipids, substantially (or totally) isolate the therapeutic agent from the environment allowing each vesicle or liposome to deliver many molecules of the therapeutic agent. Moreover, the surface properties of the vesicle or liposome can be modified for biological stability, enhanced penetration through biological barriers and targeting, independent of the physico-chemical properties of the encapsulated drug.

In still other embodiments, the headgroup is selected from: (i) choline or thiocholine, O-alkyl, N-alkyl or ester derivatives thereof; (ii) non-aromatic amino acids with functional side chains such as glutamic acid, aspartic acid, lysine or cysteine, or an aromatic amino acid such as tyrosine, tryptophan, phenylalanine and derivatives thereof such as levodopa (3,4-dihydroxy-phenylalanine) and p-aminophenylalanine; (iii) a peptide or a peptide derivative that is specifically cleaved by an enzyme at a diseased site selected from enkephalin, N-acetyl-ala-ala, a peptide that constitutes a domain recognized by beta and gamma secretases, and a peptide that is recognized by stromelysins; (iv) saccharides such as glucose, mannose and ascorbic acid; and (v) other compounds such as nicotine, cytosine, lobeline, polyethylene glycol, a cannabinoid, or folic acid.

In further embodiments, nano-sized particle and vesicles disclosed herein further comprise at least one additive for one or more of targeting purposes, enhancing permeability and increasing the stability the vesicle or particle. Such additives, in particular aspects, may selected from from: (i) a single headed amphiphilic derivative comprising one, two or multiple aliphatic chains, preferably two aliphatic chains linked to a midsection/spacer region such as $--NH-(CH_2)_2--N--(CH_2)_2--N--$, or $--O--(CH_2)_2-N--(CH_2)_2--O--$, and a sole headgroup, which may be a selectively cleavable headgroup or one containing a polar or ionic selectively cleavable group or moiety, attached to the N atom in the middle of said midsection. In other asepcts, the additive can be selected from among cholesterol and cholesterol derivatives such as cholesteryl hemmisuccinate; phospholipids, zwitterionic, acidic, or cationic lipids; chitosan and chitosan derivatives, such as vernolic acid-chitosan conjugate, quaternized chitosan, chitosan-polyethylene glycol (PEG) conjugates, chitosan-polypropylene glycol (PPG) conjugates, chitosan N-conjugated with different amino acids, carboxyalkylated chitosan, sulfonyl chitosan, carbohydrate-branched N-(carboxymethylidene) chitosan and N-(carboxymethyl) chitosan; polyamines such as protamine, polylysine or polyarginine; ligands of specific receptors at a target site of a biological environment such as nicotine, cytisine, lobeline, 1-glutamic acid MK801, morphine, enkephalins, benzodiazepines such as diazepam (valium) and librium, dopamine agonists, dopamine antagonists tricyclic antidepressants, muscarinic agonists, muscarinic antagonists, cannabinoids and arachidonyl ethanol amide; polycationic polymers such as polyethylene amine; peptides that enhance transport through the BBB such as OX 26, transferrins, polybrene, histone, cationic dendrimer, synthetic peptides and polymyxin B nonapeptide (PMBN); monosaccharides such as glucose, mannose, ascorbic acid and derivatives thereof; modified proteins or antibodies that undergo absorptive-mediated or receptor-mediated transcytosis through the blood-brain barrier, such as bradykinin B2 agonist RMP-7 or monoclonal antibody to the transferrin receptor; mucoadhesive polymers such as glycerides and steroidal detergents; and $Ca^{2+}$ chelators. The aforementioned head groups on the additives designed for one or more of targeting purposes and enhancing permeability may also be a head group, preferably on an asymmetric bolaamphiphile wherein the other head group is another moiety, or the head group on both sides of a symmetrical bolaamphiphile. In a further embodiment the bolaamphiphile head groups that comprise the vesicles membranes can interact with the active agents to be encapsulated to be delivered in to the brain and brain sites, and or other targeted sites, by ionic interactions to enhance the % encapsulation via complexation and well as passive encapsulation within the vesicles core. Further the formulation may contain other additives within the vehicles membranes to further enhance the degree of encapsulation of the active agents by interactions other than ionic interactions such as polar or hydrophobic interactions.

In other embodiments, nano-sized particle and vesicles discloser herein may comprises at least one biologically active agent is selected from: (i) a natural or synthetic peptide or protein such as analgesics peptides from the enkephalin class, insulin, insulin analogs, oxytocin, calcitonin, tyrotropin releasing hormone, follicle stimulating hormone, luteinizing hormone, vasopressin and vasopressin analogs, catalase, interleukin-II, interferon, colony stimulating factor, tumor necrosis factor (TNF), melanocyte-stimulating hormone, superoxide dismutase, glial cell derived neurotrophic factor (GDNF) or the Gly-Leu-Phe (GLF) families; (ii) nucleosides and polynucleotides selected from DNA or RNA molecules such as small interfering RNA (siRNA) or a DNA plasmid; (iii) antiviral and antibacterial; (iv) antineoplastic and chemotherapy agents such as cyclosporin, doxorubicin, epirubicin, bleomycin, cisplatin, carboplatin, vinca alkaloids, *e.g.* vincristine, Podophyllotoxin, taxanes, *e.g.* Taxol and Docetaxel, and topoisomerase inhibitors, *e.g.* irinotecan, topotecan.

Additional embodiments within the scope provided herein are set forth in non-limiting fashion elsewhere herein

and in the examples. It should be understood that these examples are for illustrative purposes only and are not to be construed as limiting in any manner.

## PHARMACEUTICAL COMPOSITIONS

In another aspect, the invention provides a pharmaceutical composition comprising a pharmaceutically acceptable carrier and a pharmaceutically effective amount of a compound GLH-20 or a complex thereof.

When employed as pharmaceuticals, the compounds provided herein are typically administered in the form of a pharmaceutical composition. Such compositions can be prepared in a manner well known in the pharmaceutical art and comprise at least one active compound.

In certain embodiments, with respect to the pharmaceutical composition, the carrier is a parenteral carrier, oral or topical carrier.

The present invention also relates to a compound or pharmaceutical composition of the compound as defined in claim 1. or a pharmaceutically acceptable salt or solvate thereof for use as a pharmaceutical or a medicament.

Generally, the compounds provided herein are administered in a therapeutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions provided herein can be administered by a variety of routes including oral, rectal, transdermal, subcutaneous, intravenous, intramuscular, and intranasal. Depending on the intended route of delivery, the compounds provided herein are preferably formulated as either injectable or oral compositions or as salves, as lotions or as patches all for transdermal administration.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art. As before, the active compound in such compositions is typically a minor component, often being from about 0.05 to 10% by weight with the remainder being the injectable carrier and the like.

Transdermal compositions are typically formulated as a topical ointment or cream containing the active ingredient(s), generally in an amount ranging from about 0.01 to about 20% by weight, preferably from about 0.1 to about 20% by weight, preferably from about 0.1 to about 10% by weight, and more preferably from about 0.5 to about 15% by weight. When formulated as a ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or the formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington 's Pharmaceutical Sciences, 17th edition, 1985, Mack Publishing Company, Easton, Pennsylvania.

The above-described components for orally administrable, injectable, or topically administrable compositions are merely representative. Other materials as well as processing techniques and the like are set forth in Part 8 of Remington 's The Science and Practice of Pharmacy, 21st edition, 2005, Publisher: Lippincott Williams & Wilkins.

The compounds of this invention can also be administered in sustained release forms or from sustained release

drug delivery systems. A description of representative sustained release materials can be found in *Remington's Pharmaceutical Sciences.*

The present invention also relates to the pharmaceutically acceptable formulations of compounds of Formula I. In certain embodiments, the formulation comprises water. In another embodiment, the formulation comprises a cyclodextrin derivative. In certain embodiments, the formulation comprises hexapropyl-β-cyclodextrin. In a more particular embodiment, the formulation comprises hexapropyl-β-cyclodextrin (10-50% in water).

The present invention also relates to the pharmaceutically acceptable acid addition salts of the compound as defined in claim 1. The acids which are used to prepare the pharmaceutically acceptable salts are those which form non-toxic acid addition salts, *i.e.* salts containing pharmacologically acceptable aniovs such as the hydrochloride, hydroiodide, hydrobromide, nitrate, sulfate, bisulfate, phosphate, acetate, lactate, citrate, tartrate, succinate, maleate, fumarate, benzoate, para-toluenesulfonate, and the like.

The following formulation examples illustrate representative pharmaceutical compositions that may be prepared in accordance with this invention. The present invention, however, is not limited to the following pharmaceutical compositions.

**Formulation 1 - Injection**

A compound of the invention may be dissolved or suspended in a buffered sterile saline injectable aqueous medium to a concentration of approximately 5 mg/mL.

**METHODS OF TREATMENT**

Bolaamphiphilic vesicles (bolavesicles) may have certain advantages over conventional liposomes as potential vehicles for drug delivery. Bolavesicles have thinner membranes than comparable liposomal bilayer, and therefore possess bigger inner volume and hence higher encapsulation capacity than liposomes of the same diameter. Moreover, bolavesicles are more physically-stable than conventional liposomes, but can be destabilized in a triggered fashion (e.g., by hydrolysis of the headgroups using a specific enzymatic reaction) thus allowing controlled release of the encapsulated material at the site of action (i.e., drug targeting).

Thus, various biologically active drug molecueles can be encapsulated in the bolaamphiphilic vesicles and then delivered to the brain in sufficient concentrations for therapeutic use.

The bola vesicles aggregate into encapsulating monolayer membranes which, together with functional surface groups, provide vesicle stability, penetrability through the BBB and a controlled release mechanism that enables the release of the encapsulated drug primarily in the brain.

The novel nanovesicles can encapsulates drugs, gets through the blood-brain barrier (BBB) and releases the drug in the brain. A major factor limiting the efficacy of some chemotherapeutical agents that are potentially effective in the treatment of malignant gliomas, particularly glioblastoma multiforme (GBM), is that most drugs cannot cross the BBB. A study from Duke University showed that, out of 40 drugs tested, CPT-11 (Irinotecan, used for solid tumors) was the most potent chemotherapeutic agent against patients' gliomas implanted in mice, and was effective against every tumor tested.

However, attempts to treat GBM patients with CPT-11 were unsuccessful because very little gets through the BBB and reaches the tumor. Hence, CPT-11 encapsulated within bola vesicles, can penetrate the brain via the intense capillary network that supplies blood to the brain and can release CPT-11 upon reaching tumor cells. Thus, it would be effective in treating GBM. The efficacy of CPT- 1 1 delivered by bola vesicles may be further increased by administering it with oral temozolamide which, in combination with radiotherapy, prolongs survival by months and, based on literature, acts synergistically with CPT-11 to kill gliomas.

**GENERAL SYNTHETIC PROCEDURES**

The compounds provided herein can be purchased or prepared from readily available starting materials using the following general methods and procedures. *See, e.g.*, Synthetic Schemes below. It will be appreciated that where typical or preferred process conditions (*i.e.*, reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in T. W. Greene and P. G. M.

Wuts, Protecting Groups in Organic Synthesis, Second Edition, Wiley, New York, 1991, and references cited therein.

The compounds provided herein may be isolated and purified by known standard procedures. Such procedures include (but are not limited to) recrystallization, column chromatography or HPLC. The following schemes are presented with details as to the preparation of representative substituted biarylamides that have been listed herein. The compounds provided herein may be prepared from known or commercially available starting materials and reagents by one skilled in the art of organic synthesis.

The enantiomerically pure compounds provided herein may be prepared according to any techniques known to those of skill in the art. For instance, they may be prepared by chiral or asymmetric synthesis from a suitable optically pure precursor or obtained from a racemate by any conventional technique, for example, by chromatographic resolution using a chiral column, TLC or by the preparation of diastereoisomers, separation thereof and regeneration of the desired enantiomer. *See, e.g.*, "Enantiomers, Racemates and Resolutions," by J. Jacques, A. Collet, and S.H. Wilen, (Wiley-Interscience, New York, 1981); S.H. Wilen, A. Collet, and J. Jacques, Tetrahedron, 2725 (1977); E.L. Eliel Stereochemistry of Carbon Compounds (McGraw-Hill, NY, 1962); and S.H. Wilen Tables of Resolving Agents and Optical Resolutions 268 (E.L. Eliel ed., Univ. of Notre Dame Press, Notre Dame, IN, 1972, Stereochemistry of Organic Compounds, Ernest L. Eliel, Samuel H. Wilen and Lewis N. Manda (1994 John Wiley & Sons, Inc.), and Stereoselective Synthesis A Practical Approach, Mihály Nógrádi (1995 VCH Publishers, Inc., NY, NY).

In certain embodiments, an enantiomerically pure compound of formula (1) may be obtained by reaction of the racemate with a suitable optically active acid or base. Suitable acids or bases include those described in Bighley et al., 1995, Salt Forms of Drugs and Adsorption, in Encyclopedia of Pharmaceutical Technology, vol. 13, Swarbrick & Boylan, eds., Marcel Dekker, New York; ten Hoeve & H. Wynberg, 1985, Journal of Organic Chemistry 50:4508-4514; Dale & Mosher, 1973, J. Am. Chem. Soc. 95:512; and *CRC Handbook of Optical Resolution via Diastereomeric Salt Formation.*

Enantiomerically pure compounds can also be recovered either from the crystallized diastereomer or from the mother liquor, depending on the solubility properties of the particular acid resolving agent employed and the particular acid enantiomer used. The identity and optical purity of the particular compound so recovered can be determined by polarimetry or other analytical methods known in the art. The diasteroisomers can then be separated, for example, by chromatography or fractional crystallization, and the desired enantiomer regenerated by treatment with an appropriate base or acid. The other enantiomer may be obtained from the racemate in a similar manner or worked up from the liquors of the first separation.

In certain embodiments, enantiomerically pure compound can be separated from racemic compound by chiral chromatography. Various chiral columns and eluents for use in the separation of the enantiomers are available and suitable conditions for the separation can be empirically determined by methods known to one of skill in the art. Exemplary chiral columns available for use in the separation of the enantiomers provided herein include, but are not limited to CHIRALCEL® OB, CHIRALCEL® OB-H, CHIRALCEL® OD, CHIRALCEL® OD-H, CHIRALCEL® OF, CHIRALCEL® OG, CHIRALCEL® OJ and CHIRALCEL® OK.

ABBREVIATIONS

BBB, blood brain barrier
BCECs, brain capillary endothelial cells
CF, carboxyfluorescein
CHEMS, cholesteryl hemisuccinate
CHOL, cholesterol
Cryo-TEM, Cryo-transmission electron microscope
DAPI, 4',6- diamidino-2-phenylindole
DDS, drug delivery system
DLS, dynamic light scattering
DMPC, 1,2-dimyristoyl-sn-glycero-3-phosphocholine
DMPE, 1,2-dimyristoyl-sn-glycero-3-phosphoethanolamine
DMPG,1,2-dimyristoyl-sn-glycero-3-phospho-(1'-rac-glycerol)
EPR, electron paramagnetic resonance
FACS, fluorescence-activated cell sorting
FCR, fluorescence colorimetric response
GUVs, giant unilamellar vesicles
HPLC, high performance liquid chromatography
IR, infrared
MNPs, Magnetic Nanoparticles

MRI, magnetic resonance imaging
NMR, nuclear magnetic resonance
NPs, nanoparticles
PBS, phosphate buffered saline
PC, phosphatidylcholine
PDA, polydiacetylene.
TMA-DPH, 1-(4 trimethylammoniumphenyl)-6-phenyl-1,3,5-hexatriene

Example 1

Bolaamphiphile synthesis

The boloamphiphles or bolaamphiphilic compounds of the invention can be synthesized following the procedures described previously (see below).

Briefly, the carboxylic group of methyl vernolate or vemolic acid was interacted with aliphatic diols to obtain bisvernolesters. Then the epoxy group of the vernolate moiety, located on C12 and C13 of the aliphatic chain of vernolic acid, was used to introduce two ACh headgroups on the two vicinal carbons obtained after the opening of the oxirane ring. For GLH-20 (Table 1), the ACh head group was attached to the vernolate skeleton through the nitrogen atom of the choline moiety. The bolaamphiphile was prepared in a two-stage synthesis: First, opening of the epoxy ring with a haloacetic acid and, second, quaternization with the *N,N*-dimethylamino ethyl acetate. For GLH-19 (Table 1) that contains an ACh head group attached to the vernolate skeleton through the acetyl group, the bolaamphiphile was prepared in a three-stage synthesis, including opening of the epoxy ring with glutaric acid, then esterification of the free carboxylic group with *N,N*-dimethyl amino ethanol and the final product was obtained by quaternization of the head group, using methyl iodide followed by exchange of the iodide ion by chloride using an ion exchange resin.

Each bolaamphiphile was characterized by mass spectrometry, NMR and IR spectroscopy. The purity of the two bolaamphiphiles was >97% as determined by HPLC.

Materials. Iron(III) acetylacetonate ($Fe(acac)_3$), diphenyl ether, 1,2-hexadecanediol, oleic acid, oleylamine, and carboxyfluorescein (CF) were purchased from Sigma Aldrich (Rehovot, Israel). Chloroform and ethanol were purchased from Bio-Lab Ltd. Jerusalem, Israel. 1,2-dimyristoyl-sn-glycero-3-phospho-(1'-rac-glycerol) (DMPG), 1,2-dimyristoyl-sn-glycero-3-phosphoethanolamine (DMPE), 1,2-dimyristoyl-sn-glycero-3-phosphocholine (DMPC), cholesterol (CHOL), cholesteryl hemisuccinate (CHEMS) were purchased from Avanti Lipids (Alabaster, AL, USA), The diacetylenic monomer 10,12- tricosadiynoic acid was purchased from Alfa Aesar (Karlsruhe, Germany), and purified by dissolving the powder in chloroform, filtering the resulting solution through a 0.45 μm nylon filter (Whatman Inc., Clifton, NJ, USA), and evaporation of the solvent. 1-(4 trimethylammoniumphenyl)-6-phenyl-1,3,5-hexatriene (TMA-DPH) was purchased from Molecular Probes Inc. (Eugene, OR, USA).

**SYNTHESIS OF REPRESENTATIVE BOLAAMPHIPHILIC COMPOUNDS**

The synthesis bolaamphiphilic compounds of this invention can be carried out in accordance with the methods described previously (Chemistry and Physics of Lipids 2008, 153, 85-97; Journal of Liposome Research 2010, 20, 147-59; WO2002/055011; WO2003/047499; or WO2010/128504) and using the appropriate reagents, starting materials, and purification methods known to those skilled in the art. Table 1 lists the representative bolaamphiphilic compounds.

Table 1: Representative Bolaamphiphiles

| # | Structure |
|---|---|
| GLH-3 | 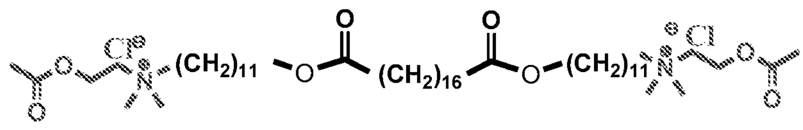 |
| GLH-4 | 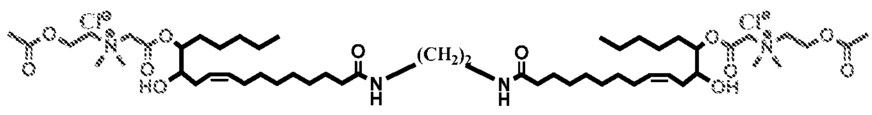 |

(continued)

| # | Structure |
|---|---|
| **GLH-5** | 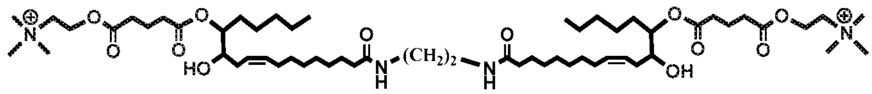 |
| **GLH-6**[a] | 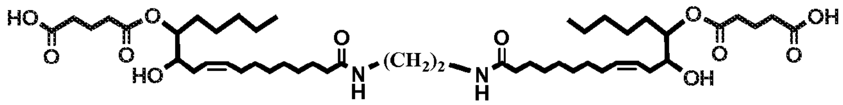 |
| **GLH-7** | 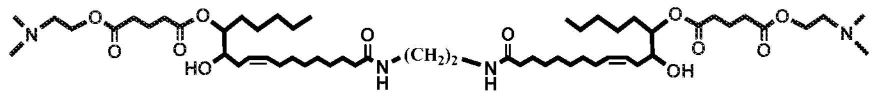 |
| **GLH-8** | 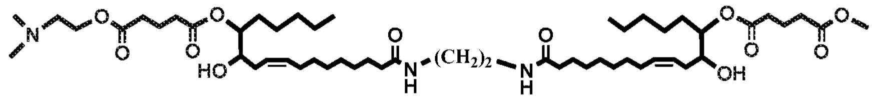 |
| **GLH-9** | 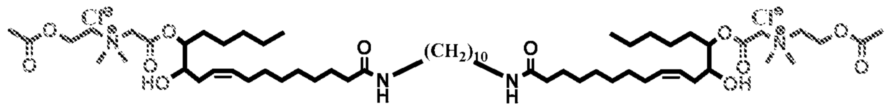 |
| **GLH-10** | 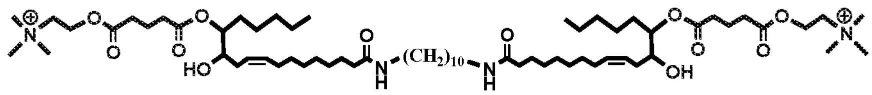 |
| **GLH-11** | 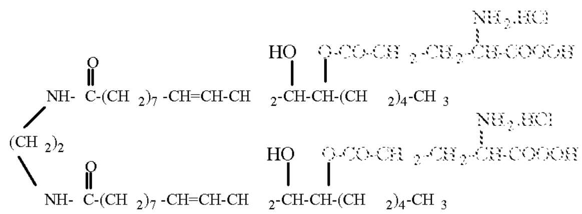 |
| **GLH-12** [a] | 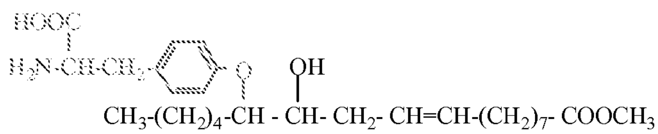 |
| **GLH-13** [a] **GLH-13** [a] | 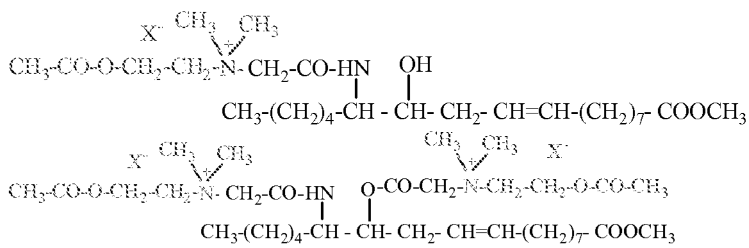 |
| **GLH-14** | 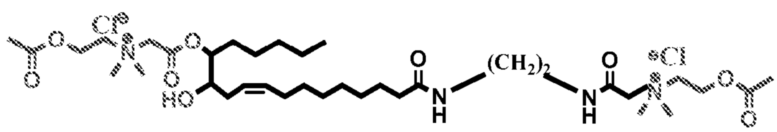 |
| **GLH-15** | |

(continued)

| # | Structure |
|---|---|
| | 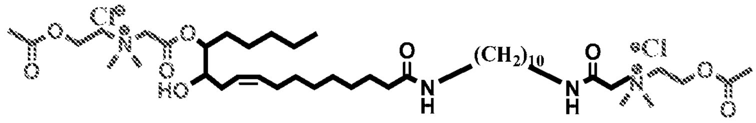 |
| GLH-16 | 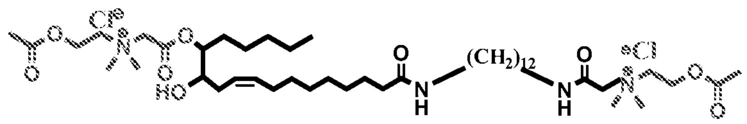 |
| GLH-17 | 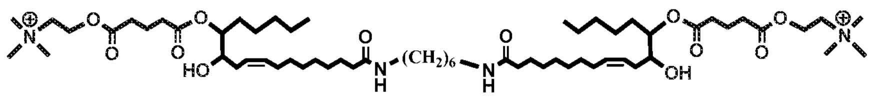 |
| GLH-18 | 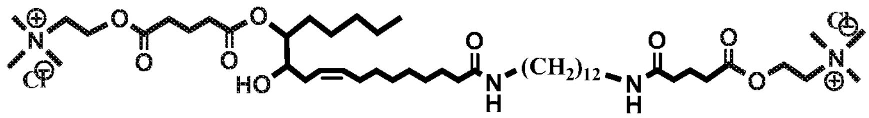 |
| GLH-19 | 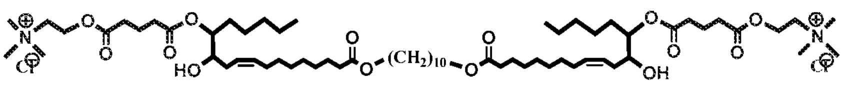 |
| GLH-20 | 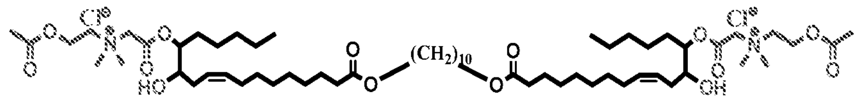 |
| GLH-21 | 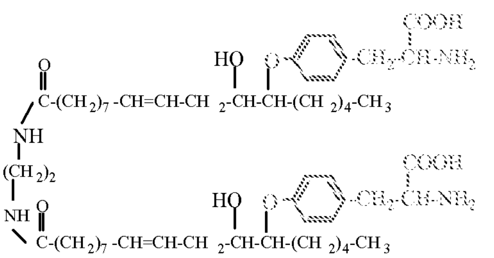 |
| GLH-22 | 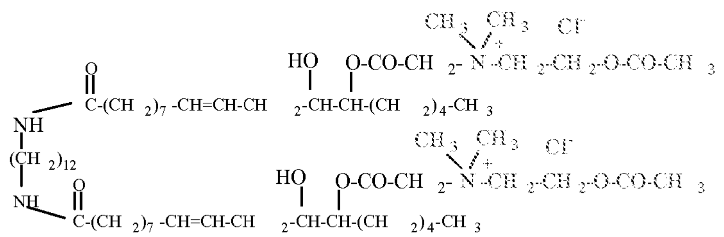 |
| GLH-23 | 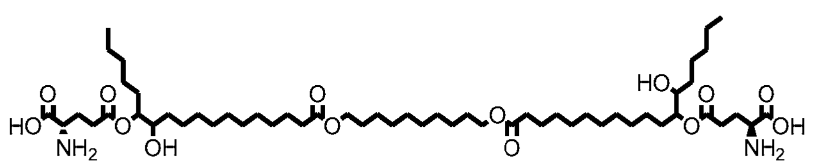 |
| GLH-24 | 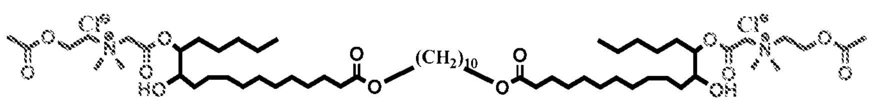 |
| GLH-25 | 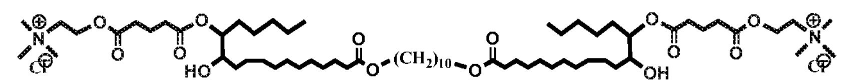 |

(continued)

| # | Structure |
|---|---|
| # | Structure |
| GLH-26 | 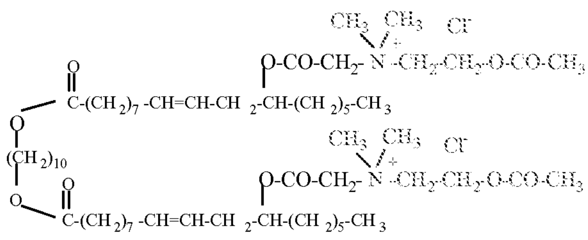 |
| GLH-27 | 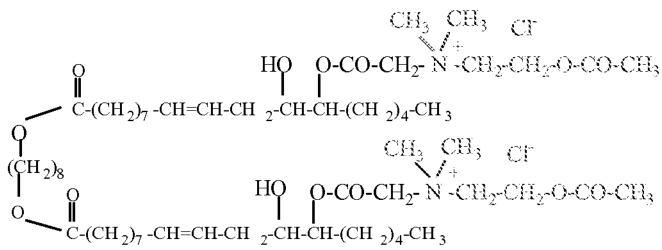 |
| GLH-28 | 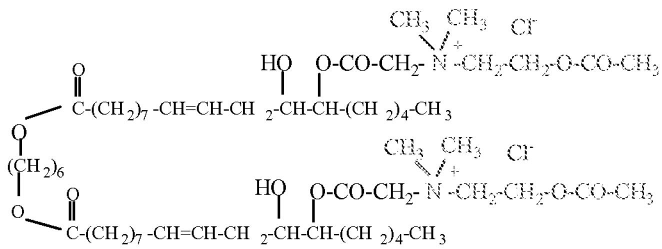 |
| GLH-29 | 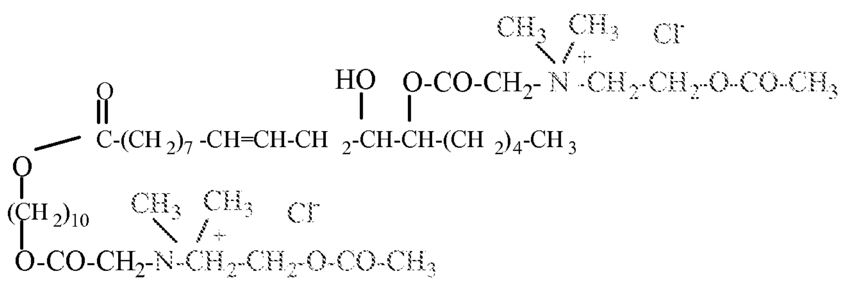 |
| GLH-30 | 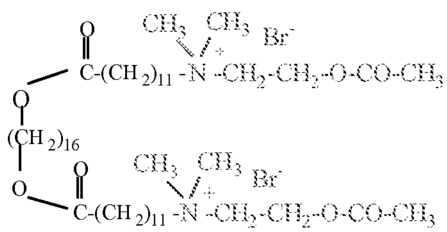 |
| GLH-30 | 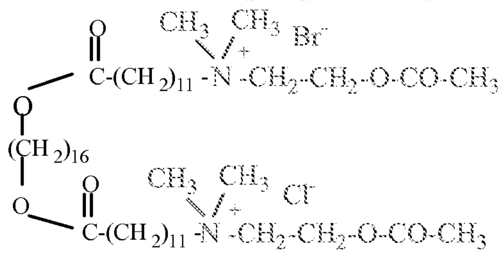 |

(continued)

| # | Structure |
|---|---|
| **GLH-31** | 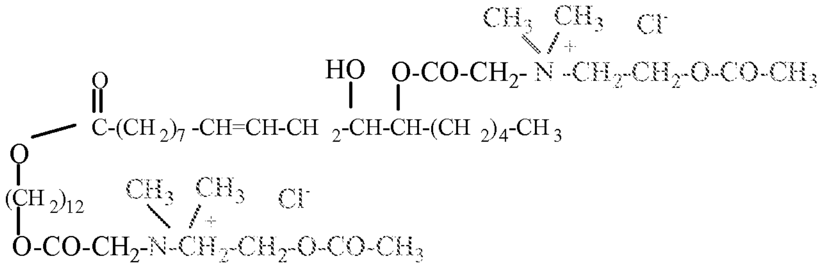 |
| **GLH-32** | 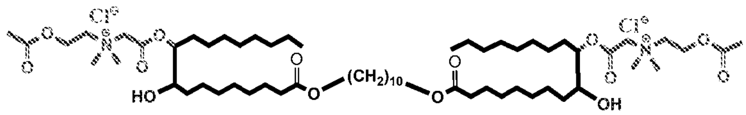 |
| **GLH-33** | 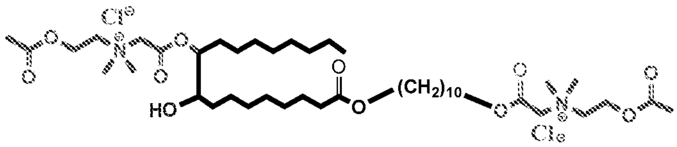 |
| **GLH-34** | 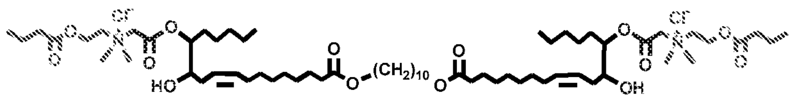 |
| **GLH-35** | 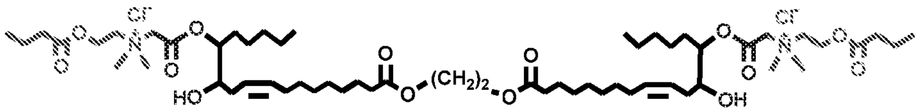 |
| **GLH-36** | 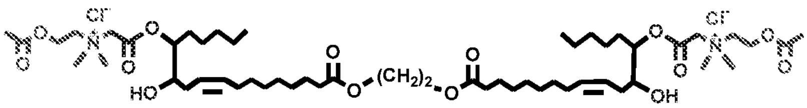 |
| **GLH-37** | 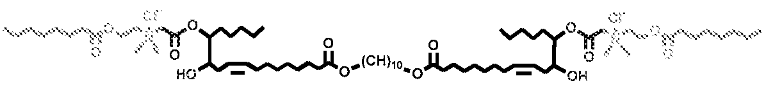 |
| **GLH-38** | 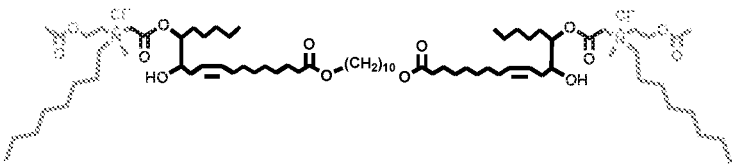 |
| **GLH-39**[a] | 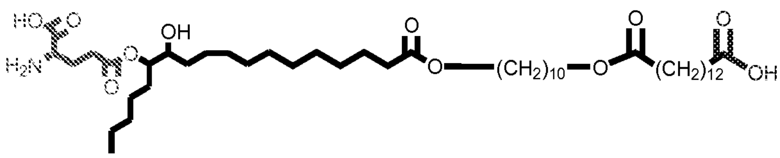 |
| **GLH-40** | 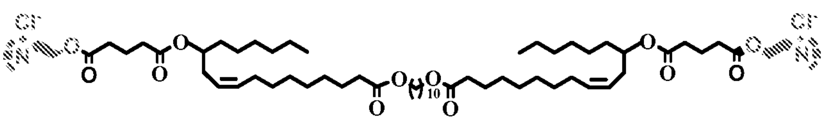 |

(continued)

| # | Structure |
|---|---|
| GLH-41 | 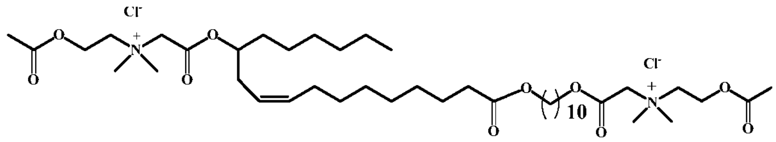 |
| GLH-42 [a] | 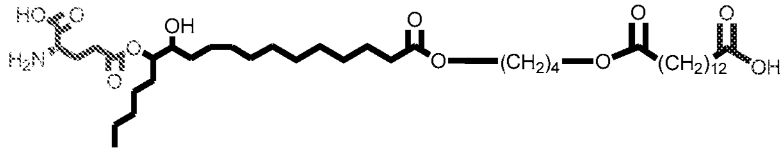 |
| GLH-43 [a] | 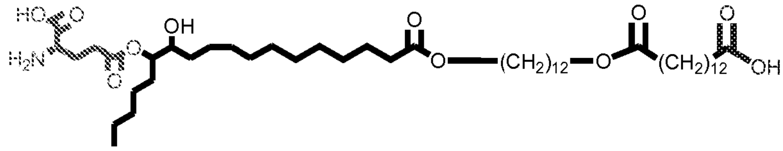 |
| GLH-44 | 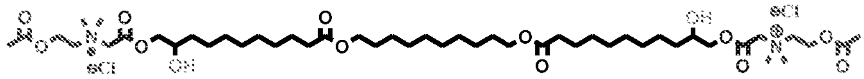 |
| GLH-45 | 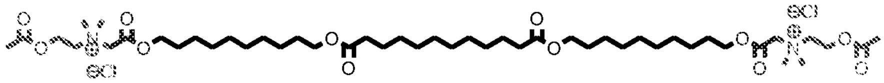 |
| GLH-46 | |
| | 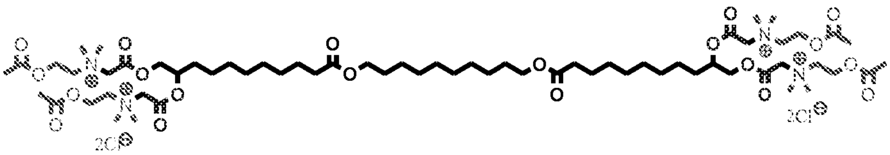 |
| GLH-47 | 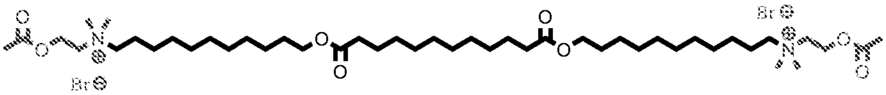 |
| GLH-48 | 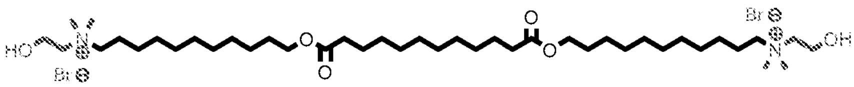 |
| GLH-49 [a] | 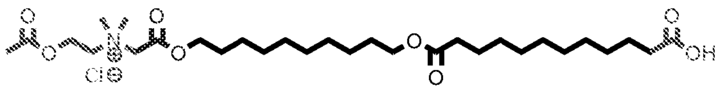 |
| GLH-50[a] | 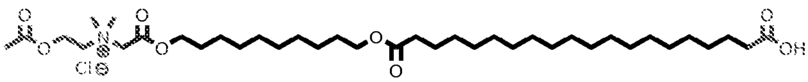 |
| GLH-51 [a] | 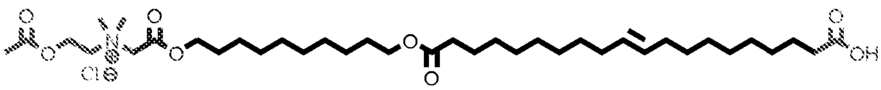 |
| GLH-52[a] | 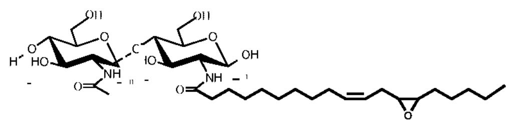 |

(continued)

| # | Structure |
|---|---|
| GLH-53[a] | 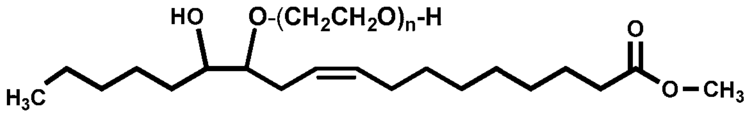 |
| GLH-54[a] | 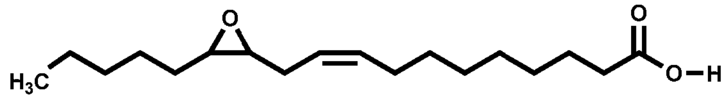 |
| GLH-55 | 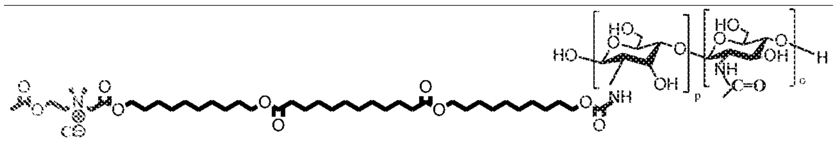 |
| GLH-56 | 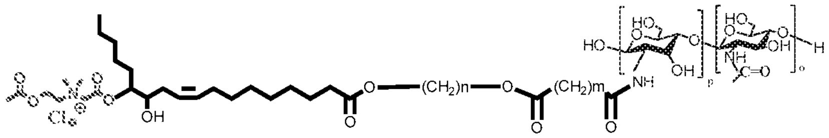 |
| GLH-57 | 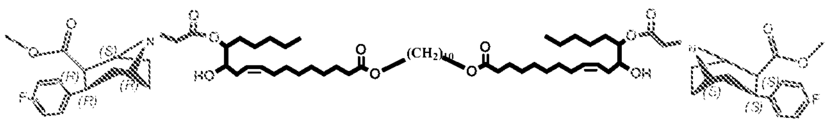 |
| [a] - an intermediate | |

Example 2

Bolavesicle preparation and characterization

Bolaamphiphiles, cholesterol, and CHEMS (2:1:1 mole ratio) are dissolved in chloroform or a suitable solvent. 0.5 mg of the biologically active drug dispersed in chloroform is added to the mix. The solvents are evaporated under vacuum and the resultant thin films are hydrated in 0.2 mg/mL CF solution in PBS and probe-sonicated (Vibra-Cell VCX130 sonicator, Sonics and Materials Inc., Newtown, CT, USA) with amplitude 20%, pulse on: 15 sec, pulse off: 10 sec to achieve homogenous vesicle dispersions. Vesicle size and zeta potential were determined using a Zetasizer Nano ZS (Malvern Instruments, UK). The amount of the biologically active drug encapsulated in the vesicles can be determined by HPLC and/or UV spectroscopy (G Gnanarajan, et al, 2009) after separating the non-encapsulated drug, by size exclusion chromatography (on Sephadex-G50).

Spectral Characterization

Example 3

Electron paramagnetic resonance (EPR)

EPR spectra of biologically active drug embedded bolavesicles resuspended in PBS can be obtained using a Bruker EMX-220 X-band (υ~9.4 GHz) EPR spectrometer equipped with an Oxford Instruments ESR 900 temperature accessories and an Agilent 53150A frequency counter. Spectra can be recorded at room temperature with the non-saturating incident microwave power 20 mW and the 100 KHz magnetic field modulation of 0.2 mT amplitude. Processing of EPR spectra, determination of spectral parameters can be done using Bruker WIN-EPR software.

Example 4

Cryogenic transmission electron microscopy (cryo-TEM)

Specimens studied by cryo-TEM were prepared. Sample solutions (4 μL) are deposited on a glow discharged, 300 mesh, lacey carbon copper grids (Ted Pella, Redding, CA, USA). The excess liquid is blotted and the specimen was vitrified in a Leica EM GP vitrification system in which the temperature and relative humidity are controlled. The samples are examined at -180 °C using a FEI Tecnai 12 G2 TWIN TEM equipped with a Gatan 626 cold stage, and the images are recorded (Gatan model 794 charge-coupled device camera) at 120 kV in low-dose mode. Figure 1 shows TEM micrograph of vesicles from GLH-20 (A) and their size distribution determined by DLS (B).

Assays

Example 5

Lipid/polydiacetylene (PDA) assay

Lipid/polydiacetylene (PDA) vesicles (PDA/DMPC 3:2, mole ratio) are prepared by dissolving the lipid components in chloroform! ethanol and drying together *in vacuo.* Vesicles are subsequently prepared in DDW by probe-sonication of the aqueous mixture at 70°C for 3 min. The vesicle samples are then cooled at room temperature for an hour and kept at 4°C overnight. The vesicles are then polymerized using irradiation at 254 nm for 10-20 s, with the resulting emulsions exhibiting an intense blue appearance. PDA fluorescence is measured in 96-well microplates (Greiner Bio-One GmbH, Frickenhausen, Germany) on a Fluoroscan Ascent fluorescence plate reader (Thermo Vantaa, Finland). All measurements are performed at room temperature at 485 nm excitation and 555 nm emission using LP filters with normal slits. Acquisition of data is automatically performed every 5 min for 60 min. Samples comprised 30 μL of DMPC/PDA vesicles and 5μL bolaamphiphilic vesicles assembled with biologically active drug, followed by addition of 30 μL 50 mM Tris-base buffer (pH 8.0).

A quantitative value for the increasing of the fluorescence intensity within the PDA/PC-labeled vesicles is given by the fluorescence colorimetric response (%FCR), which is defined as follows[27]:

$$\text{Eq. 1.} \qquad \%FCR = [(F_I - F_0)/F_{100}] \cdot 100$$

Where $F_I$ is the fluorescence emission of the lipid/PDA vesicles after addition of the tested membrane-active compounds, $F_0$ is the fluorescence of the control sample (without addition of the compounds), and $F_{100}$ is the fluorescence of a sample heated to produce the highest fluorescence emission of the red PDA phase minus the fluorescence of the control sample.

Example 6

Cell culture

b.End3 immortalized mouse brain capillary endothelium cells are kindly provided by Prof. Philip Lazarovici (Institute for Drug Research, School of Pharmacy, The Hebrew University of Jerusalem, Israel). The b.End3 cells were cultured in DMEM medium supplemented with 10% fetal bovine serum, 2 mM L-Glutamine, 100 IU/mL penicillin and 100 μg/mL streptomycin (Biological Industries Ltd., Beit Haemek, Israel). The cells are maintained in an incubator at 37°C in a humidified atmosphere with 5% $CO_2$.

Example 7

Internalization of CF by the cells *in vitro*

b.End3 cells are grown on 24-well plates or on coverslips (for FACS and fluorescence microscopy analysis, respectively). The medium is replaced with culture medium without serum and CF solution, or tested bolavesicles (equivalent to 0.5 μg/mL CF), or equivalent volume of the medium are added to the cells and incubated for 5 hr at 4°C or at 37°C. At the end of the incubation, cells are extensively washed with complete medium and with PBS, and are either detached from the plates using trypsin-EDTA solution (Biological Industries Ltd., Beit Haemek, Israel) and analyzed by FACS (FACSCalibur Flow Cytometer, BD Biosciences, USA), or fixed with 2.5% formaldehyde in PBS, washed twice

with PBS, mounted on slides using Mowiol-based mounting solution and analyzed using a FV1000-IX81 confocal microscope (Olympus, Tokyo, Japan) equipped with 60x objective. All the images are acquired using the same imaging settings and are not corrected or modified. The Figure 2 shows head group hydrolysis by AChE (**A**) of GLH-19 (blue) and GLH-20 (red) and release of CF from GLH-19 vesicles (**B**) and GLH-20 vesicles (**C**). AChE causes the release of encapsulated material from GLH-20 vesicles, but not from GLH-19 vesicles (Fig.2). The vesicles are capable of delivering small molecules, such as carboxyfluorescein (CF), into a mouse brain, but the fluorescent dye accumulates only if it is delivered in vesicles that release their encapsulated CF in presence of AChE, namely, GLH-20 vesicles (Fig. 3A). These results suggest that the release is due to head group hydrolysis by AChE in the brain. Corroboration for this conclusion also comes from an experiment showing that when an analgesic peptide is delivered to the brain by the bola vesicles, analgesia (which is caused when the encapsulated peptide is released in the brain) was observed only with GLH-20 vesicles, but not by GLH-19 vesicles (Figure 4A).The vesicles do not break the BBB, but rather penetrate it in their intact form, as indicated by the finding that analgesia is obtained only when enkephalin is administered while encapsulated within the vesicles, but not when free enkephalin is administered together with empty vesicles (Figure 4B).

The ACh head groups also provide the vesicles with cationic surfaces, which promote penetration through the BBB [Lu et al, 2005] and transport of the encapsulated material into the brain. Toxicity studies showed that the dose which induced the first toxic signs was 10-20 times higher than the doses needed to obtain analgesia by encapsulated analgesic peptides.

The addition of chitosan (CS) surface groups, by employing CS-vernolate conjugates, increased BBB permeability of the vesicles (Figure 4B), probably by increasing transcytosis [Newton, 2006]. However, the CS groups, when added to the vesicles by employing fatty acid-CS conjugate (in this case, vernolic acid), are not stable in circulation as surface groups because of the low energy barrier for lipid exchange of such conjugates. The inventors propose to make stabilized CS surface groups by using bolas that the inventors will synthesize with covalently-attached CS head groups [see, Experimental Design and Methods, below].

In addition to the peptide leu-enkephalin, and the small molecules: CF, uranyl acetate, kyotorphin and sucrose, the inventors have also successfully encapsulated in these vesicles the proteins albumin and trypsinogen and the polysaccharide Dextran-FITC (MW 9000). Albumin-FITC, encapsulated, was delivered successfully to the brain (Fig. 5B), while unencapsulated albumin-FITC showed little, if any, brain accumulation (Fig 5A), indicating that the vesicle transported the protein into the brain through the BBB. These results strongly suggest that the vesicles can be made to encapsulate other molecules, such as anti-retroviral drugs, and deliver them into the brain without harming the BBB.

Example 8

Statistical analysis

The data are presented as mean and standard deviations (SD) or standard errors of mean (SEM). Statistical differences between the control and the studied formulations are analyzed using ANOVA followed by Dunnett post-test using InStat 3.0 software (GraphPad Software Inc., La Jolla, CA, USA). P values of less than 0.05 are defined as statistically significant.

Example 9

Encapsulation of CPT-11

A) Optimization of vesicle formation: Vesicles are prepared by film hydration, followed by sonication. Each of the vesicle formulations can be examined for vesicle size (by dynamic light scattering), morphology (by cryo-transmission electron microscopy), zeta potential (by Zeta Potential Analyzer) and stability (by fluorescence measurements of encapsulated CF at various times after vesicle preparation). Stability of vesicles can be determined in presence and absence of ChE, with and without an inhibitor of the enzyme (e.g., pyridostigmine).

B) Encapsulation of CPT-11: To successfully encapsulate CPT-11 (MW 586.67, water solubility of 25 mg/ml with bis-piperidine moiety, which forms an ammonium salt in acid) within the vesicles, the active loading approach can be used. CPT-11 can be encapsulated in its active lactonic form, and not in the inactive carboxylate form. The loading conditions based on conditions developed for liposomal formulations using a pH gradient between the liposome core can be used and the bathing medium, whereas the internal volume can be acidic compared to the external solution.

For encapsulations, vesicles can be formed in acidic buffers, such as citrates. The vesicles can be purified on a GPC column to separate encapsulated CPT-11 from non-encapsulated material. Percent encapsulation can be determined by UV absorption of the CTP-11's aromatic groups after lysis with a detergent. To maximize CPT-11 loading and minimize leakage, the composition of the vesicle's membrane can be optimized by varying both the ratio between bolaamphiphiles in the vesicle formulation and the proportion of different additives used in the vesicle formulation, such as cholesterol hemisuccinate and neutral cholesterol; or drug-loading with respect to the relative concentration of CPT-1 1 to vesicles, the temperature during loading, internal buffer composition and the pH gradient across the vesicle's membrane.

The entrapped CPT-11 may be stabilized by adding, to the vesicle core, agents that help to prevent leakage, such as dextran sulfate28, copper sulfate and other transition metal salts29, and polymeric or highly charged nonpolymeric polyanionic trapping agents.

Example 10

Determination of encapsulated CPT-11 activity

To ensure that the encapsulation process did not reduce the cytotoxic activity of CPT-11, the encapsulated CPT-11 can be released from the vesicles by ChE treatment, and the released CPT-11 can be collected from the supernatant following centrifugation. The $IC_{50}$ of the released CPT-11 can be determined by using U87 glioblastoma cell line and by a standard viability assay (e.g., MTT) in comparison to that of standard CPT-11.

As described here a novel formulations of bolavesicles can be produced through co-assembly of biologically active drugs with bolaamphiphile/lipid unilamellar vesicles. The formulations can be examined for their chemical and biophysical properties.

The incorporation of biologically active drug within the bolavesicles is shown to significantly modulate interactions with membrane bilayers in model systems. This observation is important, suggesting that biologically active drugs encapsulated in bolavesicles might be excellent candidates for targeting and transport of different molecular cargoes into the brain.

At least some of the chemical names of compounds of the invention as given and set forth in this application, may have been generated on an automated basis by use of a commercially available chemical naming software program, and have not been independently verified. Representative programs performing this function include the Lexichem naming tool sold by Open Eye Software, Inc. and the Autonom Software tool sold by MDL, Inc. In the instance where the indicated chemical name and the depicted structure differ, the depicted structure will control.

Chemical structures shown herein were prepared using ISIS®/DRAW. Any open valency appearing on a carbon, oxygen or nitrogen atom in the structures herein indicates the presence of a hydrogen atom. Where a chiral center exists in a structure but no specific stereochemistry is shown for the chiral center, both enantiomers associated with the chiral structure are encompassed by the structure.

REFERENCES

*Abu Hammad I, Popov M, Linder C, Grinberg S, Heldman E, Stepensky D (2011) Bolaamphiphilic nanovesicles for the delivery of proteins to the brain, submitted to the Journal of Controlled Release.

Agyare, EK, Kandimalla KK, Poduslo JF, Yu CC, Ramakrishnan M, Curran GL (2008) Development of a smart nanovesicle to target cerebrovascular amyloid deposits and brain parenchymal plaques observed in Alzheimer's disease and cerebral amyloid angiopathy. Pharm Res Nov; 25(11):2674-2684.

Fuhrhop J.H. and Wang T. (2004) Bolaamphiphiles, Chem. Rev. 104:2901-2937.

Gisslen M and Hagberg L and Hagberg (2001) Antiretroviral treatment of central nervous system HIV-1infection: a Review. HIV Medicine (2001) 2, 97-104.

G Gnanaraian, AK Gupta, V Juval, P Kumar, PK Yadav, P Kailash "A validated method for development of tenofovir as API and tablet dosage forms by UV spectroscopy" Pharm Analysis 2009 Vol 1 Issue 4 pp 351-353.

*Grinberg S, C. Linder, E. Heldman, Z. Weizman, and V. Kolot : EP1360168, 2003-11-12 and WO2002IL00043and 20020116, Filed by BG Negev "Amphiphilic Derivatives for the Production of Vesicles, Micelles, Complexants, and Uses Thereoff" in 2003

*Grinberg S., Linder C., Kolot V., Waner T., Wiesman Z., Shaubi E., Heldman E. (2005) Novel cationic amphiphilic derivatives from vemonia oil: synthesis and self-aggregation into bilayer vesicles, nanoparticles, and DNA complexants. Langmuir. 21(17):7638-7645.

*Grinberg S., Kolot V., Linder C., Shaubi E., Kas'yanov V., Deckelbaum R.J., Heldman E. (2008) Synthesis of novel

cationic bolaamphiphiles from vemonia oil and their aggregated structures. Chem Phys Lipids 153(2):85-97.
*Grinberg, S., Kipnis, N., Linder, C., Kolot, V. and Heldman, E., (2010) Assymetric bolaamphiphiles from veronica oil designed for drug delivery. Eur. J. Lipid Sci. Technol., 112, 137-151.
*E. Heldman E, C. Linder, S. Grinberg Amphiphilic compounds and vesicles liposomes for organ-specified drug targeting" US patent Application 20060039962 + WO03047499 - 2003-06-12
Highleyman, L (2009) HIV and the Brain BETA. 2009 Summer-Fall;21(4):16-29.
*Hutter T, Linder C, Heldman E, Grinberg S (2011) Interfacial and self-assembly properties of bolaamphiphilic compounds derived from a multifunctional oil, Journal of Colloid and Interface Science, in press (doi: 10. 1016/j.jcis..08. 057).
Jonasdottir TJ, Fisher DR, Borrebaek J, Bruland OS, Larsen RH (2006) First in vivo evaluation of liposome-encapsulated 223Ra as a potential alpha-particle-emitting cancer therapeutic agent. Anticancer Res. 26(4B):2841-2848.
Letendre S, Marquie-Beck J, Capparelli E, Best B, Clifford D, Collier AC, Gelman BB, McArthur JC, McCutchan JA, Morgello S, Simpson D, Grant I, Ellis RJ; CHARTER Group.(2008) Validation of the CNS Penetration-Effectiveness rank for quantifying antiretroviral penetration into the central nervous system. Arch Neurol. 2008 Jan;65(1):65-70.
*Linder C; Grinberg S; Heldman E "Nano-sized Particles Composing Multi-Headed Amphiphiles for Targeted Drug-Delivery" WO 2010128504 (A2) 2010.
Lu W, Tan YZ, Hu KL and Jiang XG. (2005) Cationic albumin conjugated pegylated nanoparticle with its transcytosis ability and little toxicity against blood-brain barrier. Int J Pharm. May 13;295 (1-2); 247-260.
New R.R.C. (ed). (1997) Liposomes. A Practical Approach. IRL Press, Oxford.
Newton HB (2006) Advances in strategies to improve drug delivery to brain tumors. Expert Rev Neurother. 6(10):1495-509.
*Popov M., Linder C., Deckelbaum R.J., Grinberg S., Hansen I.H., Shaubi E., Waner T., Heldman E. (2009) Cationic vesicles from novel bolaamphiphilic compounds. J Liposome Res. 20(2):147-159.
*Popov M, Grinberg S, Linder C, Bachar Z, Waner T, Deckelbaum R, Heldman E. (2011) Site-directed decapsulation of bolaamphiphilic vesicles with enzymatic cleavable surface groups submitted to the Journal of Controlled Release.
*Puri, A., Loomis, K., Smith, B., Lee, J., Yavlovich, A., Heldman, E. and Blumenthal, R. (2009) Lipid-Based Nanoparticles as Pharmaceutical Drug Carriers: From Concepts to Clinic. Crit Rev Ther Drug Carrier Syst, 26(6): 523-580.
Saiyed Z, Gandhi N, and Nairi M (2010)Magnetic Nanoformulation of Azidothymidine 5'-triphosphate for Targeted Delivery across the Blood-Brain Barrier. International Journal of Nanomedicine 5 :157-166
Songjiang Z and Lixiang W. (2009) Amyloid-Beta Associated with Chitosan Nano-Carrier has Favorable Immunogenicity and Permeates the BBB. AAPS Pharm Sci Tech, 10(3):900-905.
Spudich S and Antses B (2011) Central Nervous System Complications of HIV Infection. Top. Antiviral Med 19(2), 48-57.
Stem J, Freisleben HJ, Janku S, Ring K. (1992) Black lipid membranes of tetraether lipids from Thermoplasma acidophilum, Biochim Biophys Acta 1128:227-236.
Varatharajan L and Thomas S. (2009)The transport of anti-HIV drugs across blood-CNS interfaces: Summary of current knowledge and recommendations for further Research Antiviral Res. 2009 May ; 82(2): A99-A109.
*Wiesman Z., Dom N.B., Sharvit E., Grinberg S., Linder C., Heldman E., Zaccai M. (2007) Novel cationic vesicle platform derived from vemonia oil for efficient delivery of DNA through plant cuticle membranes. J Biotechnol. 130(1):85-94.
* Zabicky J; Linder C; Grinberg S; Heldman E "Nano - and Mesosized Particles Comprising an Inorganic Core, Process and Applications Thereof" US2009011002

## Claims

**1.** A pharmaceutical composition or a formulation comprising nano-sized vesicles; wherein the nano-sized vesicles comprise a bolaamphiphilic compound and a biologically active compound,

wherein the biologically active compound is CPT-11, the composition or formulation being for delivering the biologically active compound into a brain, and
wherein the bolaamphiphilic compound is GLH-20:

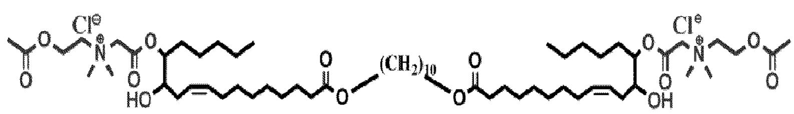 .

## Patentansprüche

1. Eine pharmazeutische Zusammensetzung oder eine Formulierung, die nanogroße Vesikel umfasst;

   wobei die nanogroßen Vesikel eine bolaamphiphile Verbindung und eine biologisch aktive Verbindung umfassen,
   wobei die biologisch aktive Verbindung CPT-11 ist, die Zusammensetzung oder Formulierung zum Einbringen der biologisch aktiven Verbindung in ein Gehirn dient, und
   wobei die bolaamphiphile Verbindung GLH-20 ist:

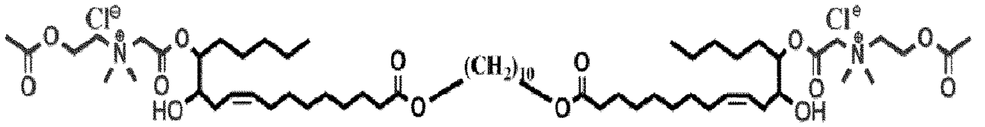
.

## Revendications

1. Une composition pharmaceutique ou une formulation comprenant des vésicules de taille nanométrique ;

   dans laquelle les vésicules de taille nanométrique comprennent un composé bolaamphiphile et un composé biologiquement actif,
   dans laquelle le composé biologiquement actif est le CPT-11, la composition ou la formulation étant destinée à administrer le composé biologiquement actif dans un cerveau, et
   dans laquelle le composé bolaamphiphile est le GLH-20 :

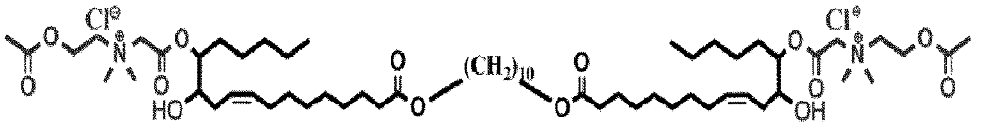
.

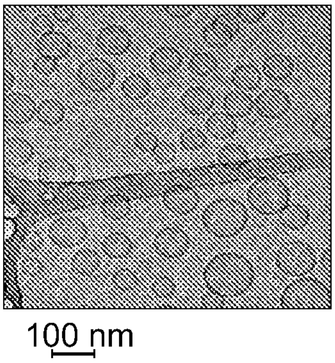
100 nm

FIG. 1A

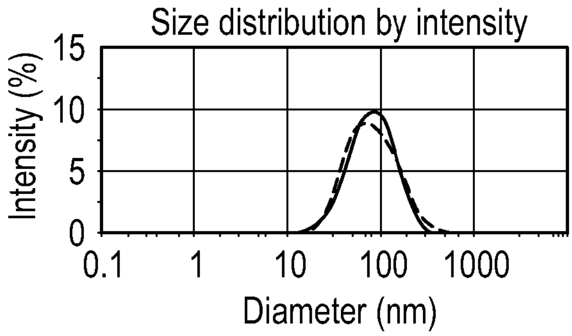
Size distribution by intensity
Intensity (%)
15
10
5
0
0.1
1
10
100
1000
Diameter (nm)

FIG. 1B

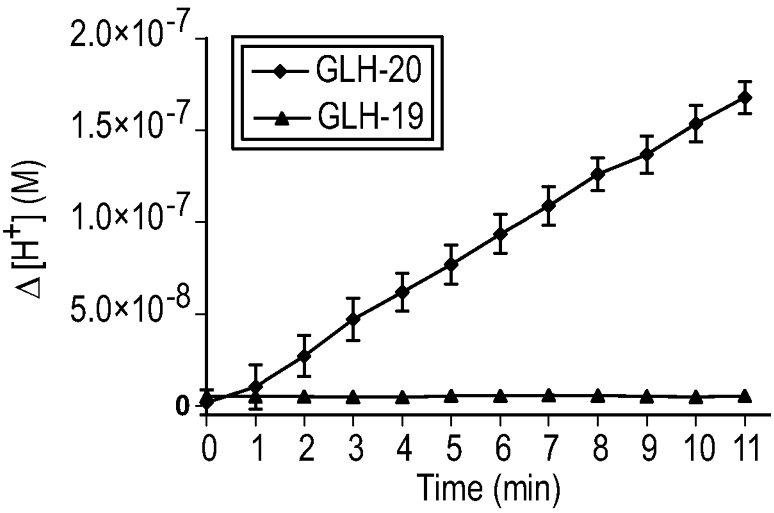
2.0×10-7
1.5×10-7
1.0×10-7
5.0×10-8
0
Δ [H+] (M)
GLH-20
GLH-19
0 1 2 3 4 5 6 7 8 9 10 11
Time (min)

*FIG. 2A*

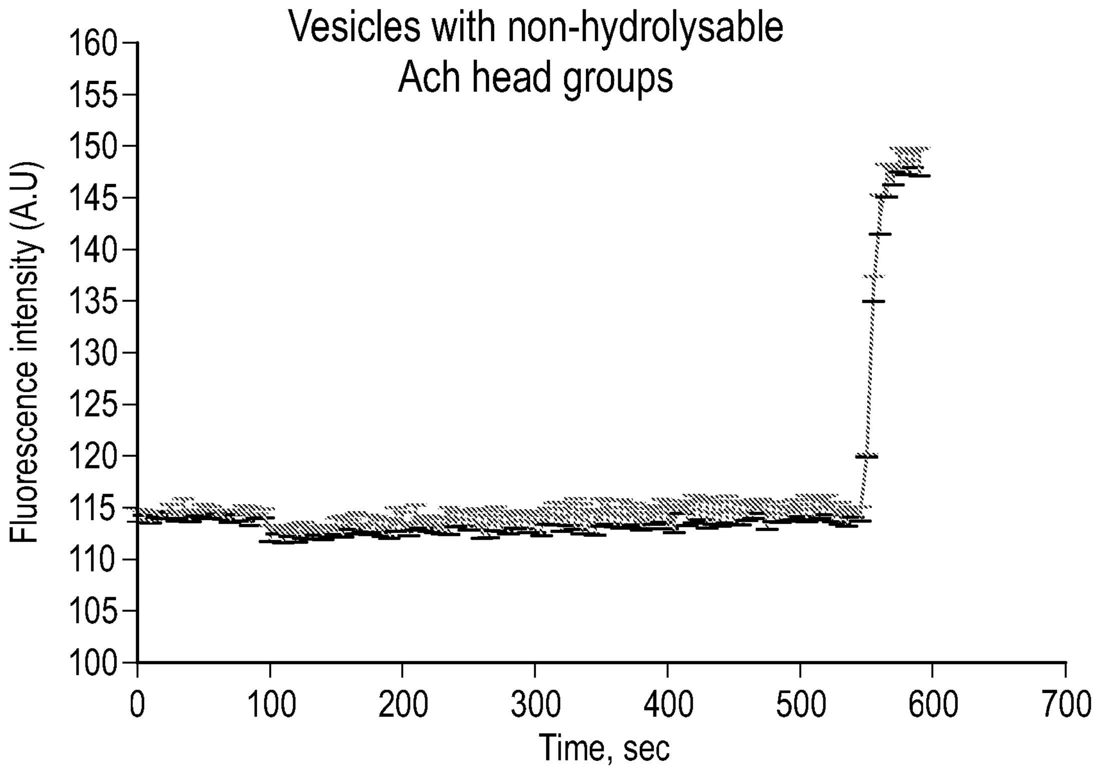
Vesicles with non-hydrolysable
Ach head groups
Fluorescence intensity (A.U)
160
155
150
145
140
135
130
125
120
115
110
105
100
0
100
200
300
400
500
600
700
Time, sec

*FIG. 2B*

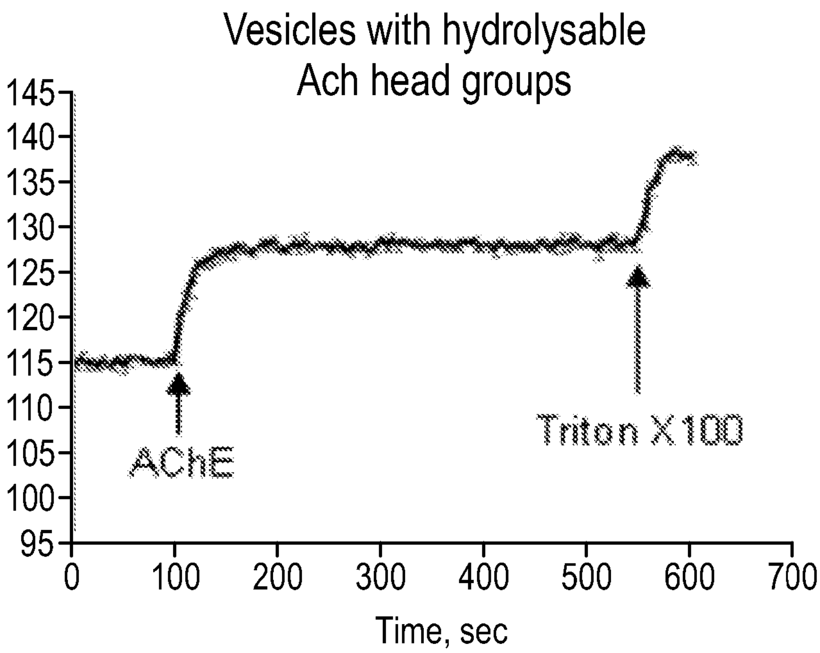
Vesicles with hydrolysable
Ach head groups
145
140
135
130
125
120
115
110
105
100
95
AChE
Triton X100
0
100
200
300
400
500
600
700
Time, sec

*FIG. 2C*

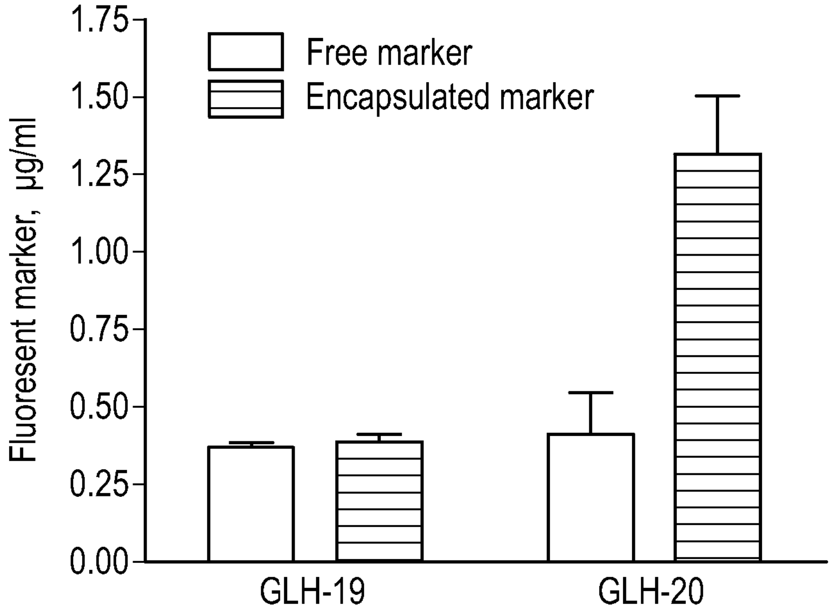
1.75
1.50
1.25
1.00
0.75
0.50
0.25
0.00
Fluoresent marker, µg/ml
Free marker
Encapsulated marker
GLH-19
GLH-20

*FIG. 3A*

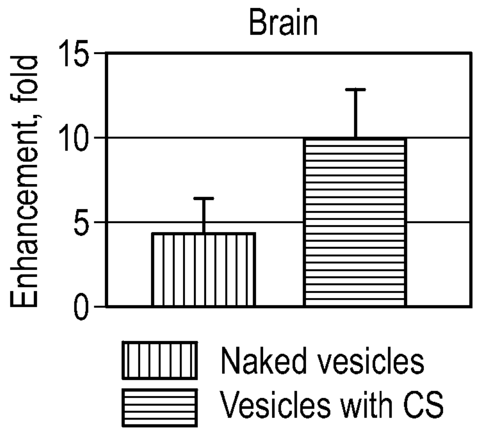
Brain
15
10
5
0
Enhancement, fold
Naked vesicles
Vesicles with CS

*FIG. 3B*

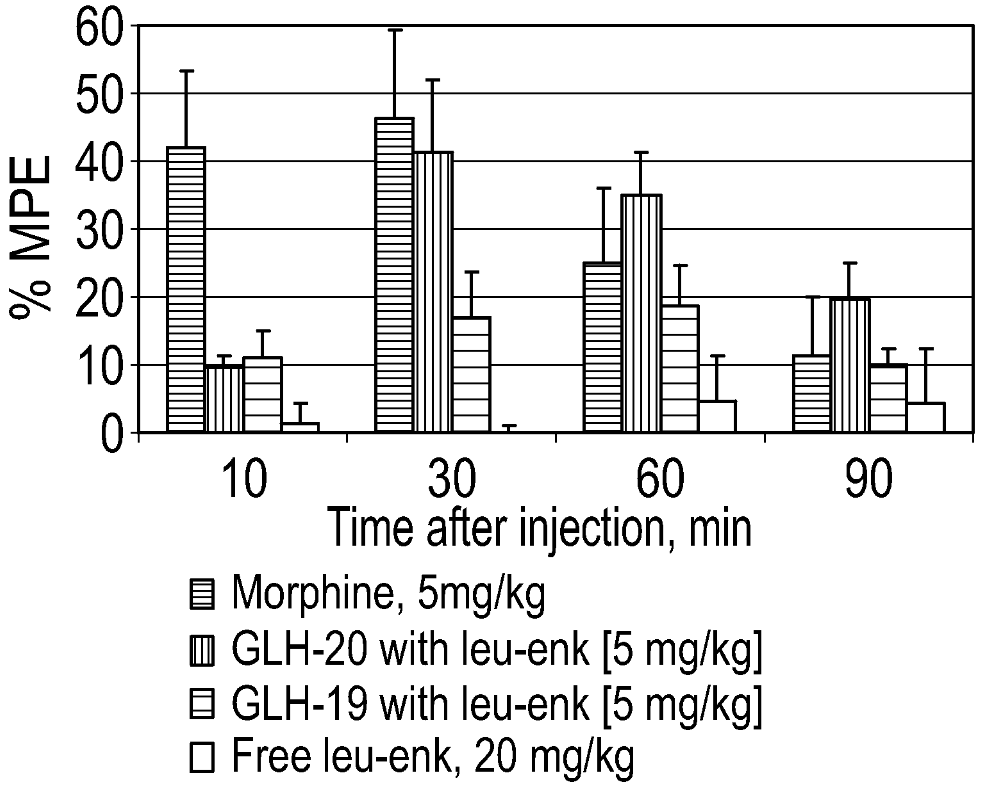
60
50
40
30
20
10
0
% MPE
10
30
60
90
Time after injection, min
Morphine, 5mg/kg
GLH-20 with leu-enk [5 mg/kg]
GLH-19 with leu-enk [5 mg/kg]
Free leu-enk, 20 mg/kg

*FIG. 4A*

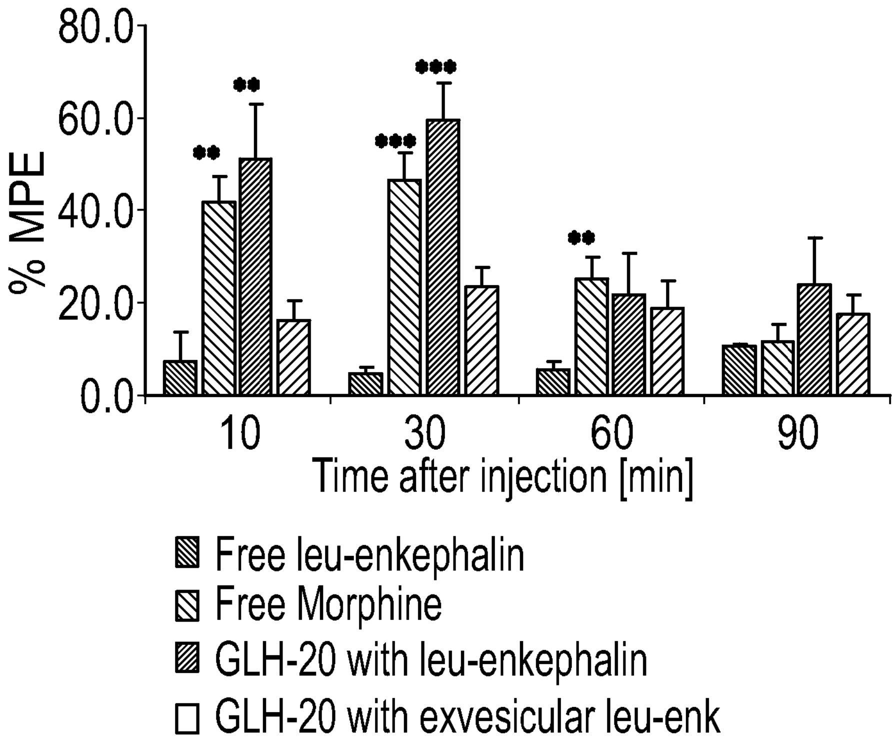
80.0
60.0
40.0
20.0
0.0
% MPE
**
***
10
30
60
90
Time after injection [min]
Free leu-enkephalin
Free Morphine
GLH-20 with leu-enkephalin
GLH-20 with exvesicular leu-enk

*FIG. 4B*

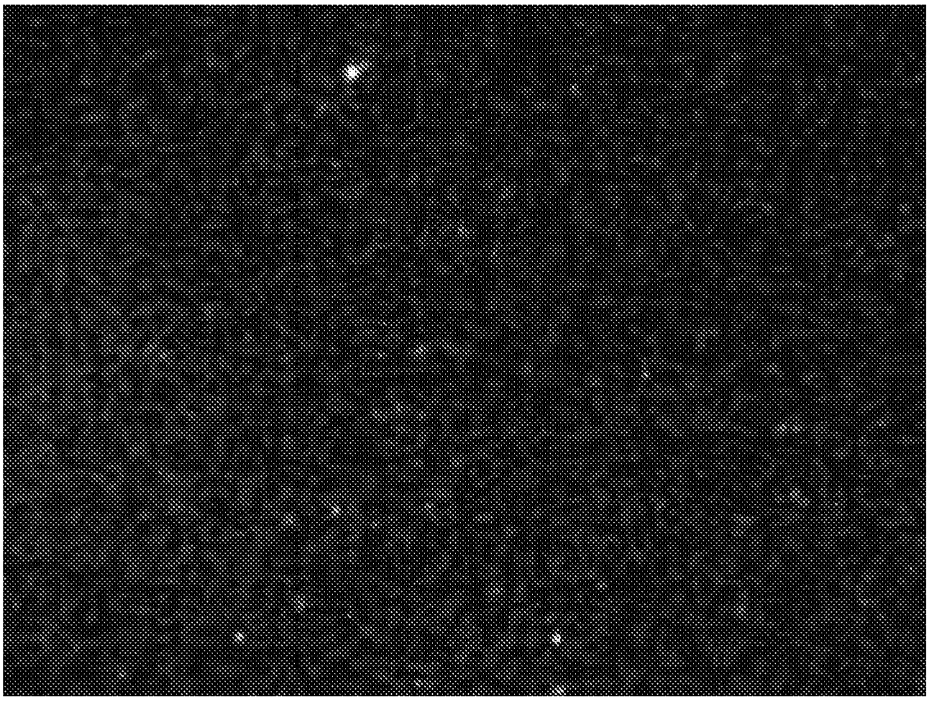

FIG. 5A

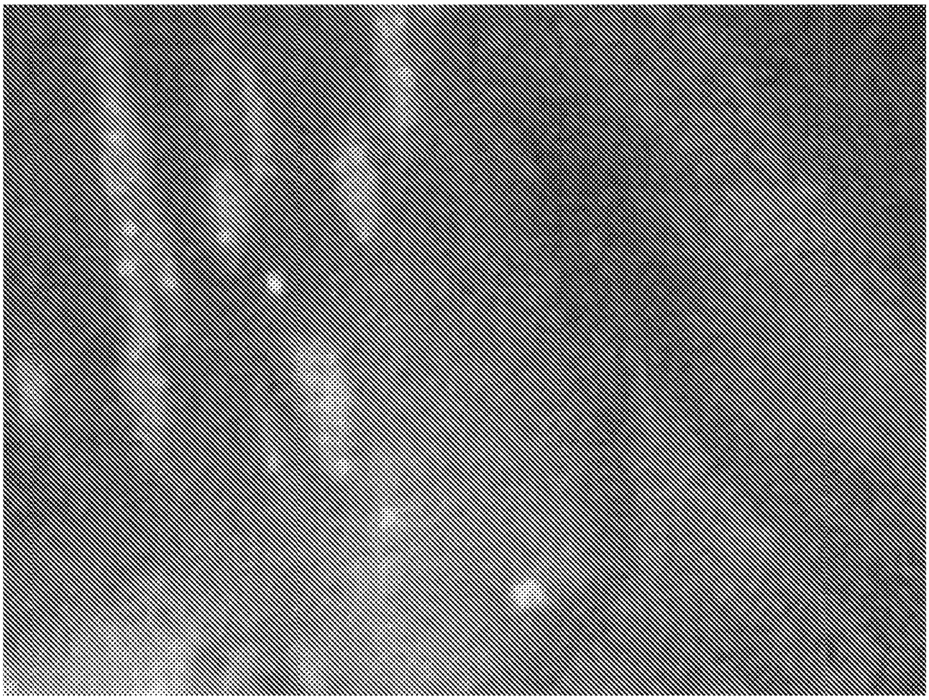

FIG. 5B

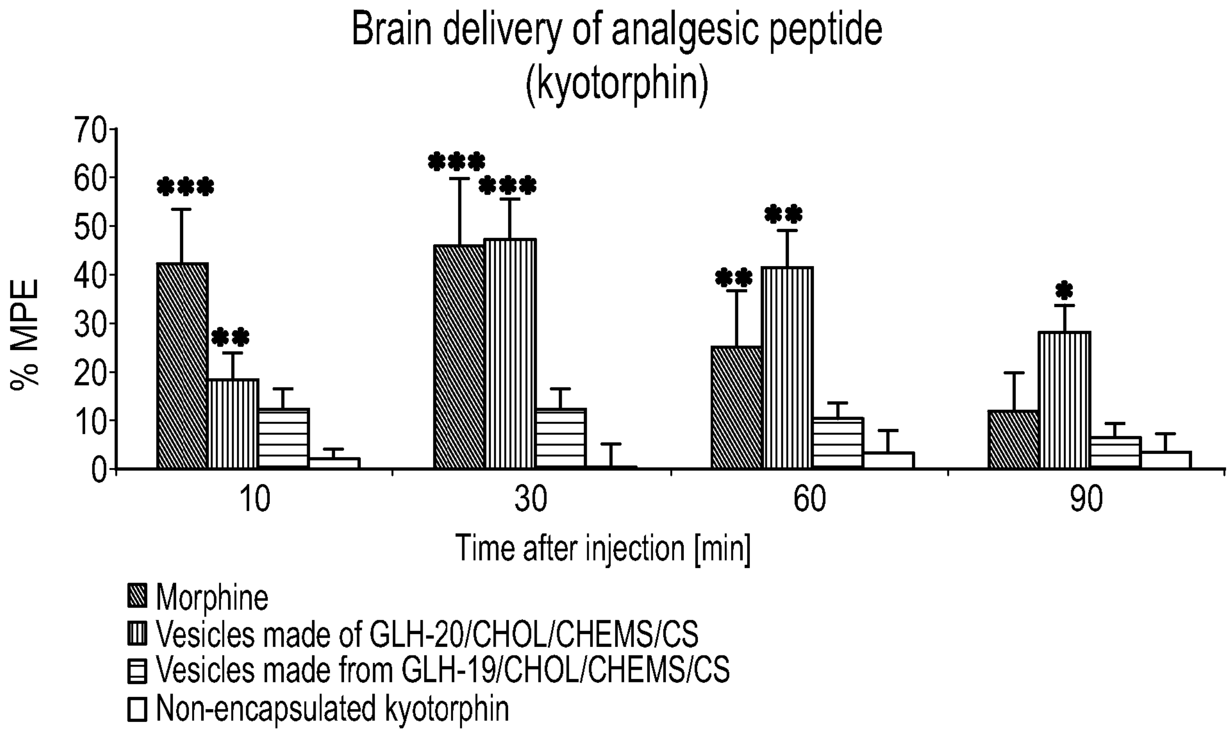
Brain delivery of analgesic peptide
(kyotorphin)
% MPE
70
60
50
40
30
20
10
0
***
**
***
***
**
**
*
10
30
60
90
Time after injection [min]
Morphine
Vesicles made of GLH-20/CHOL/CHEMS/CS
Vesicles made from GLH-19/CHOL/CHEMS/CS
Non-encapsulated kyotorphin

*FIG. 6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02055011 A
- WO 03047499 A
- WO 10128504 A
- WO 2010128504 A
- US 2008069868 A1
- WO 2002055011 A
- WO 2003047499 A
- EP 1360168 A, Grinberg S, C. Linder, E. Heldman, Z. Weizman, and V. Kolot
- WO 2002IL00043 A
- WO 20020116 A, BG Negev
- US 20060039962 A, E. Heldman E, C. Linder, S. Grinberg
- WO 2010128504 A2, Linder C; Grinberg S; Heldman E
- US 2009011002 A, Zabicky J; Linder C; Grinberg S; Heldman E


### Non-patent literature cited in the description


- **POPOV MARY et al.** Site-directed decapsulation of bolaamphiphilic vesicles with enzymatic cleavable surface groups. *JOURNAL OF CONTROLLED RELEASE,* vol. 160 (2), ISSN 0168-3659, 306-314
- Innovative bola-surfactant niosomes as topical delivery systems of 5-fluorouracil for the treatment of skin cancer. **PAOLINO et al.** INTERNATIONAL JOURNAL OF PHARMACEUTICS. ELSEVIER BV, vol. 353, 233-242
- Periodic Table of the Elements, CAS version. Handbook of Chemistry and Physics
- **THOMAS SORRELL.** Organic Chemistry. University Science Books, 1999
- **SMITH ; MARCH.** March's Advanced Organic Chemistry. John Wiley & Sons, Inc, 2001
- **LAROCK.** Comprehensive Organic Transformations. VCH Publishers, Inc, 1989
- **CARRUTHERS.** Some Modern Methods of Organic Synthesis. Cambridge University Press, 1987
- **JACQUES et al.** Enantiomers, Racemates and Resolutions. Wiley Interscience, 1981
- **WILEN et al.** *Tetrahedron,* 1977, vol. 33, 2725
- **ELIEL.** Stereochemistry of Carbon Compounds. McGraw-Hill, 1962
- **WILEN.** Tables of Resolving Agents and Optical Resolutions. Univ. of Notre Dame Press, 1972, 268
- **T. W. GREENE ; P. G. M. WUTS.** Protecting Groups in Organic Synthesis. John Wiley & Sons, 1999
- **BERGE et al.** *J. Pharm. Sci.,* January 1977, vol. 66 (1), 1-79
- **BUNDGARD, H.** Design of Prodrugs. Elsevier, 1985, 7-9, 21-24
- Remington 's Pharmaceutical Sciences. Mack Publishing Company, 1985
- Remington 's The Science and Practice of Pharmacy. Lippincott Williams & Wilkins, 2005
- **T. W. GREENE ; P. G. M. WUTS.** Protecting Groups in Organic Synthesis. Wiley, 1991
- **J. JACQUES ; A. COLLET ; S.H. WILEN.** Enantiomers, Racemates and Resolutions. Wiley-Interscience, 1981
- **S.H. WILEN ; A. COLLET ; J. JACQUES.** *Tetrahedron,* 1977, 2725
- **E.L. ELIEL.** Stereochemistry of Carbon Compounds. McGraw-Hill, 1962
- **S.H. WILEN.** Tables of Resolving Agents and Optical Resolutions. Univ. of Notre Dame Press, 1972, vol. 268
- **ERNEST L. ELIEL ; SAMUEL H. WILEN ; LEWIS N. MANDA.** Stereochemistry of Organic Compounds. John Wiley & Sons, Inc, 1994
- **MIHÁLY NÓGRÁDI.** Stereoselective Synthesis A Practical Approach. VCH Publishers, Inc, 1995
- Salt Forms of Drugs and Adsorption. **BIGHLEY et al.** Encyclopedia of Pharmaceutical Technology. Marcel Dekker, 1995, vol. 13
- **TEN HOEVE ; H. WYNBERG.** *Journal of Organic Chemistry,* 1985, vol. 50, 4508-4514
- **DALE ; MOSHER.** *J. Am. Chem. Soc.,* 1973, vol. 95, 512
- *Chemistry and Physics of Lipids,* 2008, vol. 153, 85-97
- *Journal of Liposome Research,* 2010, vol. 20, 147-59

- **ABU HAMMAD I ; POPOV M ; LINDER C ; GRINBERG S ; HELDMAN E ; STEPENSKY D.** Bolaamphiphilic nanovesicles for the delivery of proteins to the brain. *Journal of Controlled Release,* 2011
- **AGYARE, EK ; KANDIMALLA KK ; PODUSLO JF ; YU CC ; RAMAKRISHNAN M ; CURRAN GL.** Development of a smart nano-vesicle to target cerebrovascular amyloid deposits and brain parenchymal plaques observed in Alzheimer's disease and cerebral amyloid angiopathy. *Pharm Res,* November 2008, vol. 25 (11), 2674-2684
- **FUHRHOP J.H. ; WANG T.** Bolaamphiphiles. *Chem. Rev.,* 2004, vol. 104, 2901-2937
- **GISSLEN M ; HAGBERG L ; HAGBERG.** Antiretroviral treatment of central nervous system HIV-1infection: a Review. *HIV Medicine,* 2001, vol. 2, 97-104
- **G GNANARAIAN ; AK GUPTA ; V JUVAL ; P KUMAR ; PK YADAV ; P KAILASH.** A validated method for development of tenofovir as API and tablet dosage forms by UV spectroscopy. *Pharm Analysis,* 2009, vol. 1 (4), 351-353
- **GRINBERG S. ; LINDER C. ; KOLOT V. ; WANER T. ; WIESMAN Z. ; SHAUBI E. ; HELDMAN E.** Novel cationic amphiphilic derivatives from vemonia oil: synthesis and self-aggregation into bilayer vesicles, nanoparticles, and DNA complexants. *Langmuir,* 2005, vol. 21 (17), 7638-7645
- **GRINBERG S. ; KOLOT V. ; LINDER C. ; SHAUBI E. ; KAS'YANOV V. ; DECKELBAUM R.J. ; HELDMAN E.** Synthesis of novel cationic bolaamphiphiles from vemonia oil and their aggregated structures. *Chem Phys Lipids,* 2008, vol. 153 (2), 85-97
- **GRINBERG, S. ; KIPNIS, N. ; LINDER, C. ; KOLOT, V. ; HELDMAN, E.** Assymetric bolaamphiphiles from veronica oil designed for drug delivery. *Eur. J. Lipid Sci. Technol.,* 2010, vol. 112, 137-151
- **HIGHLEYMAN, L.** *HIV and the Brain BETA,* 2009, vol. 21 (4), 16-29
- **HUTTER T ; LINDER C ; HELDMAN E ; GRINBERG S.** Interfacial and self-assembly properties of bolaamphiphilic compounds derived from a multifunctional oil. *Journal of Colloid and Interface Science,* 2011
- **JONASDOTTIR TJ ; FISHER DR ; BORREBAEK J ; BRULAND OS ; LARSEN RH.** First in vivo evaluation of liposome-encapsulated 223Ra as a potential alpha-particle-emitting cancer therapeutic agent. *Anticancer Res.,* 2006, vol. 26 (4B), 2841-2848
- **LETENDRE S ; MARQUIE-BECK J ; CAPPARELLI E ; BEST B ; CLIFFORD D ; COLLIER AC ; GELMAN BB ; MCARTHUR JC ; MCCUTCHAN JA ; MORGELLO S.** Validation of the CNS Penetration-Effectiveness rank for quantifying antiretroviral penetration into the central nervous system. *Arch Neurol.,* January 2008, vol. 65 (1), 65-70
- **LU W ; TAN YZ ; HU KL ; JIANG XG.** Cationic albumin conjugated pegylated nanoparticle with its transcytosis ability and little toxicity against blood-brain barrier. *Int J Pharm.,* 13 May 2005, vol. 295 (1-2), 247-260
- A Practical Approach. Liposomes. IRL Press, 1997
- **NEWTON HB.** Advances in strategies to improve drug delivery to brain tumors. *Expert Rev Neurother.,* 2006, vol. 6 (10), 1495-509
- **POPOV M. ; LINDER C. ; DECKELBAUM R.J. ; GRINBERG S. ; HANSEN I.H. ; SHAUBI E. ; WANER T. ; HELDMAN E.** Cationic vesicles from novel bolaamphiphilic compounds. *J Liposome Res.,* 2009, vol. 20 (2), 147-159
- **POPOV M ; GRINBERG S ; LINDER C ; BACHAR Z ; WANER T ; DECKELBAUM R ; HELDMAN E.** Site-directed decapsulation of bolaamphiphilic vesicles with enzymatic cleavable surface groups. *Journal of Controlled Release,* 2011
- **PURI, A. ; LOOMIS, K. ; SMITH, B. ; LEE, J. ; YAVLOVICH, A. ; HELDMAN, E. ; BLUMENTHAL, R.** Lipid-Based Nanoparticles as Pharmaceutical Drug Carriers: From Concepts to Clinic. *Crit Rev Ther Drug Carrier Syst,* 2009, vol. 26 (6), 523-580
- **SAIYED Z ; GANDHI N ; NAIRI M.** Magnetic Nanoformulation of Azidothymidine 5'-triphosphate for Targeted Delivery across the Blood-Brain Barrier. *International Journal of Nanomedicine,* 2010, vol. 5, 157-166
- **SONGJIANG Z ; LIXIANG W.** Amyloid-Beta Associated with Chitosan Nano-Carrier has Favorable Immunogenicity and Permeates the BBB. *AAPS Pharm Sci Tech,* 2009, vol. 10 (3), 900-905
- **SPUDICH S ; ANTSES B.** Central Nervous System Complications of HIV Infection. *Top. Antiviral Med,* 2011, vol. 19 (2), 48-57
- **STEM J ; FREISLEBEN HJ ; JANKU S ; RING K.** Black lipid membranes of tetraether lipids from Thermoplasma acidophilum. *Biochim Biophys Acta,* 1992, vol. 1128, 227-236
- **VARATHARAJAN L ; THOMAS S.** The transport of anti-HIV drugs across blood-CNS interfaces: Summary of current knowledge and recommendations. *Research Antiviral Res.,* May 2009, vol. 82 (2), A99-A109
- **WIESMAN Z. ; DOM N.B. ; SHARVIT E. ; GRINBERG S. ; LINDER C. ; HELDMAN E. ; ZACCAI M.** Novel cationic vesicle platform derived from vemonia oil for efficient delivery of DNA through plant cuticle membranes. *J Biotechnol.,* 2007, vol. 130 (1), 85-94